# EUROPEAN PATENT APPLICATION

(11) **EP 2 395 001 A1**
(43) Date of publication of application: **14.12.2011**
(21) Application number: 10163597.7
(22) Date of filing: 21.05.2010
(51) Int. Cl.: C07D 403/14, A61K 31/506, A61P 35/00

(54) **Novel pyrimidine derivatives**

(71) Applicant: Chemilia AB, 141 52 Huddinge (SE)
(72) Inventor: Dahlstedt, Emma, 115 43 Stockholm (SE); Johansson, Tommy, 167 36 Bromma (SE); Högberg, Marita, 146 52 Tullinge (SE)
(74) Representative: Lundquist, Tomas

(57) **Abstract**

The invention provides novel pyrimidine derivatives of formula I, to methods of preparing such compounds, to pharmaceutical compositions containing such compounds, and to methods for using such compounds in treatment of diseases including cancer; 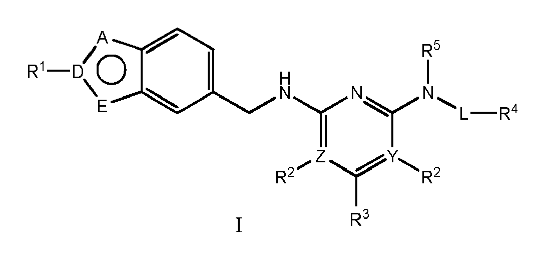 wherein R¹, R², R³, R⁴, R⁵, A, D, E, Z, and Y are as defined in the specification.

## Description

### Field of Invention

This invention relates to novel pyrimidine derivatives of formula I, to methods of preparing such compounds, to pharmaceutical compositions containing such compounds, and to methods for using such compounds in treatment of diseases including cancer.

### Background of Invention

Cancer is a major and often fatal disease. Accordingly, the development of new therapies for cancer is an ongoing process of outmost importance. The majority of cancers are present as solid tumours, such as lung cancer, breast cancer and prostate cancer, while others represent haematological and lymphoid malignancies, such as leukemias and lymphomas.

In the clinic cancer chemotherapy is used in attempts to cure or palliate the disease. In most cases this therapy is delivered in the form of combination chemotherapy, i.e. when two or more drugs having different modes of action are used together in order to optimise the effect on the cancer cells and to minimise side effects. The results obtained with chemotherapy vary according to tumour type. Some tumours are very sensitive and the treatment has a high probability of leading to beneficial treatment results including cure of the disease. Examples of this type of tumours are acute leukemias, malignant lymphomas, testicular cancer, chorion carcinomas, and Wilms tumour. Other types of cancer chemotherapy can result in effective palliation and prolonged survival. Examples of such tumours are breast cancer, colorectal cancer, ovarian cancer, small-cell lung cancer, bladder cancer, multiple myeloma, and chronic leukemias of both the lymphatic and myeloid type. Primary drug resistant tumours which respond poorly to classical chemotherapy include malignant glioma, melanoma, prostate cancer, sarcomas, and gastrointestinal tumours other than colorectal cancers (see e.g. DeVita, Hellman, and Rosenberg: Cancer: Principles & Practice of Oncology, Eighth Edition 978-0-7817-7207-5) .

During the recent decade much interest has been devoted to drugs directed to specific target molecules. It is believed that these targeted drugs may work in a more specific manner, eliminating subpopulations of cells involved in tumour survival and progression. Molecules regulating cell proliferation and death, such as Tyrosine Kinase Receptors (RTKs) for growth factors, are among the most promising targets for this type of therapeutic approach. Two classes of compounds targeting RTKs are currently used in clinical practice: monoclonal antibodies and tyrosine kinase inhibitors. The first approved targeted therapies were trastuzumab, a monoclonal antibody against HER2, for treatment of metastatic breast cancer, and imatinib, a small tyrosine kinase inhibitor targeting BCR-Abl, in Chronic Myeloid Leukemia. Despite good treatment results many of the treated patients have developed drug resistance, often due to the activation of alternative RTKs pathways. Currently there is a general understanding that molecules interfering simultaneously with multiple RTKs might be more effective than single target agents. There are a few recently approved drugs, such as sorafenib and sunitinib, that apparently target multiple pathways and could serve as representatives of this new generation of anti-cancer drugs (see e.g. Gossage and Eisen, Targeting multiple kinase pathways: a change in paradigm. Clin Cancer Res (2010) vol. 16 (7) pp. 1973-8).

Certain pyrimidine compounds and their potential use in the treatment of cancer are disclosed in for example WO2003/063794, WO2004/056807, WO2004/056786, WO2006/133426, WO2007/085833, WO2008/128231 and WO2009/063240.

What is needed in the art are targeted drugs that work in a specific manner, being selective in eliminating subpopulations of cells involved in tumour survival and progression. The present invention provides novel pyrimidine compounds having a surprisingly efficient and selective antiproliferative activity. Hence, these novel compounds are useful in the treatment of proliferative diseases, such as cancer.

### Description of the invention

The present invention provides compounds of formula I or a pharmaceutically acceptable ester, amide, solvate or salt thereof, 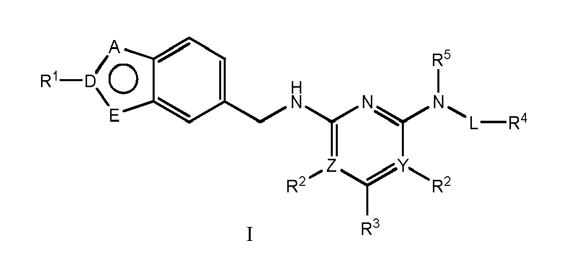 wherein
Z represents carbon or nitrogen;
Y represents carbon or nitrogen; wherein one of Z and Y represents nitrogen;
A, D and E is selected from carbon and nitrogen, wherein A represents nitrogen and
D and E represents carbon; or A and D represent nitrogen and E represents carbon; or
A and E represent nitrogen and D represents carbon; or E represents nitrogen and A
and D represent carbon;
L represents a bond or (C₁-C₂)alkyl;
R¹ represents hydrogen or methyl, when D represents carbon;
R² represents hydrogen or amino, when Y or Z represents carbon;
R³ represents hydrogen, (C₁-C₃)alkyl, amino, trifluoromethyl or (C₀-C₁)alkylaryl;
R⁴ represents heteroaryl, optionally substituted with one or more substituents; and
R⁵ represents hydrogen or methyl.

In one aspect of the invention, there is provided a compound of formula I, wherein Z, D and E represent carbon; and Y and A represent nitrogen. This is illustrated by formula Ia, wherein R¹, R², R³, R⁴, R⁵ and L are as defined under formula I above:

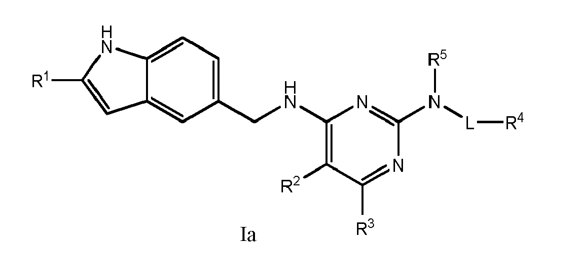

In another aspect of the invention, there is provided a compound of formula I, wherein Z and E represent carbon; and Y, D and A represent nitrogen. This is illustrated by formula Ib, wherein R², R³, R⁴, R⁵ and L are as defined under formula I above:

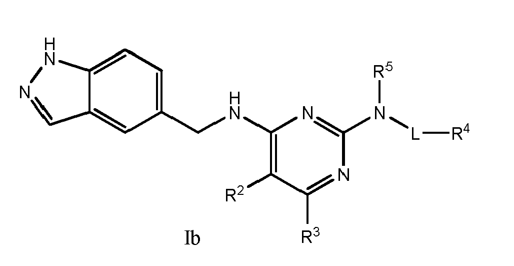

In another aspect of the invention, there is provided a compound of formula I, wherein Z and D represent carbon; and Y, E and A represent nitrogen. This is illustrated by formula Ic, wherein R¹, R², R³, R⁴, R⁵ and L are as defined under formula I above:

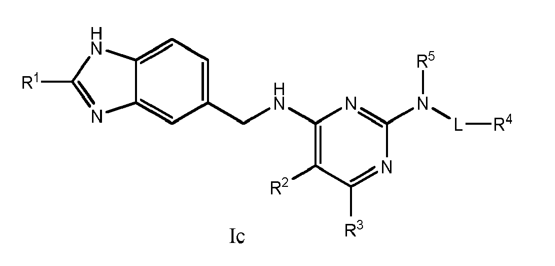

In another aspect of the invention, there is provided a compound of formula I, wherein Z, A and D represent carbon; and Y and E represent nitrogen. This is illustrated by formula Id, wherein R¹, R², R³, R⁴, R⁵ and L are as defined under formula I above:

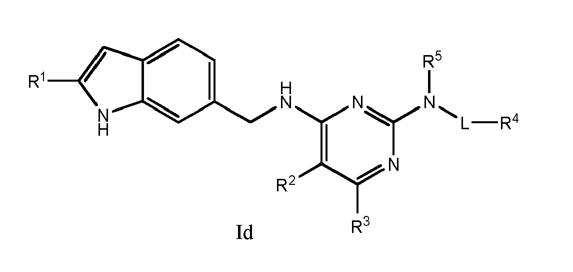

In another aspect of the invention, there is provided a compound of formula I, wherein Y, D and E represent carbon; and Z and A represent nitrogen. This is illustrated by formula Ie, wherein R¹, R², R³, R⁴, R⁵ and L are as defined under formula I above:

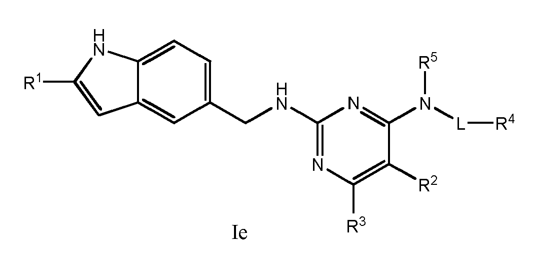

In another aspect of the invention, there is provided a compound of formula I, wherein Y and E represent carbon; and Z, D and A represent nitrogen. This is illustrated by formula If, wherein R², R³, R⁴, R⁵ and L are as defined under formula I above:

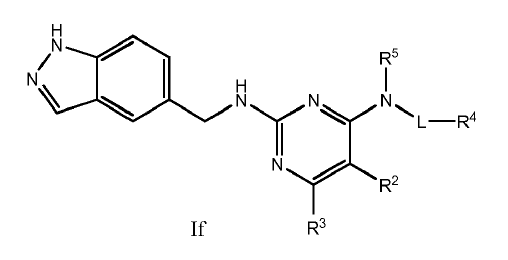

In another aspect of the invention, there is provided a compound of formula I, wherein Y and D represent carbon; and Z, E and A represent nitrogen. This is illustrated by formula Ig, wherein R¹, R², R³, R⁴, R⁵ and L are as defined under formula I above:

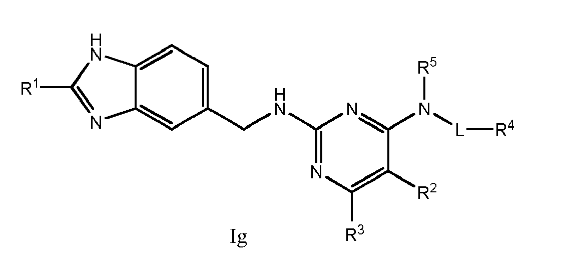

In another aspect of the invention, there is provided a compound of formula I, wherein Y, A and D represent carbon; and Z and E represent nitrogen. This is illustrated by formula Ih, wherein R¹, R², R³, R⁴, R⁵ and L are as defined under formula I above:

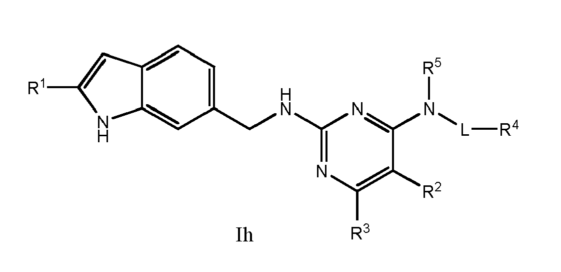

In another aspect of the invention, R⁴ represents heteroaryl that is optionally substituted with one or more substituents. Such substituents include, but are not limited to, halogen, hydroxy, amino, nitro, cyano, (C₁-C₄)alkyl, (C₁-C₄)alkyl(C₂-C₉)heterocyclyl, (C₁-C₄)alkyl(CO)OH, (C₁-C₄)alkyl(CO)O(C₁-C₄)alkyl, (C₁-C₄)alkyl(CO)NH₂, (C₁-C₄)alkyl(CO)NH(C₁-C₄)alkyl, (C₁-C₄)alkyl-OH, (C₁-C₄)alkyl-O(C₆-C₁₀)aryl, (C₁-C₄)alkyl-O(CO)(C₁-C₄)alkyl, (C₁-C₄)alky-O(CO)(C₆-C₁₀)aryl, (C₁-C₄)alkyl-NH₂, (C₁-C₄)alkyl-NH(C₁-C₄)alkyl, (C₁-C₄)alkyl-N[(C₁-C₄)alkyl][(C₁-C₄)-alkyl], (C₁-C₄)alkyl-NH(CO)(C₁-C₄)alkyl, (C₁-C₄)alkyl(CN), (C₁-C₄)alkyl(C₆-C₁₀)aryl, (CO)OH, (CO)O(C₁-C₄)alkyl, (CO)NH₂, (CO)NH(C₁-C₄)alkyl, (CO)(C₁-C₄)alkyl, (C₆-C₁₀)aryl, (C₆-C₁₀)aryl-halogen, (C₆-C₁₀)aryl-OH, (C₆-C₁₀)aryl-O(C₁-C₄)alkyl, (C₁-C₉)heteroaryl, (C₁-C₉)heteroaryl-halogen, (C₁-C₉)heteroaryl-OH, (C₁-C₉)heteroaryl-O(C₁-C₄)alkyl, (C₂-C₉)heterocyclyl, (C₂-C₉)heterocyclyl(C₁-C₄)alkyl, (C₂-C₉)heterocyclyl(C₁-C₄)alkyl-OH, O(EtO)₁₋₃H, O(EtO)₁-₃(C₁-C₄)alkyl, O(C₆-C₁₀)aryl, O(CO)(C₁-C₄)alkyl, O(CO)(C₆-C₁₀)aryl, OSO₂0OH, NH(C₁-C₄)alkyl, N[(C₁-C₄)alkyl][(C₁-C₄)alkyl], NH(CO)(C₁-C₄)alkyl, NH(CO)(C₆-C₁₀)aryl, and CF₃.

In another aspect of the invention, R⁴ represents heteroaryl that is optionally substituted with one or more substituents selected from hydroxy, methyl, and methoxy.

In another aspect of the invention, there is provided a compound of formula I, wherein L-R⁴ is selected from: 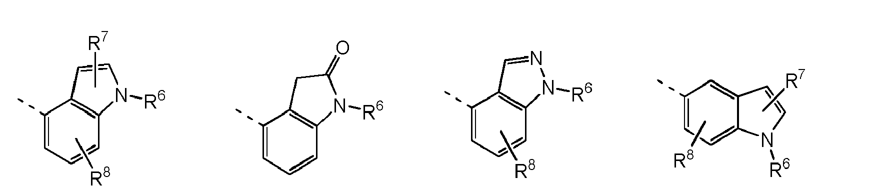 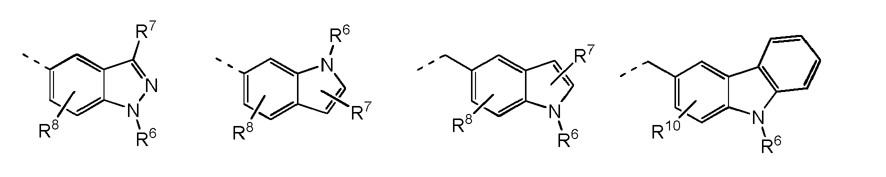 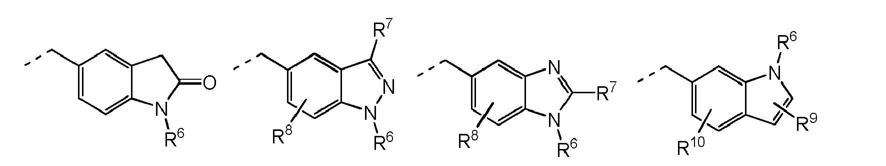 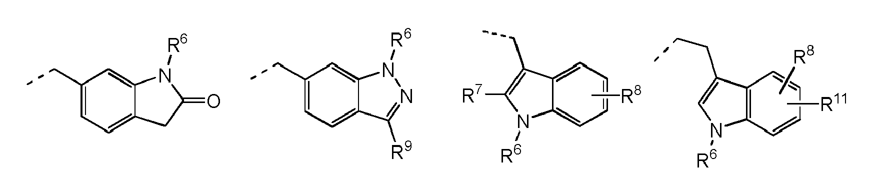 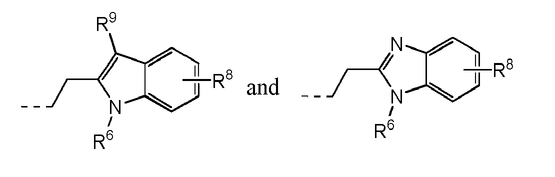 wherein R⁶ is selected from hydrogen and (C₁-C₄)alkyl;
R⁷ is selected from hydrogen, halogen, nitro, cyano, (C₁-C₄)alkyl(CO)OH, (C₁-C₄)alkyl(CO)O(C₁-C₄)alkyl, (C₁-C₄)alkyl(CO)NH₂, (C₁-C₄)alkyl(CO)NH(C₁-C₄)alkyl, (C₁-C₄)alkyl-OH, (C₁-C₄)alkyl-O(C₆-C₁₀)aryl, (C₁-C₄)alkyl-O(CO)(C₁-C₄)alkyl, (C₁-C₄)alkyl-O(CO)(C₆-C₁₀)aryl, (C₁-C₄)alkyl-NH₂, (C₁-C₄)alkyl-NH(C₁-C₄)alkyl, (C₁-C₄)alkyl-N[(C₁-C₄)alkyl][(C₁-C₄)alkyl], (C₁-C₄)alkyl-NH(CO)(C₁-C₄)alkyl, (C₁-C₄)alkyl(CN), (C₁-C₄)alkyl(C₆-C₁₀)aryl, (C₁-C₄)alkyl(C₂-C₉)heterocyclyl, (CO)OH, (CO)O(C₁-C₄)alkyl, (CO)NH₂, (CO)NH(C₁-C₄)alkyl, (CO)(C₁-C₄)alkyl, (C₆-C₁₀)aryl, (C₆-C₁₀)aryl-halogen, (C₆-C₁₀)aryl-OH, (C₆-C₁₀)aryl-O(C₁-C₄)alkyl, (C₁-C₉)heteroaryl, (C₁-C₉)heteroaryl-halogen, (C₁-C₉)heteroaryl-OH, (C₁-C₉)heteroaryl-O(C₁-C₄)alkyl, (C₂-C₉)heterocyclyl, (C₂-C₉)heterocyclyl(C₁-C₄)alkyl, and (C₂-C₉)heterocyclyl(C₁-C₄)alkyl-OH;
R⁸ is selected from hydrogen, halogen, nitro, cyano, hydroxy, amino, (C₁-C₄)alkyl, (C₁-C₄)alkyl(C₂-C₉)heterocyclyl, (C₁-C₄)alkyl-NH₂, (C₁-C₄)alkyl-NH(C₁-C₄)alkyl, (C₁-C₄)alkyl-N[(C₁-C₄)alkyl][(C₁-C₄)-alkyl], (C₁-C₄)alkyl-NH(CO)(C₁-C₄)alkyl, (C₁-C₄)alkyl-NH(CO)(C₆-C₁₀)aryl, (CO)(C₁-C₄)alkyl, (CO)OH, (CO)O(C₁-C₄)alkyl, (CO)NH₂, (CO)NH(C₁-C₄)alkyl, O(C₁-C₄)alkyl, O(C₁-C₄)alkyl(C₆-C₁₀)aryl, O(C₁-C₄)alkyl(C₁-C₉)heteroaryl, O(C₁-C₄)alkyl(C₂-C₉)heterocyclyl, O(C₁-C₄)alkyl(C₂-C₉)heterocyclyl(C₁-C₄)alkyl, O(C₁-C₄)alkyl(C₂-C₉)heterocyclyl(C₁-C₄)alkyl-OH, O(EtO)₁₋₃H, O(EtO)₁₋₃(C₁-C₄)alkyl, O(C₆-C₁₀)aryl, O(CO)(C₁-C₄)alkyl, O(CO)(C₆-C₁₀)aryl, OSO₂OH, NH(C₁-C₄)alkyl, N[(C₁-C₄)alkyl][(C₁-C₄)alkyl], NH(CO)(C₁-C₄)alkyl, NH(CO)(C₆-C₁₀)aryl, CF₃. (C₆-C₁₀)aryl, (C₆-C₁₀)aryl-halogen, (C₆-C₁₀)aryl-OH, (C₆-C₁₀)aryl-O(C₁-C₄)alkyl, (C₁-C₉)heteroaryl, (C₁-C₉)heteroaryl-halogen, (C₁-C₉)heteroaryl-OH, (C₁-C₉)heteroaryl-O(C₁-C₄)alkyl, (C₂-C₉)heterocyclyl, (C₂-C₉)heterocyclyl(C₁-C₄)alkyl, and (C₂-C₉)heterocyclyl(C₁-C₄)alkyl-OH;
R⁹ is selected from hydrogen, halogen, (C₁-C₄)alkyl, (C₁-C₄)alkyl-OH, (C₁-C₄)alkyl-O(C₁-C₄)alkyl, (CO)OH, (CO)O(C₁-C₄)alkyl, (CO)NH₂, (CO)NH(C₁-C₄)alkyl, and (C₆-C₁₀)aryl;
R¹⁰ is selected from hydrogen, halogen, hydroxy, (C₁-C₄)alkyl, (C₁-C₄)alkyl-OH, (C₁-C₄)alkyl-O(C₁-C₄)alkyl, and O(C₁-C₄)alkyl; and
R¹¹ is selected from hydrogen, hydroxy, (C₁-C₄)alkyl, and O(C₁-C₄)alkyl.

In another aspect of the invention, there is provided a compound of formula I, wherein L-R⁴ is selected from: 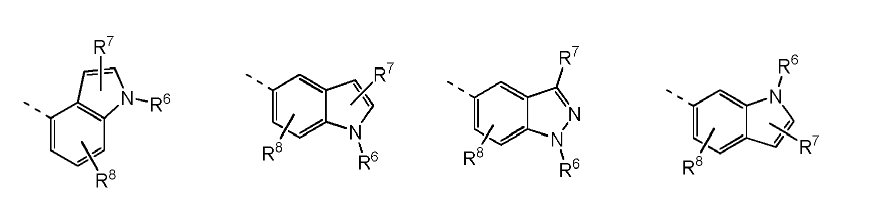 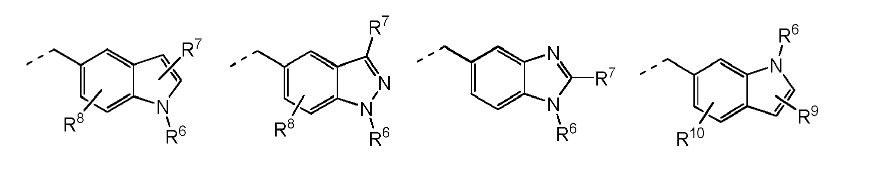 and 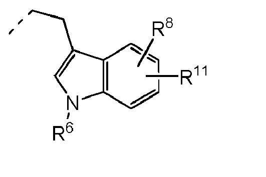 wherein
R⁶ represents hydrogen;
R⁷ is selected from hydrogen and (C₁-C₄)alkyl, preferably methyl;
R⁸, R⁹ and R¹⁰ represents hydrogen; and
R¹¹ is selected from hydrogen, hydroxy, (C₁-C₄)alkyl, preferably methyl, and O(C₁-C₄)alkyl, preferably methoxy.

In another aspect of the invention, there is provided a compound of formula I, wherein R⁵ represents hydrogen.

In another aspect of the invention, there is provided a compound of formula I, wherein R⁵ represents methyl.

In another aspect of the invention, there is provided a compound of formula I, wherein R² represents amino.

In another aspect of the invention, there is provided a compound of formula I, wherein R² represents hydrogen.

In another aspect of the invention, there is provided a compound of formula I, wherein R² represents hydrogen; and R³ represents hydrogen, methyl, trifluoromethyl or benzyl.

In another aspect of the invention, there is provided a compound of formula I,
wherein
Z, D and E represent carbon;
Y and A represent nitrogen;
L represents a bond or (C₁-C₂)alkyl;
R¹ represents hydrogen or methyl;
R² represents hydrogen;
R³ represents hydrogen or methyl;
R⁴ represents a heteroaryl selected from indolyl, indazolyl, benzimidazolyl, indolinonyl, and carbazolyl, said heteroaryl optionally substituted with one or two substituents selected from the group consisting of halogen, hydroxy, amino, nitro, cyano, (C₁-C₄)alkyl, (C₁-C₄)alkyl(C₂-C₉)heterocyclyl, (C₁-C₄)alkyl(CO)OH, (C₁-C₄)alkyl(CO)O(C₁-C₄)alkyl, (C₁-C₄)alkyl(CO)NH₂, (C₁-C₄)alkyl(CO)NH(C₁-C₄)alkyl, (C₁-C₄)alkyl-OH, (C₁-C₄)alkyl-O(C₆-C₁₀)aryl, (C₁-C₄)alkyl-O(CO)(C₁-C₄)alkyl, (C₁-C₄)alkyl-O(CO)(C₆-C₁₀)aryl, (C₁-C₄)alkyl-NH₂, (C₁-C₄)alkyl-NH(C₁-C₄)alkyl, (C₁-C₄)alkyl-N[(C₁-C₄)alkyl][(C₁-C₄)-alkyl], (C₁-C₄)alkyl-NH(CO)(C₁-C₄)alkyl, (C₁-C₄)alkyl(CN), (C₁-C₄)alkyl(C₆-C₁₀)aryl, (CO)OH, (CO)O(C₁-C₄)alkyl, (CO)NH₂, (CO)NH(C₁-C₄)alkyl, (CO)(C₁-C₄)alkyl, (C₆-C₁₀)aryl, (C₆-C₁₀)aryl-halogen, (C₆-C₁₀)aryl-OH, (C₆-C₁₀)aryl-O(C₁-C₄)alkyl, (C₁-C₉)heteroaryl, (C₁-C₉)heteroaryl-halogen, (C₁-C₉)heteroaryl-OH, (C₁-C₉)heteroaryl-O(C₁-C₄)alkyl, (C₂-C₉)heterocyclyl, (C₂-C₉)heterocyclyl(C₁-C₄)alkyl, (C₂-C₉)heterocyclyl(C₁-C₄)alkyl-OH, O(EtO)₁₋₃H, O(EtO)₁₋₃(C₁-C₄)alkyl, O(C₆-C₁₀)aryl, O(CO)(C₁-C₄)alkyl, O(CO)(C₆-C₁₀)aryl, OSO₂OH, NH(C₁-C₄)alkyl, N[(C₁-C₄)alkyl][(C₁-C₄)alkyl], NH(CO)(C₁-C₄)alkyl, NH(CO)(C₆-C₁₀)aryl, and CF₃; and
R⁵ represents hydrogen or methyl.

In another aspect of the invention, there is provided a compound of formula I,
wherein
Z and E represent carbon;
Y, D, and A represent nitrogen;
L represents a bond or (C₁-C₂)alkyl;
R² represents hydrogen;
R³ represents hydrogen or methyl;
R⁴ represents a heteroaryl selected from indolyl, indazolyl, benzimidazolyl, indolinonyl, and carbazolyl, said heteroaryl optionally substituted with one or two substituents selected from the group consisting of halogen, hydroxy, amino, nitro, cyano, (C₁-C₄)alkyl, (C₁-C₄)alkyl(C₂-C₉)heterocyclyl, (C₁-C₄)alkyl(CO)OH, (C₁-C₄)alkyl(CO)O(C₁-C₄)alkyl, (C₁-C₄)alkyl(CO)NH₂, (C₁-C₄)alkyl(CO)NH(C₁-C₄)alkyl, (C₁-C₄)alkyl-OH, (C₁-C₄)alkyl-O(C₆-C₁₀)aryl, (C₁-C₄)alkyl-O(CO)(C₁-C₄)alkyl, (C₁-C₄)alkyl-O(CO)(C₆-C₁₀)aryl, (C₁-C₄)alkyl-NH₂, (C₁-C₄)alkyl-NH(C₁-C₄)alkyl, (C₁-C₄)alkyl-N[(C₁-C₄)alkyl][(C₁-C₄)-alkyl], (C₁-C₄)alkyl- NH(CO)(C₁-C₄)alkyl, (C₁-C₄)alkyl(CN), (C₁-C₄)alkyl(C₆-C₁₀)aryl, (CO)OH, (CO)O(C₁-C₄)alkyl, (CO)NH₂, (CO)NH(C₁-C₄)alkyl, (CO)(C₁-C₄)alkyl, (C₆-C₁₀)aryl, (C₆-C₁₀)aryl-halogen, (C₆-C₁₀)aryl-OH, (C₆-C₁₀)aryl-O(C₁-C₄)alkyl, (C₁-C₉)heteroaryl, (C₁-C₉)heteroaryl-halogen, (C₁-C₉)heteroaryl-OH, (C₁-C₉)heteroaryl-O(C₁-C₄)alkyl, (C₂-C₉)heterocyclyl, (C₂-C₉)heterocyclyl(C₁-C₄)alkyl, (C₂-C₉)heterocyclyl(C₁-C₄)alkyl-OH, O(EtO)₁₋₃H, O(EtO)₁₋₃(C₁-C₄)alkyl, O(C₆-C₁₀)aryl, O(CO)(C₁-C₄)alkyl, O(CO)(C₆-C₁₀)aryl, OSO₂OH, NH(C₁-C₄)alkyl, N[(C₁-C₄)alkyl][(C₁-C₄)alkyl], NH(CO)(C₁-C₄)alkyl, NH(CO)(C₆-C₁₀)aryl, and CF₃; and
R⁵ represents hydrogen or methyl.

In another aspect of the invention, there is provided a compound of formula I,
wherein
Z and D represent carbon;
Y, E and A represent nitrogen;
L represents a bond or (C₁-C₂)alkyl;
R¹ represents hydrogen;
R² represents hydrogen;
R³ represents hydrogen or methyl;
R⁴ represents a heteroaryl selected from indolyl, indazolyl, benzimidazolyl, indolinonyl, and carbazolyl, said heteroaryl optionally substituted with one or two substituents selected from the group consisting of halogen, hydroxy, amino, nitro, cyano, (C₁-C₄)alkyl, (C₁-C₄)alkyl(C₂-C₉)heterocyclyl, (C₁-C₄)alkyl(CO)OH, (C₁-C₄)alkyl(CO)O(C₁-C₄)alkyl, (C₁-C₄)alkyl(CO)NH₂, (C₁-C₄)alkyl(CO)NH(C₁-C₄)alkyl, (C₁-C₄)alkyl-OH, (C₁-C₄)alkyl-O(C₆-C₁₀)aryl, (C₁-C₄)alkyl-O(CO)(C₁-C₄)alkyl, (C₁-C₄)alkyl-O(CO)(C₆-C₁₀)aryl, (C₁-C₄)alkyl-NH₂, (C₁-C₄)alkyl-NH(C₁-C₄)alkyl, (C₁-C₄)alkyl-N[(C₁-C₄)alkyl][(C₁-C₄)-alkyl], (C₁-C₄)alkyl-NH(CO)(C₁-C₄)alkyl, (C₁-C₄)alkyl(CN), (C₁-C₄)alkyl(C₆-C₁₀)aryl, (CO)OH, (CO)O(C₁-C₄)alkyl, (CO)NH₂, (CO)NH(C₁-C₄)alkyl, (CO)(C₁-C₄)alkyl, (C₆-C₁₀)aryl, (C₆-C₁₀)aryl-halogen, (C₆-C₁₀)aryl-OH, (C₆-C₁₀)aryl-O(C₁-C₄)alkyl, (C₁-C₉)heteroaryl, (C₁-C₉)heteroaryl-halogen, (C₁-C₉)heteroaryl-OH, (C₁-C₉)heteroaryl-O(C₁-C₄)alkyl, (C₂-C₉)heterocyclyl, (C₂-C₉)heterocyclyl(C₁-C₄)alkyl, (C₂-C₉)heterocyclyl(C₁-C₄)alkyl-OH, O(EtO)₁₋₃H, O(EtO)₁₋₃(C₁-C₄)alkyl, O(C₆-C₁₀)aryl, O(CO)(C₁-C₄)alkyl, O(CO)(C₆-C₁₀)aryl, OSO₂OH, NH(C₁-C₄)alkyl, N[(C₁-C₄)alkyl][(C₁-C₄)alkyl], NH(CO)(C₁-C₄)alkyl, NH(CO)(C₆-C₁₀)aryl, and CF₃; and
R⁵ represents hydrogen or methyl.

In another aspect of the invention, there is provided a compound of formula I,
wherein Z, A and D represent carbon;
Y and E represent nitrogen;
L represents a bond or (C₁-C₂)alkyl;
R¹ represents hydrogen or methyl;
R² represents hydrogen;
R³ represents hydrogen or methyl;
R⁴ represents a heteroaryl selected from indolyl, indazolyl, benzimidazolyl, indolinonyl, and carbazolyl, said heteroaryl optionally substituted with one or two substituents selected from the group consisting of halogen, hydroxy, amino, nitro, cyano, (C₁-C₄)alkyl, (C₁-C₄)alkyl(C₂-C₉)heterocyclyl, (C₁-C₄)alkyl(CO)OH, (C₁-C₄)alkyl(CO)O(C₁-C₄)alkyl, (C₁-C₄)alkyl(CO)NH₂, (C₁-C₄)alkyl(CO)NH(C₁-C₄)alkyl, (C₁-C₄)alkyl-OH, (C₁-C₄)alkyl-O(C₆-C₁₀)aryl, (C₁-C₄)alkyl-O(CO)(C₁-C₄)alkyl, (C₁-C₄)alkyl-O(CO)(C₆-C₁₀)aryl, (C₁-C₄)alkyl-NH₂, (C₁-C₄)alkyl-NH(C₁-C₄)alkyl, (C₁-C₄)alkyl-N[(C₁-C₄)alkyl][(C₁-C₄)-alkyl], (C₁-C₄)alkyl-NH(CO)(C₁-C₄)alkyl, (C₁-C₄)alkyl(CN), (C₁-C₄)alkyl(C₆-C₁₀)aryl, (CO)OH, (CO)O(C₁-C₄)alkyl, (CO)NH₂, (CO)NH(C₁-C₄)alkyl, (CO)(C₁-C₄)alkyl, (C₆-C₁₀)aryl, (C₆-C₁₀)aryl-halogen, (C₆-C₁₀)aryl-OH, (C₆-C₁₀)aryl-O(C₁-C₄)alkyl, (C₁-C₉)heteroaryl, (C₁-C₉)heteroaryl-halogen, (C₁-C₉)heteroaryl-OH, (C₁-C₉)heteroaryl-O(C₁-C₄)alkyl, (C₂-C₉)heterocyclyl, (C₂-C₉)heterocyclyl(C₁-C₄)alkyl, (C₂-C₉)heterocyclyl(C₁-C₄)alkyl-OH, O(EtO)₁₋₃H, O(EtO)₁₋₃(C₁-C₄)alkyl, O(C₆-C₁₀)aryl, O(CO)(C₁-C₄)alkyl, O(CO)(C₆-C₁₀)aryl, OSO₂OH, NH(C₁-C₄)alkyl, N[(C₁-C₄)alkyl][(C₁-C₄)alkyl], NH(CO)(C₁-C₄)alkyl, NH(CO)(C₆-C₁₀)aryl, and CF₃; and
R⁵ represents hydrogen or methyl.

In another aspect of the invention, there is provided a compound of formula I,
wherein
Y, D and E represent carbon;
Z and A represent nitrogen;
L represents a bond or (C₁-C₂)alkyl;
R¹ represents hydrogen or methyl;
R² represents hydrogen;
R³ represents hydrogen or methyl;
R⁴ represents a heteroaryl selected from indolyl, indazolyl, benzimidazolyl, indolinonyl, and carbazolyl, said heteroaryl optionally substituted with one or two substituents selected from the group consisting of halogen, hydroxy, amino, nitro, cyano, (C₁-C₄)alkyl, (C₁-C₄)alkyl(C₂-C₉)heterocyclyl, (C₁-C₄)alkyl(CO)OH, (C₁-C₄)alkyl(CO)O(C₁-C₄)alkyl, (C₁-C₄)alkyl(CO)NH₂, (C₁-C₄)alkyl(CO)NH(C₁-C₄)alkyl, (C₁-C₄)alkyl-OH, (C₁-C₄)alkyl-O(C₆-C₁₀)aryl, (C₁-C₄)alkyl-O(CO)(C₁-C₄)alkyl, (C₁-C₄)alkyl-O(CO)(C₆-C₁₀)aryl, (C₁-C₄)alkyl-NH₂, (C₁-C₄)alkyl-NH(C₁-C₄)alkyl, (C₁-C₄)alkyl-N[(C₁-C₄)alkyl][(C₁-C₄)-alkyl], (C₁-C₄)alkyl-NH(CO)(C₁-C₄)alkyl, (C₁-C₄)alkyl(CN), (C₁-C₄)alkyl(C₆-C₁₀)aryl, (CO)OH, (CO)O(C₁-C₄)alkyl, (CO)NH₂, (CO)NH(C₁-C₄)alkyl, (CO)(C₁-C₄)alkyl, (C₆-C₁₀)aryl, (C₆-C₁₀)aryl-halogen, (C₆-C₁₀)aryl-OH, (C₆-C₁₀)aryl-O(C₁-C₄)alkyl, (C₁-C₉)heteroaryl, (C₁-C₉)heteroaryl-halogen, (C₁-C₉)heteroaryl-OH, (C₁-C₉)heteroaryl-O(C₁-C₄)alkyl, (C₂-C₉)heterocyclyl, (C₂-C₉)heterocyclyl(C₁-C₄)alkyl, (C₂-C₉)heterocyclyl(C₁-C₄)alkyl-OH, O(EtO)₁₋₃H, O(EtO)₁₋₃(C₁-C₄)alkyl, O(C₆-C₁₀)aryl, O(CO)(C₁-C₄)alkyl, O(CO)(C₆-C₁₀)aryl, OSO₂OH, NH(C₁-C₄)alkyl, N[(C₁-C₄)alkyl][(C₁-C₄)alkyl], NH(CO)(C₁-C₄)alkyl, NH(CO)(C₆-C₁₀)aryl, and CF₃; and
R⁵ represents hydrogen or methyl.

In another aspect of the invention, there is provided a compound of formula I,
wherein
Y and E represent carbon;
Z, D, and A represent nitrogen;
L represents a bond or (C₁-C₂)alkyl;
R² represents hydrogen;
R³ represents hydrogen or methyl;
R⁴ represents a heteroaryl selected from indolyl, indazolyl, benzimidazolyl, indolinonyl, and carbazolyl, said heteroaryl optionally substituted with one or two substituents selected from the group consisting of halogen, hydroxy, amino, nitro, cyano, (C₁-C₄)alkyl, (C₁-C₄)alkyl(C₂-C₉)heterocyclyl, (C₁-C₄)alkyl(CO)OH, (C₁-C₄)alkyl(CO)O(C₁-C₄)alkyl, (C₁-C₄)alkyl(CO)NH₂, (C₁-C₄)alkyl(CO)NH(C₁-C₄)alkyl, (C₁-C₄)alkyl-OH, (C₁-C₄)alkyl-O(C₆-C₁₀)aryl, (C₁-C₄)alkyl-O(CO)(C₁-C₄)alkyl, (C₁-C₄)alkyl-O(CO)(C₆-C₁₀)aryl, (C₁-C₄)alkyl-NH₂, (C₁-C₄)alkyl-NH(C₁-C₄)alkyl, (C₁-C₄)alkyl-N[(C₁-C₄)alkyl][(C₁-C₄)-alkyl], (C₁-C₄)alkyl- NH(CO)(C₁-C₄)alkyl, (C₁-C₄)alkyl(CN), (C₁-C₄)alkyl(C₆-C₁₀)aryl, (CO)OH, (CO)O(C₁-C₄)alkyl, (CO)NH₂, (CO)NH(C₁-C₄)alkyl, (CO)(C₁-C₄)alkyl, (C₆-C₁₀)aryl, (C₆-C₁₀)aryl-halogen, (C₆-C₁₀)aryl-OH, (C₆-C₁₀)aryl-O(C₁-C₄)alkyl, (C₁-C₉)heteroaryl, (C₁-C₉)heteroaryl-halogen, (C₁-C₉)heteroaryl-OH, (C₁-C₉)heteroaryl-O(C₁-C₄)alkyl, (C₂-C₉)heterocyclyl, (C₂-C₉)heterocyclyl(C₁-C₄)alkyl, (C₂-C₉)heterocyclyl(C₁-C₄)alkyl-OH, O(EtO)₁₋₃H, O(EtO)₁₋₃(C₁-C₄)alkyl, O(C₆-C₁₀)aryl, O(CO)(C₁-C₄)alkyl, O(CO)(C₆-C₁₀)aryl, OSO₂OH, NH(C₁-C₄)alkyl, N[(C₁-C₄)alkyl][(C₁-C₄)alkyl], NH(CO)(C₁-C₄)alkyl, NH(CO)(C₆-C₁₀)aryl, and CF₃; and
R⁵ represents hydrogen or methyl.

In another aspect of the invention, there is provided a compound of formula I,
wherein
Y and D represent carbon;
Z, E and A represent nitrogen;
L represents a bond or (C₁-C₂)alkyl;
R¹ represents hydrogen;
R² represents hydrogen;
R³ represents hydrogen or methyl;
R⁴ represents a heteroaryl selected from indolyl, indazolyl, benzimidazolyl, indolinonyl, and carbazolyl, said heteroaryl optionally substituted with one or two substituents selected from the group consisting of halogen, hydroxy, amino, nitro, cyano, (C₁-C₄)alkyl, (C₁-C₄)alkyl(C₂-C₉)heterocyclyl, (C₁-C₄)alkyl(CO)OH, (C₁-C₄)alkyl(CO)O(C₁-C₄)alkyl, (C₁-C₄)alkyl(CO)NH₂, (C₁-C₄)alkyl(CO)NH(C₁-C₄)alkyl, (C₁-C₄)alkyl-OH, (C₁-C₄)alkyl-O(C₆-C₁₀)aryl, (C₁-C₄)alkyl-O(CO)(C₁-C₄)alkyl, (C₁-C₄)alkyl-O(CO)(C₆-C₁₀)aryl, (C₁-C₄)alkyl-NH₂, (C₁-C₄)alkyl-NH(C₁-C₄)alkyl, (C₁-C₄)alkyl-N[(C₁-C₄)alkyl][(C₁-C₄)-alkyl], (C₁-C₄)alkyl-NH(CO)(C₁-C₄)alkyl, (C₁-C₄)alkyl(CN), (C₁-C₄)alkyl(C₆-C₁₀)aryl, (CO)OH, (CO)O(C₁-C₄)alkyl, (CO)NH₂, (CO)NH(C₁-C₄)alkyl, (CO)(C₁-C₄)alkyl, (C₆-C₁₀)aryl, (C₆-C₁₀)aryl-halogen, (C₆-C₁₀)aryl-OH, (C₆-C₁₀)aryl-O(C₁-C₄)alkyl, (C₁-C₉)heteroaryl, (C₁-C₉)heteroaryl-halogen, (C₁-C₉)heteroaryl-OH, (C₁-C₉)heteroaryl-O(C₁-C₄)alkyl, (C₂-C₉)heterocyclyl, (C₂-C₉)heterocyclyl(C₁-C₄)alkyl, (C₂-C₉)heterocyclyl(C₁-C₄)alkyl-OH, O(EtO)₁₋₃H, O(EtO)₁₋₃(C₁-C₄)alkyl, O(C₆-C₁₀)aryl, O(CO)(C₁-C₄)alkyl, O(CO)(C₆-C₁₀)aryl, OSO₂OH, NH(C₁-C₄)alkyl, N[(C₁-C₄)alkyl][(C₁-C₄)alkyl], NH(CO)(C₁-C₄)alkyl, NH(CO)(C₆-C₁₀)aryl, and CF₃; and
R⁵ represents hydrogen or methyl.

In another aspect of the invention, there is provided a compound of formula I,
wherein
Y, A and D represent carbon;
Z and E represent nitrogen;
L represents a bond or (C₁-C₂)alkyl;
R¹ represents hydrogen;
R² represents hydrogen;
R³ represents hydrogen or methyl;
R⁴ represents a heteroaryl selected from indolyl, indazolyl, benzimidazolyl, indolinonyl, and carbazolyl, said heteroaryl optionally substituted with one or two substituents selected from the group consisting of halogen, hydroxy, amino, nitro, cyano, (C₁-C₄)alkyl, (C₁-C₄)alkyl(C₂-C₉)heterocyclyl, (C₁-C₄)alkyl(CO)OH, (C₁-C₄)alkyl(CO)O(C₁-C₄)alkyl, (C₁-C₄)alkyl(CO)NH₂, (C₁-C₄)alkyl(CO)NH(C₁-C₄)alkyl, (C₁-C₄)alkyl-OH, (C₁-C₄)alkyl-O(C₆-C₁₀)aryl, (C₁-C₄)alkyl-O(CO)(C₁-C₄)alkyl, (C₁-C₄)alkyl-O(CO)(C₆-C₁₀)aryl, (C₁-C₄)alkyl-NH₂, (C₁-C₄)alkyl-NH(C₁-C₄)alkyl, (C₁-C₄)alkyl-N[(C₁-C₄)alkyl][(C₁-C₄)-alkyl], (C₁-C₄)alkyl-NH(CO)(C₁-C₄)alkyl, (C₁-C₄)alkyl(CN), (C₁-C₄)alkyl(C₆-C₁₀)aryl, (CO)OH, (CO)O(C₁-C₄)alkyl, (CO)NH₂, (CO)NH(C₁-C₄)alkyl, (CO)(C₁-C₄)alkyl, (C₆-C₁₀)aryl, (C₆-C₁₀)aryl-halogen, (C₆-C₁₀)aryl-OH, (C₆-C₁₀)aryl-O(C₁-C₄)alkyl, (C₁-C₉)heteroaryl, (C₁-C₉)heteroaryl-halogen, (C₁-C₉)heteroaryl-OH, (C₁-C₉)heteroaryl-O(C₁-C₄)alkyl, (C₂-C₉)heterocyclyl, (C₂-C₉)heterocyclyl(C₁-C₄)alkyl, (C₂-C₉)heterocyclyl(C₁-C₄)alkyl-OH, O(EtO)₁₋₃H, O(EtO)₁₋₃(C₁-C₄)alkyl, O(C₆-C₁₀)aryl, O(CO)(C₁-C₄)alkyl, O(CO)(C₆-C₁₀)aryl, OSO₂OH, NH(C₁-C₄)alkyl, N[(C₁-C₄)alkyl][(C₁-C₄)alkyl], NH(CO)(C₁-C₄)alkyl, NH(CO)(C₆-C₁₀)aryl, and CF₃; and
R⁵ represents hydrogen or methyl.

In another aspect of the invention, there is provided a compound of formula I,
wherein
Z, D and E represent carbon;
Y and A represent nitrogen;
L represents a bond or (C₁-C₂)alkyl;
R¹ represents hydrogen or methyl;
R² represents hydrogen;
R³ represents hydrogen or methyl;
R⁴ represents a heteroaryl selected from indolyl, indazolyl, and benzimidazolyl, said heteroaryl optionally substituted with one or two substituents selected from the group consisting of hydroxy, methyl, and methoxy; and
R⁵ represents hydrogen.

In another aspect of the invention, there is provided a compound of formula I,
wherein
Z and E represent carbon;
Y, D, and A represent nitrogen;
L represents a bond or (C₁-C₂)alkyl;
R² represents hydrogen;
R³ represents hydrogen or methyl;
R⁴ represents a heteroaryl selected from indolyl, indazolyl, and benzimidazolyl, said heteroaryl optionally substituted with one or two substituents selected from the group consisting of hydroxy, methyl, and methoxy; and
R⁵ represents hydrogen.

In another aspect of the invention, there is provided a compound of formula I,
wherein
Z and D represent carbon;
Y, E and A represent nitrogen;
L represents a bond or (C₁-C₂)alkyl;
R¹ represents hydrogen;
R² represents hydrogen;
R³ represents hydrogen or methyl;
R⁴ represents a heteroaryl selected from indolyl, indazolyl, and benzimidazolyl, said heteroaryl optionally substituted with one or two substituents selected from the group consisting of hydroxy, methyl, and methoxy; and
R⁵ represents hydrogen.

In another aspect of the invention, there is provided a compound of formula I,
wherein Z, A and D represent carbon;
Y and E represent nitrogen;
L represents a bond or (C₁-C₂)alkyl;
R¹ represents hydrogen or methyl;
R² represents hydrogen;
R³ represents hydrogen or methyl;
R⁴ represents a heteroaryl selected from indolyl, indazolyl, and benzimidazolyl, said heteroaryl optionally substituted with one or two substituents selected from the group consisting of hydroxy, methyl, and methoxy; and
R⁵ represents hydrogen.

In another aspect of the invention, there is provided a compound of formula I,
wherein
Y, D and E represent carbon;
Z and A represent nitrogen;
L represents a bond or (C₁-C₂)alkyl;
R¹ represents hydrogen or methyl;
R² represents hydrogen;
R³ represents hydrogen or methyl;
R⁴ represents a heteroaryl selected from indolyl, indazolyl, and benzimidazolyl, said heteroaryl optionally substituted with one or two substituents selected from the group consisting of hydroxy, methyl, and methoxy; and
R⁵ represents hydrogen.

In another aspect of the invention, there is provided a compound of formula I,
wherein
Y and E represent carbon;
Z, D, and A represent nitrogen;
L represents a bond or (C₁-C₂)alkyl;
R² represents hydrogen;
R³ represents hydrogen or methyl;
R⁴ represents a heteroaryl selected from indolyl, indazolyl, and benzimidazolyl, said heteroaryl optionally substituted with one or two substituents selected from the group consisting of hydroxy, methyl, and methoxy; and
R⁵ represents hydrogen.

In another aspect of the invention, there is provided a compound of formula I,
wherein
Y and D represent carbon;
Z, E and A represent nitrogen;
L represents a bond or (C₁-C₂)alkyl;
R¹ represents hydrogen;
R² represents hydrogen;
R³ represents hydrogen or methyl;
R⁴ represents a heteroaryl selected from indolyl, indazolyl, and benzimidazolyl, said heteroaryl optionally substituted with one or two substituents selected from the group consisting of hydroxy, methyl, and methoxy; and
R⁵ represents hydrogen.

In another aspect of the invention, there is provided a compound of formula I,
wherein
Y, A and D represent carbon;
Z and E represent nitrogen;
L represents a bond or (C₁-C₂)alkyl;
R¹ represents hydrogen;
R² represents hydrogen;
R³ represents hydrogen or methyl;
R⁴ represents a heteroaryl selected from indolyl, indazolyl, and benzimidazolyl, said heteroaryl optionally substituted with one or two substituents selected from the group consisting of hydroxy, methyl, and methoxy; and
R⁵ represents hydrogen.

In another aspect of the invention, there is provided a compound of formula I, said compound being selected from:
*N*⁴-(1*H*-indol-5-ylmethyl)-*N*²-(1*H*-indol-4-yl)pyrimidine-2,4-diamine;
*N*⁴-(1H-indol-5-ylmethyl)-*N*²-(1*H*-indol-5-yl)pyrimidine-2,4-diamine;
*N*⁴-(1H-indol-5-ylmethyl)-*N*²-(2-methyl-1*H*-indol-5-yl)pyrimidine-2,4-diamine;
*N*⁴-(1*H*-indol-5-ylmethyl)-*N*²-(1*H*-indazol-5-yl)pyrimidine-2,4-diamine;
*N*²*,N*⁴-bis(1*H*-indol-5-ylmethyl)pyrimidine-2,4-diamine;
*N*⁴*-*(1*H*-indol-5-ylmethyl)-*N*²-(2-methyl-1*H*-indol-5-ylmethyl)pyrimidine-2,4-diamine;
*N*²-(1*H*-indazol-5-ylmethyl)-*N*⁴-(1*H*-indol-5-ylmethyl)pyrimidine-2,4-diamine;
*N²-*(1*H*-benzo[*d*]imidazol-5-ylmethyl)-*N*⁴-(1*H*-indol-5-ylmethyl)pyrimidine-2,4-diamine;
*N*⁴-(1*H*-indol-5-ylmethyl)-*N*²-(1*H*-indol-6-ylmethyl)pyrimidine-2,4-diamine;
*N*²-[2-(1*H*-indol-3-yl)ethyl]-*N*⁴-(1*H*-indol-5-ylmethyl)pyrimidine-2,4-diamine;
3-{2-[4-(1*H*-indol-5-ylmethylamino)-pyrimidin-2-ylamino]ethyl}-1*H*-indol-5-ol;
*N⁴*-(1*H*-indol-5-ylmethyl)-*N*²-[2-(5-methyl-1*H*-indol-3-yl)ethyl]pyrimidine-2,4-diamine;
*N⁴*-(1*H*-indol-5-ylmethyl)-*N*²-[2-(5-methoxy-1*H*-indol-3-yl)ethyl]pyrimidine-2,4-diamine;
*N²*-(1*H*-indol-4-yl)-*N*⁴-(2-methyl-1*H*-indol-5ylmethyl)pyrimidine-2,4-diamine;
*N²*-(1*H*-indol-5-ylmethyl)-*N*⁴-(2-methyl-1*H*-indol-5-ylmethyl)pyrimidine-2,4-diamine;
*N*²,*N*⁴-Bis-(2-methyl-1*H*-indol-5-ylmethyl)pyrimidine-2,4-diamine;
*N*²-(1*H*-indazol-5-ylmethyl)-*N*⁴-(2-methyl-1*H*-indol-5ylmethyl)pyrimidine-2,4-diamine;
*N*²-(2-(1*H*-indol-3-yl)-ethyl)-*N*⁴-(2-methyl-1*H*-indol-5ylmethyl)pyrimidine-2,4-diamine;
*N*²-(1*H*-indol-4-yl)-*N*⁴-(1*H*-indazol-5ylmethyl)pyrimidine-2,4-diamine;
*N*⁴-(1*H*-benzo[*d*]imidazol-5-ylmethyl)-*N*²-(1*H*-indol-4-yl)pyrimidine-2,4-diamine;
*N*⁴-(1*H*-indol-6-ylmethyl)-*N*²-(1*H*-indol-4-yl)pyrimidine-2,4-diamine;
*N*²-(1*H*-indol-5-ylmethyl)-*N*⁴-(1*H*-indol-6-ylmethyl)pyrimidine-2,4-diamine;
*N*²,*N*⁴-bis-(1*H*-indol-6-ylmethyl)pyrimidine-2,4-diamine;
*N*²-(1*H*-indol-5-ylmethyl)-*N*⁴-(1*H*-indol-4-yl)pyrimidine-2,4-diamine;
*N*²-(1*H*-indol-5-ylmethyl)-*N*⁴-(2-methyl-1*H*-indol-5-yl)pyrimidine-2,4-diamine;
*N*⁴-[2-(1*H*-indol-3-yl)ethyl]-*N*²-(1*H*-indol-5-ylmethyl)pyrimidine-2,4-diamine;
3-{2-[2-(1*H*-indol-5-ylmethylamino)-pyrimidin-4-ylamino]ethyl}-1*H*-indol-5-ol;
*N*²-(1*H*-indol-5-ylmethyl)-*N*⁴-[2-(5-methyl-1*H*-indol-3-yl)ethyl]pyrimidine-2,4-diamine;
*N*²-(1*H*-indol-5-ylmethyl)-*N*⁴-[2-(5-methoxy-1*H*-indol-3-yl)ethyl]pyrimidine-2,4-diamine;
*N*²-(1*H*-indazol-5-ylmethyl)-*N*⁴-(1*H*-indol-4-yl)pyrimidine-2,4-diamine;
*N*²-(1*H*-indol-4-yl)-*N*⁴-(1*H*-indol-5ylmethyl)-6-methylpyrimidine-2,4-diamine;
*N*²,*N*⁴-bis(1*H*-indol-5-ylmethyl)-6-methylpyrimidine-2,4-diamine;
3-{2-[4-(1*H*-indol-5-ylmethylamino)-6-methyl-pyrimidin-2-ylamino]-ethyl}-1*H-*indol-5-ol;
*N*⁴-(1*H*-indol-5-ylmethyl)-*N*²-(2-methyl-1*H*-indol-5-yl)-6-trifluoromethylpyrimidine-2,4-diamine;
*N*²,*N*⁴-bis-(1*H*-indol-5-ylmethyl)-6-trifluoromethylpyrimidine-2,4-diamine;
*N*²-(2-(1H-indol-3-yl)ethyl)-N⁴-(1H-indol-5-ylmethyl)-6-trifluoromethylpyrimidine-2,4-diamine;
*N*²,*N*⁴*-*bis(1*H*-indol-5-ylmethyl)-6-benzylpyrimidine-2,4-diamine;
*N*⁴-(1*H*-indazol-5-ylmethyl)-*N*²-(1*H*-indol-4-yl)-6-methylpyrimidine-2,4-diamine;
*N*⁴-(1*H*-indol-5-ylmethyl)-*N*²-(1*H*-indol-4-yl)pyrimidine-2,4,5-triamine;
*N*⁴-(1*H*-indol-5-ylmethyl)-*N*²-(1*H*-indol-5-yl)pyrimidine-2,4,5-triamine;
*N*⁴-(1*H*-indol-5-ylmethyl)-*N*²-(1*H*-indol-6-yl)pyrimidine-2,4,5-triamine; and
*N*²,*N*⁴-bis(1*H*-indol-5-ylmethyl)pyrimidine-2,4,5-triamine.

In another aspect of the invention, there is provided a compound of formula I, for use in therapy.

In another aspect of the invention, there is provided a compound of formula I, for use in treatment of cancer.

In another aspect of the invention, there is provided use of a compound of formula I, in the manufacture of a medicament and pharmaceutical compositions for treatment of cancer.

In another aspect of the invention, there is provided a pharmaceutical composition comprising a compound of formula I, together with pharmaceutically acceptable diluents and carriers.

In another aspect of the invention, there is provided a method for treatment of cancer, which comprises administering to a subject in need thereof, a therapeutically effective amount of a compound of formula I.

In another aspect of the invention, there is provided a method for treatment of cancer, which comprises administering to a subject in need thereof, a therapeutically effective amount of a compound of formula I, in combination with another compound of formula I, in combination with radiation therapy, or in combination with another anticancer agent selected from alkylating agents, antimetabolites, anticancer camptothecin derivatives, plan-derived anticancer agents, antibiotics, enzymes, platinum coordination complexes, tyrosine kinase inhibitors, hormones, hormone antagonists, monoclonal antibodies, interferons, and biological response modifiers.

Further compounds related to the invention include, but are not limited to, the following compounds:
*N*²-(1*H*-indol-5-ylmethyl)-*N*⁴-(2-methyl-1*H*-benzo[d]imidazol-5-ylmethyl)pyrimidine-2,4-diamine;
*N*²-(1*H-*indazol-6-ylmethyl)-*N*⁴-(1*H*-indol-5-ylmethyl)pyrimidine-2,4-diamine;
*N*⁴-(1*H-*indazol-6-ylmethyl)-*N*²-(1*H*-indol-5-ylmethyl)pyrimidine-2,4-diamine;
4-[4-(1*H*-indol-5-ylmethylamino)pyrimidin-2-ylamino]indolin-2-one;
4-[6-(1*H*-indol-5-ylmethylamino)pyrimidin-2-ylamino]indolin-2-one;
5-{[4-(1*H*-indol-5-ylmethylamino)pyrimidin-2-ylamino]methyl}indolin-2-one;
5-{[2-(1*H*-indol-5-ylmethylamino)pyrimidin-4-ylamino]methyl}indolin-2-one;
6-{[4-(1*H*-indol-5-ylmethylamino)pyrimidin-2-ylamino]methyl}indolin-2-one;
6-{[2-(1*H*-indol-5-ylmethylamino)pyrimidin-4-ylamino]methyl}indolin-2-one;
*N*²-[2-(1*H-*benzo[d]imidazol-2-yl)ethyl]-*N*⁴-(1*H*-indol-5-ylmethyl)pyrimidine-2,4-diamine;
*N*⁴-[2-(1*H-*benzo[d]imidazol-2-yl)ethyl]-*N*²-(1*H*-indol-5-ylmethyl)pyrimidine-2,4-diamine;
*N*²-(2-methyl-1*H*-indol-5-yl)-*N*⁴-(2-methyl-1*H*-indol-5-ylmethyl)pyrimidine-2,4-diamine;
*N*⁴-(2-methyl-1*H*-indol-5-yl)-*N*²-(2-methyl-1*H*-indol-5-ylmethyl)pyrimidine-2,4-diamine;
*N*²-(1*H* benzo[*d*]imidazol-5-ylmethyl)-*N*⁴-(2-methyl-1*H* indol-5-ylmethyl)pyrimidine-2,4-diamine;
*N*⁴-(1*H* benzo[*d*]imidazol-5-ylmethyl)-*N*²-(2-methyl-1*H* indol-5-ylmethyl)pyrimidine-2,4-diamine;
*N*²-(2-methyl-1*H*-benzo[*d*]imidazol-5-ylmethyl)-*N*⁴-(2-methyl-1*H*-indol-5-ylmethyl)pyrimidine-2,4-diamine;
*N*⁴-(2-methyl-1*H*-benzo[*d*]imidazol-5-ylmethyl)-*N*²-(2-methyl-1*H*-indol-5-ylmethyl)pyrimidine-2,4-diamine;
*N*²-(1*H*-indol-6-ylmethyl)-*N*⁴-(2-methyl-1*H*-indol-5-ylmethyl)pyrimidine-2,4-diamine;
*N*⁴-(1*H*-indol-6-ylmethyl)-*N*²-(2-methyl-1*H*-indol-5-ylmethyl)pyrimidine-2,4-diamine;
3-{2-[4-(2-methyl-1*H*-indol-5-ylmethylamino)pyrimidin-2-ylamino]ethyl}-1*H*-indol-5-ol;
*N*²-[2-(5-methyl-1*H*-indol-3-yl]ethyl)-*N*⁴-(2-methyl-1*H*-indol-5-ylmethyl)pyrimidine-2,4-diamine;
*N*²-[2-(5-methoxy-1*H*-indol-3-yl)ethyl]-*N*⁴-(2-methyl-1*H*-indol-5-ylmethyl)pyrimidine-2,4-diamine;
4-[4-(2-methyl-1*H*-indol-5-ylmethylamino)pyrimidin-2-ylamino]indolin-2-one;
*N*⁴-(1*H-*indazol-5-ylmethyl)-*N*²-(2-methyl-1*H*-indol-5-yl)pyrimidine-2,4-diamine;
*N*²-(1*H*-indazol-5-ylmethyl)-*N*⁴-(2-methyl-1*H*-indol-5-yl)pyrimidine-2,4-diamine;
*N*⁴-(1*H*-indazol-5-ylmethyl)-*N*²-(1H-indol-5-ylmethyl)pyrimidine-2,4-diamine;
*N*⁴-(1*H-*indazol-5-ylmethyl)-N²-(2-methyl-1H-indol-5-ylmethyl)pyrimidine-2,4-diamine;
*N*²,*N*⁴-bis(1*H*-indazol-5-ylmethyl)pyrimidine-2,4-diamine;
*N*²-(1*H*-benzo[*d*]imidazol-5-ylmethyl)-*N*⁴-(1*H*-indazol-5-ylmethyl)pyrimidine-2,4-diamine;
*N*⁴-(1*H-*benzo[*d*]imidazol-5-ylmethyl)-*N*²-(1*H*-indazol-5-ylmethyl)pyrimidine-2,4-diamine;
*N*⁴-(1*H*-indazol-5-ylmethyl)-*N*²-(2-methyl-1*H*-benzo[*d*]imidazol-5-ylmethyl)pyrimidine-2,4-diamine;
*N*²-(1*H*-indazol-5-ylmethyl)-*N*⁴-(2-methyl-1H-benzo[*d*]imidazol-5-ylmethyl)pyrimidine-2,4-diamine;
*N*⁴-(1*H*-indazol-5-ylmethyl)-*N*²-(1*H*-indol-6-ylmethyl)pyrimidine-2,4-diamine;
*N*²-(1*H*-indazol-5-ylmethyl)-*N*⁴-(1*H*-indol-6-ylmethyl)pyrimidine-2,4-diamine;
*N*⁴-(1*H*-indazol-5-ylmethyl)-*N*²-[2-(1*H*-indol-3-yl)ethyl]pyrimidine-2,4-diamine;
*N*²-(1*H*-indazol-5-ylmethyl)-*N*⁴-[2-(1*H*-indol-3-yl)ethyl]pyrimidine-2,4-diamine;
3-{2-[4-(1*H*-indazol-5-ylmethylamino)pyrimidin-2-ylamino]ethyl}-1*H*-indol-5-ol;
3-{2-[2-(1*H*-indazol-5-ylmethylamino)pyrimidin-4-ylamino]ethyl}-1*H*-indol-5-ol;
*N*⁴-(1*H*-indazol-5-ylmethyl)-*N*²-[2-(5-methyl-1*H*-indol-3-yl)ethyl]pyrimidine-2,4-diamine;
*N*²-(1*H*-indazol-5-ylmethyl)-*N*⁴-[2-(5-methyl-1*H*-indol-3-yl)ethyl]pyrimidine-2,4-diamine;
*N*⁴*-*(1*H*-indazol-5-ylmethyl)-*N*²-[2-(5-methoxy-1*H*-indol-3-yl)ethyl]pyrimidine-2,4-diamine;
*N*²-(1*H*-indazol-5-ylmethyl)-*N*⁴-[2-(5-methoxy-1*H*-indol-3-yl)ethyl]pyrimidine-2,4-diamine;
4-[4-(1*H*-indazol-5-ylmethylamino)pyrimidin-2-ylamino]indolin-2-one;
5-{[4-(1*H*-indazol-5-ylmethylamino)pyrimidin-2-ylamino]methyl}indolin-2-one;
*N*²-[2-(1*H*-benzo[*d*]imidazol-2-yl)ethyl]-*N*⁴-(1*H*-indazol-5-ylmethyl)pyrimidine-2,4-diamine;
*N*⁴-(1*H*-benzo[*d*]imidazol-5-ylmethyl)-*N*²-(2-methyl-1*H*-indol-5-yl)pyrimidine-2,4-diamine;
*N*²-(1*H*-benzo[*d*]imidazol-5-ylmethyl)-*N*⁴-(2-methyl-1*H*-indol-5-yl)pyrimidine-2,4-diamine;
*N*⁴-(1*H*-benzo[*d*]imidazol-5-ylmethyl)-*N*²-(1*H*-indol-5-ylmethyl)pyrimidine-2,4-diamine;
*N*²,*N*⁴-bis(1*H*-benzo[*d*]imidazol-5-ylmethyl)pyrimidine-2,4-diamine;
*N*⁴-(1*H*-benzo[*d*]imidazol-5-ylmethyl)-*N*²-(2-methyl-1*H*-benzo[d]imidazol-5-ylmethyl)pyrimidine-2,4-diamine;
*N*²-(1*H*-benzo[*d*]imidazol-5-ylmethyl)-*N*⁴-(2-methyl-1*H*-benzo[d]imidazol-5-ylmethyl)pyrimidine-2,4-diamine;
*N*⁴-(1*H*-benzo[*d*]imidazol-5-ylmethyl)-*N*²-(1*H*-indol-6-ylmethyl)pyrimidine-2,4-diamine;
*N*²-(1*H*-benzo[d]imidazol-5-ylmethyl)-*N*⁴-(1*H*-indol-6-ylmethyl)pyrimidine-2,4-diamine;
*N*⁴-(1*H*-benzo[*d*]imidazol-5-ylmethyl)-*N*²-[2-(1*H*-indol-3-yl)ethyl]pyrimidine-2,4-diamine;
*N*²-(1*H*-benzo[*d*]imidazol-5-ylmethyl)-*N*⁴-[2-(1*H*-indol-3-yl)ethyl]pyrimidine-2,4-diamine;
3-{2-[4-(1*H*-benzo[*d*]imidazol-5-ylmethylamino)pyrimidin-2-ylamino]ethyl}-1*H-*indol-5-ol;
3-{2-[2-(1*H-*benzo[*d*]imidazol-5-ylmethylamino)pyrimidin-4-ylamino]ethyl}-1*H-*indol-5-ol;
*N*⁴-(1*H*-benzo[*d*]imidazol-5-ylmethyl)-*N*²-[2-(5-methyl-1*H*-indol-3-yl)ethyl]pyrimidine-2,4-diamine;
*N*²-(1*H*-benzo[*d*]imidazol-5-ylmethyl)-*N*⁴-[2-(5-methyl-1*H*-indol-3-yl)ethyl]pyrimidine-2,4-diamine;
*N*⁴-(1*H*-benzo[*d*]imidazol-5-ylmethyl)-*N*²-[2-(5-methoxy-1*H*-indol-3-yl)ethyl]pyrimidine-2,4-diamine;
*N*²-(1*H*-benzo[*d*]imidazol-5-ylmethyl)-*N*⁴-[2-(5-methoxy-1*H*-indol-3-yl)ethyl]pyrimidine-2,4-diamine;
4-[4-(1*H*-benzo[*d*]imidazol-5-ylmethylamino)pyrimidin-2-ylamino]indolin-2-one;
*N*²-(1*H*-indol-4-yl)-*N*⁴-(2-methyl-1*H*-benzo[*d*]imidazol-5-ylmethyl)pyrimidine-2,4-diamine;
*N*⁴-(2-methyl-1*H*-benzo[*d*]imidazol-5-ylmethyl)-*N*²-(2-methyl-1*H*-indol-5-yl)pyrimidine-2,4-diamine;
*N*²-(2-methyl-1*H*-benzo[*d*]imidazol-5-ylmethyl)-*N*⁴-(2-methyl-1*H*-indol-5-yl)pyrimidine-2,4-diamine;
*N*²,*N*⁴-bis(2-methyl-1*H*-benzo[*d*]imidazol-5-ylmethyl)pyrimidine-2,4-diamine;
*N*²-(1*H*-indol-6-ylmethyl)-*N*⁴-(2-methyl-1*H*-benzo[*d*]imidazol-5-ylmethyl)pyrimidine-2,4-diamine;
*N*⁴-(1*H*-indol-6-ylmethyl)-*N*²-(2-methyl-1*H*-benzo[*d*]imidazol-5-ylmethyl)pyrimidine-2,4-diamine;
*N*²-[2-(1*H*-indol-3-yl)ethyl]-*N*⁴-(2-methyl-1*H*-benzo[*d*]imidazol-5-ylmethyl)pyrimidine-2,4-diamine;
*N*⁴-[2-(1*H*-indol-3-yl)ethyl]-*N*²-(2-methyl-1*H*-benzo[*d*]imidazol-5-ylmethyl)pyrimidine-2,4-diamine;
3-{2-[4-(2-methyl-1*H*-benzo[*d*]imidazol-5-ylmethylamino)pyrimidin-2-ylamino]ethyl}-1*H*-indol-5-ol;
3-{2-[2-(2-methyl-1*H*-benzo[*d*]imidazol-5-ylmethylamino)pyrimidin-4-ylamino]ethyl}-1*H*-indol-5-ol;
*N*⁴-(2-methyl-1*H*-benzo[*d*]imidazol-5-ylmethyl)-*N*²-[2-(5-methyl-1*H*-indol-3-yl)ethyl]pyrimidine-2,4-diamine;
*N*²-(2-methyl-1*H*-benzo[*d*]imidazol-5-ylmethyl)-*N*⁴-[2-(5-methyl-1*H*-indol-3-yl)ethyl]pyrimidine-2,4-diamine;
*N*²-[2-(5-methoxy-1*H*-indol-3-yl)ethyl]-*N*⁴-(2-methyl-1*H*-benzo[*d*]imidazol-5-ylmethyl)pyrimidine-2,4-diamine;
*N*⁴-[2-(5-methoxy-1*H*-indol-3-yl)ethyl]-*N*²-(2-methyl-1*H*-benzo[*d*]imidazol-5-ylmethyl)pyrimidine-2,4-diamine;
4-[4-(2-methyl-1*H*-benzo[*d*]imidazol-5-ylmethylamino)pyrimidin-2-ylamino]indolin-2-one;
*N*⁴-(1*H-*indol-6-ylmethyl)-*N*²-(2-methyl-1*H*-indol-5-yl)pyrimidine-2,4-diamine;
*N*²-(1*H*-indol-6-ylmethyl)-*N*⁴-(2-methyl-1*H*-indol-5-yl)pyrimidine-2,4-diamine;
*N*²-[2-(1*H-*indol-3-yl)ethyl]-*N*⁴-(1*H*-indol-6-ylmethyl)pyrimidine-2,4-diamine;
*N*⁴-[2-(1*H-*indol-3-yl)ethyl]-*N*²-(1*H*-indol-6-ylmethyl)pyrimidine-2,4-diamine;
3-{2-[4-(1*H*-indol-6-ylmethylamino)pyrimidin-2-ylamino]ethyl}-1*H*-indol-5-ol;
3-{2-[2-(1*H*-indol-6-ylmethylamino)pyrimidin-4-ylamino]ethyl}-1*H*-indol-5-ol;
*N*⁴-(1*H*-indol-6-ylmethyl)-*N*²-[2-(5-methyl-1*H*-indol-3-yl)ethyl]pyrimidine-2,4-diamine;
*N*²-(1*H*-indol-6-ylmethyl)-*N*⁴-[2-(5-methyl-1*H*-indol-3-yl)ethyl]pyrimidine-2,4-diamine;
*N*⁴-(1*H*-indol-6-ylmethyl)-*N*²-[2-(5-methoxy-1*H*-indol-3-yl)ethyl]pyrimidine-2,4-diamine;
*N*²-(1*H*-indol-6-ylmethyl)-*N*⁴-[2-(5-methoxy-1*H*-indol-3-yl)ethyl]pyrimidine-2,4-diamine;
4-[4-(1*H*-indol-6-ylmethylamino)pyrimidin-2-ylamino]indolin-2-one;
5-{[4-(1*H*-indol-6-ylmethylamino)pyrimidin-2-ylamino]methyl}indolin-2-one;
5-{[2-(1*H*-indol-6-ylmethylamino)pyrimidin-4-ylamino]methyl}indolin-2-one;
6-{[4-(1*H*-indol-6-ylmethylamino)pyrimidin-2-ylamino]methyl}indolin-2-one;
6-{[2-(1*H*-indol-6-ylmethylamino)pyrimidin-4-ylamino]methyl}indolin-2-one;
*N*²-(1*H*-indol-4-yl)-*N*⁴-(2-methyl-1*H*-indol-5-ylmethyl)-6-methylpyrimidine-2,4-diamine;
*N*⁴-(1*H*-benzo[d]imidazol-5-ylmethyl)-*N*²-(1*H*-indol-4-yl)-6-methylpyrimidine-2,4-diamine;
*N*²-(1*H-*indol-4-yl)-*N*⁴-(2-methyl-1*H*-benzo[d]imidazol-5-ylmethyl)-6-methylpyrimidine-2,4-diamine;
*N*²-(1*H-*indol-4-yl)-*N*⁴-(1*H*-indol-6-ylmethyl)-6-methylpyrimidine-2,4-diamine;
4-[4-(1*H*-indol-5-ylmethylamino)-6-methylpyrimidin-2-ylamino]indolin-2-one;
4-[4-(1*H*-indazol-5-ylmethylamino)-6-methylpyrimidin-2-ylamino]indolin-2-one;
4-[4-(1*H*-benzo[d]imidazol-5-ylmethylamino)-6-methylpyrimidin-2-ylamino]indolin-2-one;
4-[4-(2-methyl-1*H*-benzo[d]imidazol-5-ylmethylamino)-6-methylpyrimidin-2-ylamino]indo lin-2-one;
4-[4-(1*H*-indol-6-ylmethylamino)-6-methylpyrimidin-2-ylamino]indolin-2-one
*N*⁴-(1*H-*indol-5-ylmethyl)-*N*²-[2-(5-ethoxy-1*H*-indol-3-yl)ethyl]pyrimidine-2,4-diamine;
*N*²-(1*H-*indol-5-ylmethyl)-*N*⁴-[2-(5-ethoxy-1*H*-indol-3-yl)ethyl]pyrimidine-2,4-diamine;
*N*⁴-(1*H*-indol-5-ylmethyl)-*N*²-[2-(5-isobutoxy-1*H*-indol-3-yl)ethyl]pyrimidine-2,4-diamine;
*N*²-(1*H*-indol-5-ylmethyl)-*N*⁴-[2-(5-isobutoxy-1*H*-indol-3-yl)ethyl]pyrimidine-2,4-diamine
*N*⁴-(1*H*-indol-5-ylmethyl)-*N*²-{2-[5-(benzyloxy)-1*H*-indol-3-yl]ethyl}pyrimidine-2,4-diamine;
*N*²-(1*H-*indol-5-ylmethyl)-*N*⁴-{2-[5-(benzyloxy)-1*H*-indol-3-yl]ethyl}pyrimidine-2,4-diamine;
*N*⁴-(1*H*-indol-5-ylmethyl)-*N*²-{2-[5-(2-morpholinoethoxy)-1*H*-indol-3-yl]ethyl}pyrimidine-2,4-diamine;
*N*²-(1*H*-indol-5-ylmethyl)-*N*⁴-{2-[5-(2-morpholinoethoxy)-1*H*-indol-3-yl]ethyl}pyrimidine-2,4-diamine;
2-(3-{2-[4-(1*H*-indol-5-ylmethylamino)pyrimidin-2-ylamino]ethyl}-1*H*-indol-5-yloxy)ethanol;
*N*⁴-(1*H*-indol-5-ylmethyl)-*N*²-[2-(4-methoxy-1*H*-indol-3-yl)ethyl]pyrimidine-2,4-diamine;
*N*²-(1*H*-indol-5-ylmethyl)-*N*⁴-[2-(4-methoxy-1*H*-indol-3-yl)ethyl]pyrimidine-2,4-diamine;
*N*⁴-(1*H*-indol-5-ylmethyl)-*N*²-[2-(6-methoxy-1*H*-indol-3-yl)ethyl]pyrimidine-2,4-diamine;
*N*²-(1*H*-indol-5-ylmethyl)-*N*⁴-[2-(6-methoxy-1*H*-indol-3-yl)ethyl]pyrimidine-2,4-diamine;
*N*⁴-(1*H*-indol-5-ylmethyl)-*N*²-[2-(5,7-dimethoxy-1*H*-indol-3-yl)ethyl]pyrimidine-2,4-diamine
*N*²-(1*H*-indol-5-ylmethyl)-*N*⁴-[2-(5,7-dimethoxy-1*H*-indol-3-yl)ethyl]pyrimidine-2,4-diamine;
*N*⁴-(1*H*-indol-5-ylmethyl)-*N*²-[2-(5-methoxy-1*H*-indol-3-yl)ethyl]-*N*²-methylpyrimidine-2,4-diamine;
*N*⁴-(1*H*-indol-5-ylmethyl)-*N*²-[2-(5-methoxy-1-methyl-1*H*-indol-3-yl)ethyl]pyrimidine-2,4-diamine;
*N*⁴-(1*H*-indol-5-ylmethyl)-*N*²-[2-(5-phenoxy-1*H*-indol-3-yl)ethyl]pyrimidine-2,4-diamine;
*N*⁴-(1*H*-indol-5-ylmethyl)-*N*²-[2-(5-amino-1*H*-indol-3-yl)ethyl]pyrimidine-2,4-diamine;
*N*⁴-(1*H* indol-5-ylmethyl)-*N*²-{2-[5-(methylamino)-1*H*-indol-3-yl]ethyl}pyrimidine-2,4-diamine;
*N*⁴-(1*H*-indol-5-ylmethyl)-*N*²-{2-[5-(dimethylamino)-1*H*-indol-3-yl]ethyl}pyrimidine-2,4-diamine;
*N*²-(1*H*-indol-5-ylmethyl)-*N*⁴-{2-[5-(dimethylamino)-1*H*-indol-3-yl]ethyl}pyrimidine-2,4-diamine;
3-{2-[4-(1*H*-indol-5-ylmethylamino)pyrimidin-2-ylamino]ethyl}-1*H*-indole-5-carbonitrile;
3-{2-[4-(1*H*-indol-5-ylmethylamino)pyrimidin-2-ylamino] ethyl}-1*H*-indol-5-yl hydrogen sulfate;
*N*-(3-{2-[4-(1*H*-indol-5-ylmethylamino)pyrimidin-2-ylamino]ethyl}-7-methoxy-1*H* indol-5-yl)acetamide;
1-(3-{2-[4-(1*H*-indol-5-ylmethylamino)pyrimidin-2-ylamino]ethyl}-1*H*-indol-5-yl)ethanone;
*N*⁴-(1*H*-indol-5-ylmethyl)-*N*²-{2-[5-(2-methoxyethoxy)-1*H*-indol-3-yl]ethyl}pyrimidine-2,4-diamine;
*N*²-(1*H*-indol-5-ylmethyl)-*N*⁴-{2-[5-(2-methoxyethoxy)-1*H*-indol-3-yl]ethyl}pyrimidine-2,4-diamine;
*N*⁴-(1*H*-indol-5-ylmethyl)-*N*²-(2-{5-[2-(2-methoxyethoxy)ethoxy]-1*H*-indol-3-yl}ethyl)pyrimidine-2,4-diamine;
*N*⁴-(1*H*-indol-5-ylmethyl)-*N*²-[2-(5-butyl-1*H*-indol-3-yl)ethyl]pyrimidine-2,4-diamine;
*N²*-(1*H-*indol-5-ylmethyl)-*N*⁴-[2-(5-butyl-1*H*-indol-3-yl)ethyl]pyrimidine-2,4-diamine;
3-{2-[4-(1*H*-indol-5-ylmethylamino)pyrimidin-2-ylamino]ethyl}-1*H*-indol-4-ol;
3{2-[2-(1*H*-indol-5-ylmethylamino)pyrimidin-4-ylamino]ethyl}-1*H*-indol-4-ol;
3-{2-[4-(1*H*-indol-5-ylmethylamino)pyrimidin-2-ylamino]ethyl}-1*H*-indol-6-ol;
3-{2-[2-(1*H-*indol-5-ylmethylamino)pyrimidin-4-ylamino]ethyl}-1*H*-indol-6-ol;
*N*⁴-(1*H-*indol-5-ylmethyl)-*N*²-[2-(5-nitro-1*H*-indol-3-yl)ethyl]pyrimidine-2,4-diamine;
*N*⁴-(1*H-*indol-5-ylmethyl)-*N*²-[2-(5-fluoro-1*H*-indol-3-yl)ethyl]pyrimidine-2,4-diamine;
3-(2-(4-((1*H*-indol-5-yl)methylamino)pyrimidin-2-ylamino)ethyl)-1*H*-indole-5-carboxamide;
*N*⁴-(1*H*-indol-5-ylmethyl)-*N*²-[2-(6-fluoro-1*H*-indol-3-yl)ethyl]pyrimidine-2,4-diamine;
*N*⁴-(1*H*-indol-5-ylmethyl)-*N*²-[2-(5-phenyl-1*H*-indol-3-yl)ethyl]pyrimidine-2,4-diamine;
*N*⁴-(1*H*-indol-5-ylmethyl)-*N*²-{2-[5-(aminomethyl)-1*H*-indol-3-yl]ethyl}pyrimidine-2,4-diamine;
*N*⁴-(1*H*-indol-5-ylmethyl)-*N*²-(2-{5-[(methylamino)methyl]-1*H*-indol-3-yl}ethyl)pyrimidine-2,4-diamine;
*N*⁴-(1*H*-indol-5-ylmethyl)-*N*²-(2-{5-[(dimethylamino)methyl]-1*H*-indol-3-yl}ethyl)pyrimidine-2,4-diamine;
*N*²-(1*H-*indol-5-ylmethyl)-*N*⁴-(2-{5-[(dimethylamino)methyl]-1*H*-indol-3-yl}ethyl)pyrimidine-2,4-diamine;
*N*²-[2-(1*H-*indol-2-yl)ethyl]-*N*⁴-(1*H*-indol-5-ylmethyl)pyrimidine-2,4-diamine;
*N*⁴-[2-(1*H-*indol-2-yl)ethyl]-*N*²-(1*H*-indol-5-ylmethyl)pyrimidine-2,4-diamine;
*N*²-[(1*H-*indol-3-yl)methyl]-*N*⁴-(1*H*-indol-5-ylmethyl)pyrimidine-2,4-diamine;
*N*⁴-[(1*H-*indol-3-yl)methyl]-*N*²-(1*H*-indol-5-ylmethyl)pyrimidine-2,4-diamine;
*N*⁴-(1*H-*indol-5-ylmethyl)-*N*²-(2-methyl-1*H*-indol-4-yl)pyrimidine-2,4-diamine;
*N*⁴-(1*H-*indol-5-ylmethyl)-6-methyl-*N*²-(2-methyl-1*H*-indol-4-yl)pyrimidine-2,4-diamine;
*N*⁴-(1*H-*indol-5-ylmethyl)-*N*²-(3-methyl-1*H*-indol-4-yl)pyrimidine-2,4-diamine;
*N*⁴-(1*H*-indol-5-ylmethyl)-*N*²-(6-methyl-1*H*-indol-4-yl)pyrimidine-2,4-diamine;
*N*⁴-(1*H-*indol-5-ylmethyl)-*N*²-(7-methyl-1*H*-indol-4-yl)pyrimidine-2,4-diamine;
4-[4-(1*H*-indol-5-ylmethylamino)pyrimidin-2-ylamino]-1*H*-indole-2-carbonitrile;
4-[4-(1*H*-indol-5-ylmethylamino)pyrimidin-2-ylamino]-1*H*-indole-6-carbonitrile;
*N*⁴-(1*H-*indol-5-ylmethyl)-*N*²-(5-methoxy-1*H*-indol-4-yl)pyrimidine-2,4-diamine;
*N*⁴-(1*H-*indol-5-ylmethyl)-*N*²-(6-methoxy-1*H*-indol-4-yl)pyrimidine-2,4-diamine;
*N*⁴-(1*H-*indol-5-ylmethyl)-*N*²-(6-methoxy-1*H*-indol-4-yl)-6-methylpyrimidine-2,4-diamine;
*N*⁴-(1*H-*indol-5-ylmethyl)-*N*²-(6-fluoro-1*H*-indol-4-yl)pyrimidine-2,4-diamine;
*N*⁴-(1*H-*indol-5-ylmethyl)-*N*²-(6-chloro-1*H*-indol-4-yl)pyrimidine-2,4-diamine;
*N*⁴*-*(1*H-*indol-5-ylmethyl)-*N*²-(6-nitro-1*H*-indol-4-yl)pyrimidine-2,4-diamine;
4-[4-(1*H*-indol-5-ylmethylamino)pyrimidin-2-ylamino]-1*H*-indole-2-carboxylic acid;
4-[4-(1*H*-indol-5-ylmethylamino)pyrimidin-2-ylamino]-1*H*-indole-6-carboxylic acid;
1-{4-[4-(1*H*-indol-5-yl)methylamino]pyrimidin-2-ylamino}-1*H*-indol-3-yl)ethanone;
4-[4-(1*H*-indol-5-ylmethylamino)pyrimidin-2-ylamino]-1*H-*indole-3-carboxylic acid;
2-{4-[4-(1*H*-indol-5-ylmethylamino)pyrimidin-2-ylamino]-1*H*-indol-3-yl}acetic acid;
2-{4-[4-(1*H*-indol-5-ylmethylamino)pyrimidin-2-ylamino]-1*H*-indol-3-yl}acetonitrile;
*N*⁴-(1*H-*indol-5-ylmethyl)-*N*²-(2-phenyl-1*H*-indol-4-yl)pyrimidine-2,4-diamine;
*N*⁴-(1*H-*indol-5-ylmethyl)-*N*²-(6-phenyl-1*H*-indol-4-yl)pyrimidine-2,4-diamine;
*N*⁴-(1*H-*indol-5-ylmethyl)-*N*²-[6-(benzyloxy)-1*H*-indol-4-yl]pyrimidine-2,4-diamine;
*N*⁴-(1*H-*indol-5-ylmethyl)-*N*²-[6-(2-morpholinoethoxy)-1*H*-indol-4-yl]pyrimidine-2,4-diamine;
*N*⁴-(1*H-*indol-5-ylmethyl)-*N*²-[6-(2-methoxyethoxy)-1*H*-indol-4-yl]pyrimidine-2,4-diamine;
*N*⁴-[4-(1*H-*indol-5-ylmethylamino)pyrimidin-2-yl]-1*H*-indole-4,6-diamine;
*N*⁴-[4-(1*H-*indol-5-ylmethylamino)pyrimidin-2-yl]-*N*⁶-methyl-1*H*-indole-4,6-diamine;
*N*⁴-[4-(1*H-*indol-5-ylmethylamino)pyrimidin-2-yl]-*N*⁶,*N*⁶-dimethyl-1*H*-indole-4,6-diamine;
*N*⁴-[4-(1*H-*indol-5-ylmethylamino)-6-methylpyrimidin-2-yl]-*N*⁶,*N*⁶-dimethyl-1*H-*indole-4,6-diamine;
*N*⁴-(1*H-*indol-5-ylmethyl)-*N*²-[6-(aminomethyl)-1*H*-indol-4-yl]pyrimidine-2,4-diamine;
*N*⁴-(1*H*-indol-5-ylmethyl)-*N*²-{6-[(methylamino)methyl]-1*H*-indol-4-yl}pyrimidine-2,4-diamine;
*N*⁴-(1*H*-indol-5-ylmethyl)-*N*²-{6-[(dimethylamino)methyl]-1*H*-indol-4-yl}pyrimidine-2,4-diamine;
4-[4-(1*H*-indol-5-ylmethylamino)pyrimidin-2-ylamino]-1*H*-indol-5-ol;
4-[4-(1*H*-indol-5-ylmethylamino)-6-methylpyrimidin-2-ylamino]-1*H*-indol-5-ol;
*N*⁴-(1*H-*indol-5-ylmethyl)-*N*²-[5-(benzyloxy)-1*H*-indol-4-yl]pyrimidine-2,4-diamine;
*N*⁴-(1*H*-indol-5-ylmethyl)-*N*²-[5-(2-morpholinoethoxy)-1*H*-indol-4-yl]pyrimidine-2,4-diamine;
*N*⁴-(1*H-*indol-5-ylmethyl)-*N*²-[5-(2-methoxyethoxy)-1*H*-indol-4-yl]pyrimidine-2,4-diamine;
*N*⁴-(1*H-*indol-5-ylmethyl)-*N*²-[2-(aminomethyl)-1*H*-indol-4-yl]pyrimidine-2,4-diamine;
*N*⁴-(1*H*-indol-5-ylmethyl)-*N*²-{2-[(methylamino)methyl]-1*H*-indol-4-yl}pyrimidine-2,4-diamine;
*N*⁴-(1*H*-indol-5-ylmethyl)-*N*²-{2-[(dimethylamino)methyl]-1*H*-indol-4-yl}pyrimidine-2,4-diamine;
*N*⁴-(1*H-*indol-5-ylmethyl)-*N*²-[3-(2-aminoethyl)-1*H*-indol-4-yl]pyrimidine-2,4-diamine;
*N*⁴-(1*H-*indol-5-ylmethyl)-*N*²-{3-[2-(methylamino)ethyl]-1*H*-indol-4-yl}pyrimidine-2,4-diamine;
*N*⁴-(1*H*-indol-5-ylmethyl)-*N*²-{3-[2-(dimethylamino)ethyl]-1*H*-indol-4-yl}pyrimidine-2,4-diamine;
*N*⁴-(1*H-*indol-5-ylmethyl)-*N*²-(1*H*-indol-4-yl)-*N*²-methylpyrimidine-2,4-diamine;
*N*⁴-(1*H-*indol-5-ylmethyl)-*N*²-(1-methyl-1*H*-indol-4-yl)pyrimidine-2,4-diamine;
*N*⁴-(1*H-*indol-5-ylmethyl)-*N*²-(1*H*-indazol-4-yl)pyrimidine-2,4-diamine;
*N*⁴-(1*H-*indol-5-ylmethyl)-*N*²-(1*H*-indazol-4-yl)-6-methylpyrimidine-2,4-diamine;
*N*⁴-(1*H-*indol-5-ylmethyl)-*N*²-(6-methyl-1*H*-indazol-4-yl)pyrimidine-2,4-diamine;
*N*⁴-(1*H-*indol-5-ylmethyl)-*N*²-(7-methyl-1*H*-indazol-4-yl)pyrimidine-2,4-diamine;
*N*⁴-(1*H-*indol-5-ylmethyl)-*N*²-(6-methoxy-1*H*-indazol-4-yl)pyrimidine-2,4-diamine;
4-[4-(1*H*-indol-5-ylmethylamino)pyrimidin-2-ylamino]-1*H*-indazol-6-ol;
4-[4-(1*H*-indol-5-ylmethylamino)pyrimidin-2-ylamino]-1*H*-indazole-6-carboxylic acid;
*N*⁴-[4-(1*H-*indol-5-ylmethylamino)pyrimidin-2-yl]-1*H*-indazole-4,7-diamine; *N*²*,N*⁴-bis(1*H-*indol-5-ylmethyl)-*N*²-methylpyrimidine-2,4-diamine;
*N*⁴-(1*H-*indol-5-ylmethyl)-*N*²-[(1-methyl-1*H*-indol-5-yl)methyl]pyrimidine-2,4-diamine;
*N*⁴-(1*H-*indol-5-ylmethyl)-*N*²-[(4-methyl-1*H*-indol-5-yl)methyl]pyrimidine-2,4-diamine;
*N*⁴*-(1H-*indol-5-ylmethyl)-N²-[(7-methyl-1H-indol-5-yl)methyl]pyrimidine-2,4-diamine;
*N*⁴-(1*H-*indol-5-ylmethyl)-*N*²-({3-[(dimethylamino)methyl]-1*H*-indol-5-yl}methyl)pyrimidine-2,4-diamine;
1-(5-{[4-(1*H*-indol-5-ylmethylamino)pyrimidin-2-ylamino]methyl}-1*H*-indol-3-yl)ethanone;
5-{[4-(1*H*-indol-5-ylmethylamino)pyrimidin-2-ylamino]methyl}-1*H*-indole-3-carboxylic acid;
5-{[4-(1*H*-indol-5-ylmethylamino)pyrimidin-2-ylamino]methyl}-*N*-methyl-1*H-*indole-3-carboxamide;
2-(5-{[4-(1*H*-indol-5-ylmethylamino)pyrimidin-2-ylamino]methyl}-1*H*-indol-3-yl)acetic acid;
2-(5-{[4-(1*H*-indol-5-ylmethylamino)pyrimidin-2-ylamino]methyl}-1*H*-indol-3-yl)-*N*-ethylacetamide;
*N*⁴-(1*H*-indol-5-ylmethyl)-*N*²-({2-[(dimethylamino)methyl]-1*H*-indol-5-yl}methyl)pyrimidine-2,4-diamine;
*N*⁴-(1*H-*indol-5-ylmethyl)-*N*²-{[2-(morpholinomethyl)-1*H*-indol-5-yl]methyl]pyrimidine-2,4-diamine;
*N*⁴-(1*H-*indol-5-ylmethyl)-*N*²-{[2-(piperazin-1-ylmethyl)-1*H*-indol-5-yl]methyl}pyrimidine-2,4-diamine;
*N*⁴-(1*H-*indol-5-ylmethyl)-*N*²-[(2-benzyl-1*H-*indol-5-yl)methyl]pyrimidine-2,4-diamine;
(5-{[4-(1*H*-indol-5-ylmethylamino)pyrimidin-2-ylamino]methyl}-1*H*-indol-2-yl)methanol;
*N*⁴-(1*H-*indol-5-ylmethyl)-*N*²-[2-(phenoxymethyl)-1*H*-indol-5-yl]methyl}pyrimidine-2,4-diamine;
*N*⁴-(1*H-*indol-5-ylmethyl)-*N*²-[(3-nitro-1*H*-indol-5-yl)methyl]pyrimidine-2,4-diamine;
*N*⁴-(1*H-*indol-5-ylmethyl)-*N*²-{[3-(2-aminoethyl)-1*H*-indol-5-yl]methyl}pyrimidine-2,4-diamine;
*N*⁴-(1*H-*indol-5-ylmethyl)-*N*²-({3-[2-(methylamino)ethyl]-1*H*-indol-5-yl}methyl)pyrimidine-2,4-diamine; and
*N⁴*-(1*H*-indol-5-ylmethyl)-*N*²-({3-[2-(dimethylamino)ethyl]-1*H*-indol-5-yl}methyl)pyrimidine-2,4-diamine; and
a pharmaceutically acceptable ester, amide, solvate or salt thereof.

In the list above and in the Examples, the compound names were generated in accordance with IUPAC by ChemBioDraw Ultra version 11.0.

Depending upon the substituents present in compounds of the formula I, the compounds may form esters, amides and/or salts which are within the scope of the present invention. Salts and solvates of compounds of formula I which are suitable for use in medicine are those wherein a counterion or an associated solvent is pharmaceutically acceptable. However, salts and solvates having non-pharmaceutically acceptable counterions or associated solvents are within the scope of the present invention, for example, for use as intermediates in the preparation of the compounds of formula I and their pharmaceutically acceptable salts, solvates and physiologically functional derivatives. By the term "physiologically functional derivative" is meant a chemical derivative of a compound of formula I having the same physiological function as the free compound of formula I, for example, by being convertible in the body thereto. Esters and amides are examples of physiologically functional derivatives.

A compound which, upon administration to the recipient, is capable of being converted into a compound of formula I as described above, or an active metabolite or residue thereof, is known as a "prodrug". A prodrug may, for example, be converted within the body, e. g. by hydrolysis in the blood, into its active form that has medical effects. Pharmaceutical acceptable prodrugs are described in T. Higuchi and V. Stella, Prodrugs as Novel Delivery Systems, Vol. 14 of the A. C. S. Symposium Series (1976); "Design of Prodrugs" ed. H. Bundgaard, Elsevier, 1985; and in Edward B. Roche, ed., Bioreversible Carriers in Drug Design, American Pharmaceutical Association and Pergamon Press, 1987, which are incorporated herein by reference.

Suitable salts according to the invention include those formed with organic or inorganic acids or bases. In particular, suitable salts formed with acids according to the invention include those formed with mineral acids, strong organic carboxylic acids, such as alkanecarboxylic acids of 1 to 4 carbon atoms which are unsubstituted or substituted, for example, by halogen, such as saturated or unsaturated dicarboxylic acids, such as hydroxycarboxylic acids, such as amino acids, or with organic sulfonic acids, such as (C₁-C₄)alkyl- or aryl-sulfonic acids which are unsubstituted or substituted, for example by halogen. Pharmaceutically acceptable acid addition salts include those formed from hydrochloric, hydrobromic, sulphuric, nitric, citric, tartaric, acetic, phosphoric, lactic, pyruvic, acetic, trifluoroacetic, succinic, perchloric, fumaric, maleic, glycolic, lactic, salicylic, oxaloacetic, methanesulfonic, ethanesulfonic, p-toluenesulfonic, formic, benzoic, malonic, naphthalene-2-sulfonic, benzenesulfonic, isethionic, ascorbic, malic, phthalic, aspartic, and glutamic acids, lysine and arginine. Other acids such as oxalic, while not in themselves pharmaceutically acceptable, may be useful as intermediates in obtaining the compounds of the invention and their pharmaceutical acceptable acid addition salts.

Pharmaceutically acceptable base salts include ammonium salts, alkali metal salts, for example those of potassium and sodium, alkaline earth metal salts, for example those of calcium and magnesium, and salts with organic bases, for example dicyclohexylamine, *N*-methyl-D-glucamine, morpholine, thiomorpholine, piperidine, pyrrolidine, a mono-, di- or tri-lower alkylamine, for example ethyl-, tert-butyl-, diethyl-, diisopropyl-, triethyl-, tributyl- or dimethyl-propylamine, or a mono-, di- or trihydroxy lower alkylamine, for example mono-, di- or triethanolamine. Corresponding internal salts may furthermore be formed.

Those skilled in the art of organic chemistry will appreciate that many organic compounds can form complexes with solvents in which they are reacted or from which they are precipitated or crystallized. These complexes are known as "solvates". For example, a complex with water is known as a "hydrate".

The following definitions apply to the terms as used throughout this specification, unless otherwise limited in specific instances.

As used herein, the term "alkyl" means both straight and branched chain saturated hydrocarbon groups. Examples of alkyl groups include methyl, ethyl, n-propyl, iso-propyl, n-butyl, t-butyl, iso-butyl, and sec-butyl groups. Among unbranched alkyl groups, there are preferred methyl, ethyl, n-propyl, and n-butyl groups. Among branched alkyl groups, there may be mentioned iso-propyl, t-butyl, and iso-butyl groups.

As used herein, the term "alkoxy" means the group O-alkyl, where "alkyl" is used as described above. Examples of alkoxy groups include, but are not limited to, methoxy and ethoxy groups. Other examples include propoxy and butoxy.

As used herein, the term "aryl" means a monocyclic or bicyclic aromatic carbocyclic group. Examples of aryl groups include phenyl and naphthyl. A naphthyl group may be attached through the 1 or the 2 position. In a bicyclic aromatic group, one of the rings may, for example, be partially saturated. Examples of such groups include indanyl and tetrahydronaphthyl. Specifically, the term (C₆-C₁₀)aryl is used herein to mean a group comprising from 6 to 10 carbon atoms in a monocyclic or bicyclic aromatic group. A particularly preferred (C₆-C₁₀)aryl group is phenyl.

As used herein, the term "halogen" means fluorine, chlorine, bromine or iodine. Fluorine, chlorine and bromine are particularly preferred.

As used herein, the term "heteroaryl" means an aromatic cyclic group of carbon atoms wherein from one to three of the carbon atoms is/are replaced by one or more heteroatoms independently selected from nitrogen, oxygen or sulfur. A heteroaryl group may, for example, be monocyclic, bicyclic or tricyclic.

Examples of monocyclic heteroaryl groups include, but are not limited to, furanyl, thienyl, pyrrolyl, oxazolyl, thiazolyl, imidazolyl, oxadiazolyl, thiadiazolyl, pyridyl, triazolyl, triazinyl, pyridazyl, isothiazolyl, isoxazolyl, pyrazinyl, pyrazolyl, and pyrimidinyl.

Examples of bicyclic heteroaryl groups include, but are not limited to, quinoxalinyl, quinazolinyl, pyridopyrazinyl, benzoxazolyl, benzothiophenyl, benzimidazolyl, naphthyridinyl, quinolinyl, benzofuranyl, indolyl, indazolyl, benzothiazolyl, pyridopyrimidinyl, and isoquinolinyl.

Examples of tricyclic heteroaryl groups include, but are not limited to, carbazole, dibenzofuran, xanthene, and acridine.

As used herein, the term "heterocyclyl" means a cyclic group of carbon atoms wherein from one to three of the carbon atoms is/are replaced by one or more heteroatoms independently selected from nitrogen, oxygen and sulfur.

Examples of heterocyclyl groups include, but are not limited to, tetrahydrofuranyl, pyrrolidinyl, piperidinyl, piperazinyl, morpholinyl, and dioxanyl.

The compounds of the invention may be used in the prophylaxis and treatment as such, or preferably in a form of a pharmaceutical composition. While it is possible for the active ingredient to be administered alone, it is preferable for it to be present in a pharmaceutical formulation or composition. Accordingly, the invention provides a pharmaceutical formulation comprising a compound according to the invention, and a pharmaceutically acceptable diluent, excipient or carrier (collectively referred to herein as "carrier" materials). Pharmaceutical compositions of the invention may take the form of a pharmaceutical formulation as described below. Thus, the present invention relates to a pharmaceutical composition containing at least one compound of Formula I together with conventional excipients.

Exemplary compositions for oral administration include suspensions which can contain, for example, microcrystalline cellulose for imparting bulk, alginic acid or sodium alginate as a suspending agent, methylcellulose as a viscosity enhancer, and sweeteners or flavoring agents such as those known in the art; and immediate release tablets which can contain, for example, microcrystalline cellulose, dicalcium phosphate, starch, magnesium stearate, calcium sulfate, sorbitol, glucose and/or lactose and/or other excipients, binders, extenders, disintegrants, diluents and lubricants such as those known in the art. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Disintegrators include without limitation starch, methylcellulose, agar, bentonite, xanthan gum and the like. The compounds of formula I can also be delivered through the oral cavity by sublingual and/or buccal administration. Molded tablets, compressed tablets or freeze-dried tablets are exemplary forms which may be used. Exemplary compositions include those formulating the present compound(s) with fast dissolving diluents such as mannitol, lactose, sucrose and/or cyclodextrins. Also included in such formulations may be high molecular weight excipients such as celluloses (avicel) or polyethylene glycols (PEG). Such formulations can also include an excipient to aid mucosal adhesion such as hydroxy propyl cellulose (HPC), hydroxy propyl methyl cellulose (HPMC), sodium carboxy methyl cellulose (SCMC), maleic anhydride copolymer (e.g., Gantrez), and agents to control release such as polyacrylic copolymer (e.g. Carbopol 934). Lubricants, glidants, flavors, coloring agents and stabilizers may also be added for ease of fabrication and use. Lubricants used in these dosage forms include sodium oleate, sodium stearate, magnesium stearate, sodium benzoate, sodium acetate, sodium chloride and the like. For oral administration in liquid form, the oral drug components can be combined with any oral, non-toxic, pharmaceutically acceptable inert carrier such as ethanol, glycerol, water, and the like.

The pharmaceutical formulations according to the invention include those suitable for oral, parenteral (including subcutaneous, intradermal, intramuscular, intravenous [bolus or infusion], and intraarticular), inhalation (including fine particle dusts or mists which may be generated by means of various types of metered dose pressurized aerosols), nebulizers or insufflators, rectal, intraperitoneal and topical (including dermal, buccal, sublingual, and intraocular) administration, although the most suitable route may depend upon, for example, the condition and disorder of the recipient.

Formulations of the present invention suitable for oral administration may be presented as discrete units such as capsules, cachets, pills or tablets each containing a predetermined amount of the active ingredient; as a powder or granules; as a solution or a suspension in an aqueous liquid or a non-aqueous liquid, for example as elixirs, tinctures, suspensions or syrups; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein. The present compounds can, for example, be administered in a form suitable for immediate release or extended release. Immediate release or extended release can be achieved by the use of suitable pharmaceutical compositions comprising the present compounds, or, particularly in the case of extended release, by the use of devices such as subcutaneous implants or osmotic pumps. The present compounds can also be administered liposomally.

Preferred unit dosage formulations are those containing an effective dose, as hereinbefore recited, or an appropriate fraction thereof, of the active ingredient.

It should be understood that in addition to the ingredients particularly mentioned above, the formulations of this invention may include other agents conventional in the art having regard to the type of formulation in question, for example those suitable for oral administration may include flavouring agents.

The formulations may conveniently be presented in unit dosage form and may be prepared by any of the methods well known in the art of pharmacy. All methods include the step of bringing the active ingredient into association with the carrier which constitutes one or more accessory ingredients. In general the formulations are prepared by uniformly and intimately bringing into association the active ingredient with liquid carriers or finely divided solid carriers or both and then, if necessary, shaping the product into the desired formulation.

The compounds of the present invention can also be administered in the form of liposome delivery systems, such as small unilamellar vesicles, large unilamellar vesicles and multilamellar vesicles. Liposomes can be formed from a variety of phospholipids, 1,2-dipalmitoylphosphatidylcholine, phosphatidyl ethanolamine (cephaline), or phosphatidylcholine (lecithin).

Formulations for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example saline or water-for-injection, immediately prior to use. Extemporaneous injection solutions and suspensions may be prepared from sterile powders, granules and tablets of the kind previously described. Exemplary compositions for parenteral administration include injectable solutions or suspensions which can contain, for example, suitable non-toxic, parenterally acceptable diluents or solvents, such as mannitol, 1,3-butanediol, water, Ringer's solution, an isotonic sodium chloride solution, or other suitable dispersing or wetting and suspending agents, including synthetic mono- or diglycerides, and fatty acids, including oleic acid, or Cremaphor.

Exemplary compositions for nasal, aerosol or inhalation administration include solutions in saline, which can contain, for example, benzyl alcohol or other suitable preservatives, absorption promoters to enhance bioavailability, and/or other solubilizing or dispersing agents such as those known in the art.

Formulations for rectal administration may be presented as a suppository with the usual carriers such as cocoa butter, synthetic glyceride esters or polyethylene glycol. Such carriers are typically solid at ordinary temperatures, but liquefy and/or dissolve in the rectal cavity to release the drug.

Formulations for topical administration in the mouth, for example buccally or sublingually, include lozenges comprising the active ingredient in a flavoured basis such as sucrose and acacia or tragacanth, and pastilles comprising the active ingredient in a basis such as gelatin and glycerine or sucrose and acacia. Exemplary compositions for topical administration include a topical carrier such as Plastibase (mineral oil gelled with polyethylene).

The amount of active ingredient which is required to achieve a therapeutic effect will, of course, vary with the particular compound, the route of administration, the subject under treatment, including the type, species, age, weight, sex, and medical condition of the subject and the renal and hepatic function of the subject, and the particular disorder or disease being treated, as well as its severity. An ordinarily skilled physician, veterinarian or clinician can readily determine and prescribe the effective amount of the drug required to prevent, counter or arrest the progress of the condition.

Oral dosages of the present invention, when used for the indicated effects, will range between about 0.01 mg per kg of body weight per day (mg/kg/day) to about 100 mg/kg/day, preferably 0.01 mg per kg of body weight per day (mg/kg/day) to 10 mg/kg/day, and most preferably 0.1 to 5.0 mg/kg/day, for adult humans. For oral administration, the compositions are preferably provided in the form of tablets or other forms of presentation provided in discrete units containing 0.01, 0.05, 0.1, 0.5, 1.0, 2.5, 5.0, 10.0, 15.0, 25.0, 50.0, 100, and 500 milligrams of the active ingredient for the symptomatic adjustment of the dosage to the patient to be treated. A medicament typically contains from about 0.01 mg to about 500 mg of the active ingredient, preferably from about 1 mg to about 100 mg of active ingredient. Intravenously, the most preferred doses will range from about 0.1 to about 10 mg/kg/minute during a constant rate infusion. Advantageously, compounds of the present invention may be administered in a single daily dose, or the total daily dosage may be administered in divided doses of two, three or four times daily. Furthermore, preferred compounds for the present invention can be administered in intranasal form via topical use of suitable intranasal vehicles, or via transdermal routes, using those forms of transdermal skin patches well known to those of ordinary skill in the art. To be administered in the form of a transdermal delivery system, the dosage administration will, of course, be continuous rather than intermittent throughout the dosage regimen.

The invention also provides the use of a compound of formula I, for the manufacture of a medicament for the treatment or prophylaxis of cancer.

The compounds and the pharmaceutical compositions of the invention may be used in the prophylaxis and treatment of diseases such as cancer, diseases caused by parasites, allergic diseases, Crohns disease, rheumatic diseases, tuberculosis, diabetes, Alzheimer's disease, inflammatory diseases, multiple sclerosis (MS), amyotrophic lateral sclerosis (ALS), Parkinson's disease and diseases caused by bacteria, viruses and fungus.

The compounds of the invention find particular application in the treatment or prophylaxis of various cancer types, including but not limited to, cancer of the bone, breast, respiratory tract, brain, reproductive organs, digestive tract, urinary tract, eye, liver, skin, head, neck, thyroid, parathyroid, and metastatic forms thereof. Proliferative disorders of the breast include, but are not limited to, invasive ductal carcinoma, invasive lobular carcinoma, ductal carcinoma, lobular carcinoma in situ, and metastatic breast cancer. Proliferative disorders of the skin include, but are not limited to, basal cell carcinoma, squamous cell carcinoma, malignant melanoma, and Kaposi's sarcoma. Proliferative disorders of the respiratory tract include, but are not limited to, small cell and non-small cell lung carcinoma, bronchial adenoma, pleuropulmonary blastoma, and malignant mesothelioma. Proliferative disorders of the brain include, but are not limited to, brain stem and hypothalamic glioma, cerebellar and cerebral astrocytoma, medulloblastoma, ependymal tumors, oligodendroglial tumors, meningiomas and neuroectodermal, and pineal tumors. Proliferative disorders of the male reproductive organs include, but are not limited to, prostate, testicular and penis cancer. Proliferative disorders of the female reproductive organs include, but are not limited to, uterine, cervical, ovarian, vaginal, vulval cancer, uterine sarcoma, and ovarian germ cell tumor. Proliferative disorders of the digestive tract include, but are not limited to, anal, colon, colorectal, esophageal, gall bladder, stomach, pancreatic, rectal, small intestine, and salivary gland cancer.

Proliferative disorders of the liver include, but are not limited to, hepatocellular carcinoma, cholangiocarcinoma, and primary liver cancer. Proliferative disorders of the eye include, but are not limited to, intraocular melanoma, retinoblastoma, and rhabdomyosarcoma. Proliferative disorders of the head include, but are not limited to, laryngeal, hypopharyngeal, nasopharyngeal, oropharyngeal, lip, oral, and metastatic paranasal sinus cancer. Proliferative disorders of the lymphomas include, but are not limited to, T cell and B cell lymphomas, non-Hodgkins lymphoma, cutaneous T cell lymphoma, Hodgkins disease, and lymphoma of the central nervous system. Leukemia includes, but is not limited to, acute myeloid leukemia, chronic myelogenous leukemia, and hairy cell leukemia. Proliferative disorders of the thyroid include, but are not limited to, thyroid cancer, thymoma, and malignant thymoma. Proliferative disorders of the urinary tract include, but are not limited to, bladder cancer. Sarcomas include, but are not limited to, sarcoma of the soft tissue, osteosarcoma, malignant fibrous histiocytoma, lymphosarcoma, and rhabdomyosarcoma.

Whilst a compound of the invention may be used alone, it is also possible for the compounds to be used in combination with each other, in combination with radiation therapy, or in combination with other anticancer agents. Various classes of anticancer and antineoplastic compounds include, but are not limited to, alkylating agents, antimetabolites, anticancer camptothecin derivatives, plant-derived anticancer agents, antibiotics, enzymes, platinum coordination complexes, tyrosine kinase inhibitors, hormones and hormone antagonists, monoclonal antibodies, interferons, biological response modifiers and other anticancer agents. Examples of alkylating agents include, but are not limited to, mechlorethamine, cyclophosphamide, ifosfamide, melphalan, chlorambucil, busulfan, mitobronitol, ranimustin, nimustin, temozolomide and carmustine; examples of antimetabolites include, but are not limited to, methotrexate, fluorouracil, cytarabine, gemcitabine, fludarabine, mercaptopurine, thioguanine, and azathioprine; examples of camptothecin derivatives include, but are not limited to, irinotecan, topotecan, and camptothecin; examples of plant-derived agents include, but are not limited to, vinblastine, vincristine, taxanes, paclitaxel, and colchicines; examples of antibiotics include, but are not limited to, actinomycin D, daunorubicin, and bleomycin. One example of enzyme effective as antineoplastic agent includes L-asparaginase. Examples of coordination compounds include, but are not limited to, cisplatin and carboplatin; examples of tyrosine kinase inhibitors include, but are not limited to, gefitinib, imatinib, sunitinib, nilotinib, dasatinib, erlotinib, and pazopanib; examples of hormones and hormone related compounds include, but are not limited to, prednisone, dexamethasone, formestane, amino glutethimide, anastrozole, hydroxyprogesterone caproate, medroxyprogesterone and tamoxifen; examples of interferons include, but are not limited to, interferon α, interferon α-2a, interferon α-2b, interferon β, interferon γ-1a, and interferon γ-n1; examples of biological response modifiers include, but are not limited to, krestin, lentinan, sizofiran, picibanil, and ubenimex. Examples of other anticancer agents include, but are not limited to, mitoxantrone, procarbazine, dacarbazine, hydroxycarbamide, pentostatin, tretinoin, leuprorelin, flutamide, and aldesleukin.

Numerous synthetic routes to the compounds of the present invention can be devised by any person skilled in the art and the possible synthetic routes described below do not limit the invention.

### Procedure for synthesizing the compounds of general formula I

The appropriate amine (II) was dissolved in ethylene glycol (0.2 g/mL). 1.1 Eq of pyrimidine (III) and 1.2 eq of *N*,*N*-diisopropylethylamine (DIPEA) were added and the mixture was stirred at 50-120 °C for 1-3 h. The reaction mixture was dissolved in EtOAc/MeOH 5:1 and washed with saturated aqueous NaHCO₃, water and brine. The solvent was removed *in vacuo* and the residue was purified by column chromatography on silica gel with heptane/EtOAc, EtOAc or EtOAc/MeOH as eluent to give the major intermediate (IV') and the minor intermediate (IV").

The intermediate (IV' or IV") was dissolved in ethylene glycol (0.2 g/mL) and 1.1 eq of amine (V) was added. The mixture was then stirred at 100-150 °C for 1-3 h. The reaction mixture was dissolved in EtOAc/MeOH 5:1 and washed with saturated aqueous NaHCO₃, water and brine. The solvent was removed in vacuo and the residue was purified by column chromatography on silica gel with heptane/EtOAc, EtOAc or EtOAc/MeOH as eluent to give the compound of formula I. This procedure is exemplified in Scheme 1.

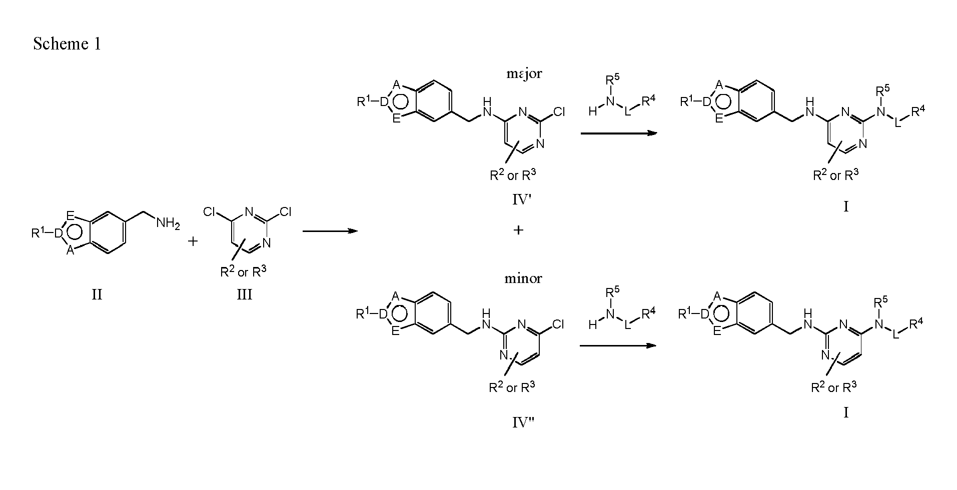

Compounds of Examples 1-38 were synthesized by this general reaction procedure (as set out in Scheme 1.). A, D, E, R¹ to R⁵, and L are as defined in formula I.

### General procedure for synthesizing the compounds of general formula I, represented by formula VII, starting from compounds of formula VI

The intermediate VI which was synthesized by the general procedure in Scheme 1 was dissolved in MeOH (1.5 mg/mL) and 10 % Pd/C (20 mol %) was added. The flask was pump-purged with argon and then with hydrogen. The reaction mixture was left stirring vigorously under hydrogen atmosphere at room temperature for 20 h. The catalyst was filtered off and the solvent was evaporated yielding pure product VII, as exemplified in Scheme 2.

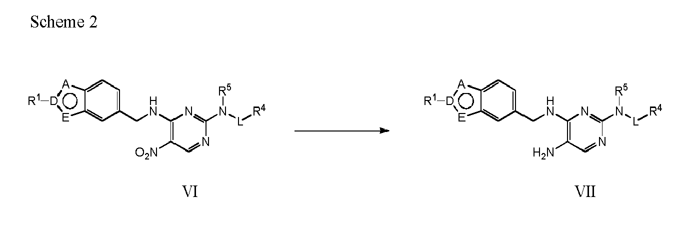

Compounds of Examples 39 - 42 were synthesized by this general reaction procedure (as set out in Scheme 2.). A, D, E, R¹, R⁴, R⁵, and L are as defined in formula I.

### Example 1

### N⁴-(1H-indol-5-ylmethyl)-N²-(1H-indol-4-yl)pyrimidine-2,4-diamine

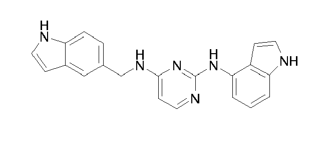

¹H NMR (500 MHz, DMSO-*d*₆) *δ* 11.00 (s, 1H), 10.95 (s, 1H), 8.33 (s, 1H), 7.85 (d, 1H), 7.81 (d, 1H), 7.58 (br s, 1H), 7.49 (s, 1H), 7.34 (d, 1H), 7.30 (t, 1H), 7.19 (t, 1H), 7.09 (d, 1H), 6.99 (m, 2H), 6.80 (m, 1H), 6.36 (m, 1H), 5.98 (d, 1H), 4.61 (br s, 2H).
MS (ESI⁺) *m*/*z* 355.3 (M⁺+H).

### Example 2

### N⁴-(1H-indol-5-ylmethyl)-N²-(1H-indol-5-yl)pyrimidine-2,4-diamine

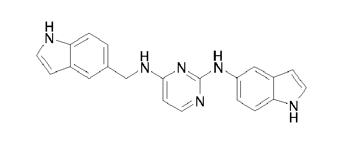

¹H NMR (500 MHz, DMSO-*d*₆) *δ* 11.00 (s, 1H), 10.80 (s, 1H), 8.60 (s, 1H), 8.00 (m, 1H), 7.76 (d, 1H), 7.50 (s, 1H), 7.49 (br s, 1H), 7.34-7.28 (m, 3H), 7.22-7.19 (m, 2H), 7.10 (m, 1H), 6.35 (m, 1H), 6.27 (m, 1H), 5.91 (d, 1H), 4.61 (br s, 2H).
MS (ESI⁺) *m*/*z* 355.3 (M⁺+H).

### Example 3

### N⁴-(1H-indol-5-ylmethyl)-N²-(2-methyl-1H-indol-5-yl)pyrimidine-2,4-diamine

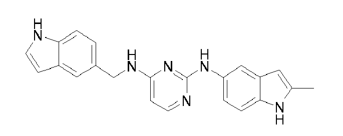

¹H-NMR (500 MHz, DMSO-*d*₆) *δ* 10.99 (s, 1H), 10.60 (s, 1H), 8.53 (s, 1H), 7.84 (d, 1H), 7.75 (d, 1H), 7.50 (s, 1H), 7.46 (br. s, 1H), 7.33 (d, 1H), 7.29 (t, 1H), 7.21 (dd, 1H), 7.10 (dd, 1H), 7.07 (d, 1H), 6.35 (br. s, 1H), 5.96 (s, 1H), 5.89 (d, 1H), 4.59 (br. s, 2H), 2.33 (s, 3H).
MS (ESI⁺) *m*/*z* 369.3 (M⁺+H).

### Example 4

### N⁴-(1H-indol-5-ylmethyl)-N²-(2-methyl-1H-indol-5-yl)pyrimidine-2,4-diamine

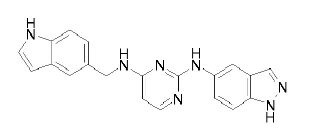

¹H-NMR (500 MHz, CD₃OD) *δ* 8.01 (s, 1H), 7.80 (br. s, 1H), 7.72 (d, 1H), 7.51 (s, 1H), 7.43 (dd, 1H), 7.39 (d, 1H), 7.33 (d, 1H), 7.19 (d, 1H), 7.10 (dd, 1H), 6.37 (d, 1H), 5.97 (d, 1H), 4.64 (br. s, 2H).
MS (ESI⁺) *m*/*z* 356.3 (M⁺+H).

### Example 5

### N⁴,N⁴-bis(1H-indol-5-ylmethyl)pyrimidine-2,4-diamine

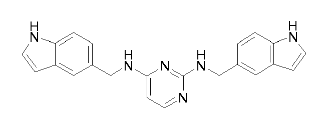

¹H NMR (300 MHz, CDCl₃) δ 8.15 (m, 2H), 7.86 (d, 1H), 7.61 (d, 2H), 7.33 (m, 2H), 7.23-7.15 (m, 4H), 6.51 (m, 2H), 5.75 (d, 1H), 5.18 (m, 1H), 4.96 (m, 1H), 4.69 (d, 2H), 4.59 (d, 2H).
MS (ESI⁺) *m*/*z* 369.3 (M⁺+H).

### Example 6

### N⁴-(1H-indol-5-ylmethyl)-N²-(2-methyl-1H-indol-5-yl)pyrimidine-2,4-diamine

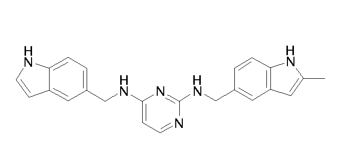

¹H NMR (500 MHz, DMSO-*d*₆) *δ* 10.99 (s, 1H), 10.74 (s, 1H), 7.61 (d, 1H), 7.45 (s, 1H), 7.30-7.28 (m, 3H), 7.25 (br s, 1H), 7.13 (d, 1H), 7.05 (dd, 1H), 6.96 (dd, 1H), 6.75 (br s, 1H), 6.34 (br s, 1H), 5.99 (br s, 1H), 5.72 (d, 1H), 4.51 (br s, 2H), 4.46 (d, 2H), 2.34 (s, 3H).
MS (ESI⁺) *m*/*z* 383.3 (M⁺+H).

### Example 7

### N²-(1H-indazol-5-ylmethyl)-N⁴-(1H-indol-5-ylmethyl)pyrimidine-2,4-diamine

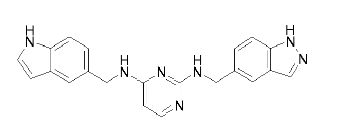

¹H NMR (500 MHz, DMSO-*d*₆) *δ* 12.92 (s, 1H), 10.99 (s, 1H), 7.92 (br s, 1H), 7.61 (d, 1H), 7.59 (s, 1H), 7.43-7.39 (m, 2H), 7.33-7.28 (m, 4H), 7.02 (d, 1H), 6.95 (br s, 1H), 6.32 (s, 1H), 5.74 (d, 1H), 4.51 (d, 4H).
MS (ESI⁺) *m*/*z* 370.3 (M⁺+H).

### Example 8

### N²-(1H-benzo[d]imidazol-5-ylmethyl)-N⁴-(1H-indol-5-ylmethyl)pyrimidine-2,4-diamine diamine

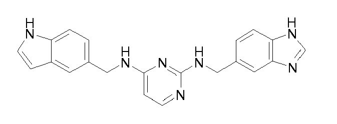

¹H NMR (500 MHz, DMSO-*d*₆, 75°C) *δ* 12.10 (br s, 1H), 10.83 (br s, 1H), 8.10 (s, 1H), 7.65 (s, 1H), 7.54 (s, 1H), 7.48-7.46 (m, 3H), 7.30 (d, 1H), 7.27 (t, 1H), 7.18 (d, 1H), 7.04 (d, 1H), 7.00 (br s, 1H), 6.34 (br s, 1H), 5.85 (d, 1H), 4.60 (d, 2H), 4.54 (d, 2H).
MS (ESI⁺) *m*/*z* 370.2 (M⁺+H).

### Example 9

### N⁴-(1H-indol-5-ylmethyl)-N²-(1H-indol-6-ylmethyl)pyrimidine-2,4-diamine

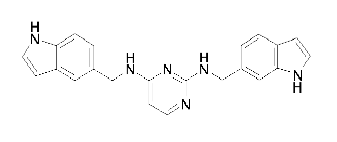

¹H NMR (500 MHz, DMSO-*d*₆) *δ* 10.98 (s, 1H), 10.93 (s, 1H), 7.62 (d, 1H), 7.44 (s, 1H), 7.40 (d, 1H), 7.32 (s, 1H), 7.29-7.24 (m, 4H), 7.03 (d, 1H), 6.96 (d, 1H), 6.88 (br s, 1H), 6.35 (m, 1H), 6.32 (br s, 1H), 5.73 (d, 1H), 4.52 (d, 2H), 4.50 (br s, 2H).
MS (ESI⁺) *m*/*z* 369.3 (M⁺+H).

### Example 10

### N²-[2-(1H-indol-3-yl)ethyl]-N⁴-(1H-indol-5-ylmethyl)pyrimidine-2,4-diamine

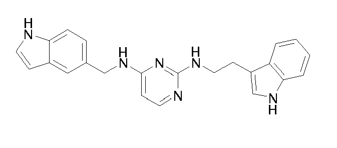

¹H-NMR (300 MHz, CDCl₃) *δ* 8.20 (br s, 1H), 7.95 (br s, 1H), 7.83 (d, 1H), 7.65 (d, 1H), 7.60 (s, 1H), 7.38 6.91 (m, 7H), 6.53 (br s, 1H), 5.73 (d, 1H), 4.94 (m, 2H), 4.58 (m, 2H), 3.73 (q, 2H), 3.06 (t, 2H).
MS (ESI⁺) *m*/*z* 383.3 (M⁺+H).

### Example 11

### 3-{2-[4-(1H-indol-5-ylmethylamino)-pyrimidin-2-ylamino]ethyl}-1H-indol-5-ol

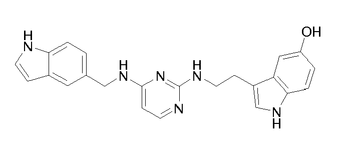

¹H-NMR (500 MHz, DMSO-*d*₆) *δ* 10.99 (s, 1H), 10.44 (s, 1H), 8.56 (s, 1H), 7.65 (br. s, 1H), 7.47 (s, 1H), 7.31 (d, 1H), 7.29 (t, 1H), 7.25 (br. s, 1H), 7.11 (d, 1H), 7.06 (d, 1H), 7.02 (s, 1H), 6.87 (d, 2H), 6.58 (dd, 1H), 6.34 (br. s, 2H), 5.75 (br. s, 1H), 4.52 (br. s, 2H), 3.46 (q, 2H), 2.81 (t, 2H).
MS (ESI⁺) *m*/*z* 399.3 (M⁺+H).

### Example 12

### N⁴-(1H-indol-5-ylmethyl)-N²-[2-(5-methyl-1H-indol-3-yl)ethyl]pyrimidine-2,4-diamine diamine

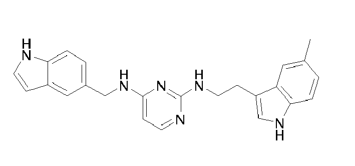

¹H-NMR (500 MHz, DMSO-*d*₆) *δ* 10.99 (s, 1H), 10.62 (s, 1H), 7.64 (br. s, 1H), 7.47 (s, 1H), 7.33 (s, 1H), 7.31 (d, 1H), 7.29 (t, 1H), 7.26 (br. s, 1H), 7.19 (d, 1H), 7.08 (s, 1H), 7.06 (d, 1H), 6.86 (d, 2H), 6.37 (br. s, 1H), 6.34 (br. s, 1H), 5.74 (dd, 1H), 4.54 (br. s, 2H), 3.48 (q, 2H), 2.88 (t, 2H), 2.32 (s, 3H).
MS (ESI⁺) *m*/*z* 397.3 (M⁺+H).

### Example 13

### N⁴-(1H-indol-5-ylmethyl)-N²-[2-(5-methoxy-1H-indol-3-yl)ethyl]pyrimidine-2,4-diamine diamine

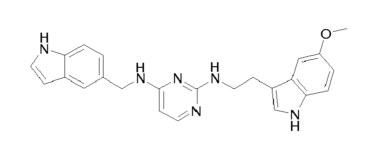

¹H-NMR (500 MHz, DMSO-*d*₆) *δ* 10.99 (s, 1H), 10.60 (s, 1H), 7.65 (br. s, 1H), 7.47 (s, 1H), 7.31 (d, 1H), 7.29 (t, 1H), 7.26 (br. s, 1H), 7.20 (d, 1H), 7.09 (d, 1H), 7.06 (d, 1H), 6.69 (dd, 1H), 6.37 (br. s, 1H), 6.34 (br. s, 1H), 5.75 (s, 1H), 4.53 (br. s, 2H), 3.71 (s, 3H), 3.48 (q, 2H), 2.88 (t, 2H).
MS (ESI⁺) *m*/*z* 413.3 (M⁺+H).

### Example 14

### N²-(1H-indol-4-yl)-N⁴-(2-methyl-1H-indol-5-ylmethyl)pyrimidine-2,4-diamine

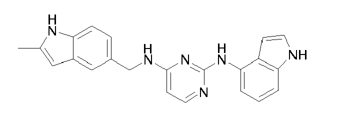

¹H-NMR (500 MHz, DMSO-*d*₆) *δ* 10.95 (s, 1H), 10.81 (s, 1H), 8.32 (s, 1H), 7.86 (d, 1H), 7.80 (d, 1H), 7.54 (br s, 1H), 7.35 (s, 1H), 7.19 (d, 1H), 7.18 (s, 1H), 6.97 (m, 3H), 6.80 (s, 1H), 6.05 (s, 1H), 5.98 (d, 1H), 4.58 (br s, 2H), 2.35 (s, 3H).
MS (ESI⁺) *m*/*z* 369.3 (M⁺+H).

### Example 15

### N²-(1H-indol-5-ylmethyl)-N⁴-(2-methyl-1H-indol-5-ylmethyl)pyrimidine-2,4-diamine

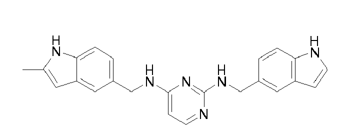

¹H-NMR (500 MHz, DMSO-*d*₆) *δ* 10.95 (s, 1H), 10.79 (s, 1H), 7.61 (d, 1H), 7.44 (s, 1H), 7.30 (s, 1H), 7.26 (dd, 2H), 7.23 (br. s, 1H), 7.15 (d, 1H), 7.06 (d, 2H), 6.94 (d, 1H), 6.79 (br.s, 1H), 6.32 (d, 1H), 6.01 (d, 1H), 5.72 (d, 1H), 4.49 (d, 4H), 2.35 (s, 3H).
MS (ESI⁺) *m*/*z* 383.3 (M⁺+H).

### Example 16

### N²,N⁴-Bis-(2H-methyl-1H-indol-5-ylmethyl)pyrimidine-2,4-diamine

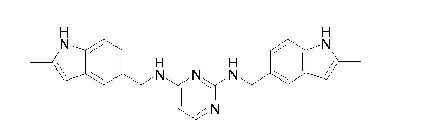

¹H-NMR (500 MHz, DMSO-*d*₆, 75°C) *δ* 7.63 (d, 1H), 7.32 (s, 2H), 7.17 (d, 1H), 7.14 (d, 1H), 6.97 (t, 2H), 6.01 (d, 2H), 5.75 (d, 1H), 4.49 (s, 4H), 2.36 (s, 6H).
MS (ESI⁺) *m*/*z* 397.4 (M⁺+H).

### Example 17

### N²-(1H-indazol-5-ylmethyl)-N⁴-(2-methyl-1H-indol-5-ylmethyl)pyrimidine-2,4-diamine diamine

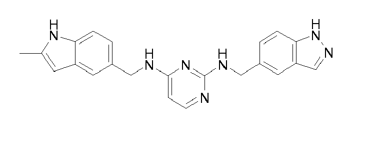

¹H-NMR (500 MHz, CD₃OD) *δ* 7.85 (br s, 1H), 7.63 (s, 1H), 7.58 (br s, 1H), 7.41 (d, 1H), 7.36 (d, 1H), 7.29 (s, 1H), 7.11 (d, 1H), 6.91 (d, 1H), 5.97 (s, 1H), 5.83 (d, 1H), 4.64 (s, 2H), 4.59 (br s, 2H), 2.38 (s, 3H).
MS (ESI⁺) *m*/*z* 384.3 (M⁺+H).

### Example 18

### N²-(2-(1H-indol-3-yl)-ethyl)-N⁴-(2-methyl-1H-indol-5-ylmethyl)pyrimidine-2,4-diamine diamine

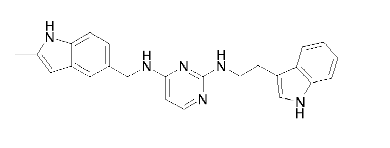

¹H-NMR (500 MHz, DMSO-*d*₆) *δ* 10.80 (s, 1H), 10.76 (s, 1H), 7.64 (br s, 1H), 7.55 (d, 1H), 7.32 (s, 1H), 7.31 (s, 1H), 7.25 (br s, 1H), 7.18 (d, 1H), 7.14 (s, 1H), 7.04 (t, 1H), 6.97 (d, 1H), 6.92 (br s, 1H), 6.39 (br s, 1H), 6.02 (s, 1H), 5.74 (s, 1H), 4.51 (br s, 2H), 3.45 (q, 2H), 2.90 (t, 2H), 2.34 (s, 3H).
MS (ESI⁺) *m*/*z* 397.3 (M⁺+H).

### Example 19

### N²-(1H-indol-4-yl)-N⁴-(1H-indazol-5-ylmethyl)pyrimidine-2,4-diamine

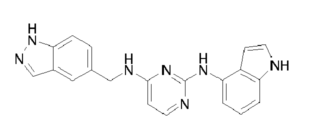

¹H-NMR (500 MHz, DMSO-*d*₆) *δ* 12.97 (s, 1H), 10.95 (s, 1H), 8.36 (s, 1H), 7.99 (s, 1H), 7.82 (d, 1H), 7.75 (br s, 1H), 7.68 (br s, 1H), 7.65 (s, 1H), 7.48 (d, 1H), 7.35 (d, 1H), 7.19 (s, 1H), 6.98 (d, 1H), 6.92 (t, 1H), 6.77 (s, 1H), 5.99 (br s, 1H), 4.62 (br s, 2H).
MS (ESI⁺) *m*/*z* 356.3 (M⁺+H).

### Example 20

### N⁴-(1H-benzo[d]imidazol-5-ylmethyl)-N²-(1H-indol-4-yl)pyrimidine-2,4-diamine

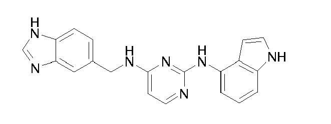

¹H NMR (500 MHz, DMSO-*d*₆, 75°C) *δ*12.16 (br s, 1H), 10.79 (s, 1H), 8.10 (s, 1H), 8.03 (s, 1H), 7.83 (d, 1H), 7.78 (d, 1H), 7.54 (br s, 2H), 7.44 (m, 1H), 7.20 (m, 1H), 7.18 (t, 1H), 7.00 (d, 1H), 6.94 (t, 1H), 6.72 (br s, 1H), 6.02 (d, 1H), 4.66 (d, 2H).
MS (ESI⁺) *m*/*z* 356.2 (M⁺+H).

### Example 21

### N⁴-(1H-indol-6-ylmethyl)-N²-(1H-indol-4-yl)pyrimidine-2,4-diamine

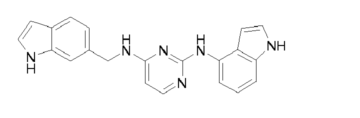

¹H NMR (500 MHz, DMSO-*d*₆) *δ* 11.00 (s, 1H), 10.94 (s, 1H), 8.34 (s, 1H), 7.83 (m, 2H), 7.63 (br s, 1H), 7.48 (d, 1H), 7.36 (s, 1H), 7.28 (t, 1H), 7.18 (t, 1H), 7.01 (dd, 1H), 6.98-6.91 (2H), 6.80 (m, 1H), 6.37 (m, 1H), 6.00 (br s, 1H), 4.65 (br s, 2H).
MS (ESI⁺) *m*/*z* 355.3 (M⁺+H).

### Example 22

### N²-(1H-indol-5-ylmethyl)-N⁴-(2-methyl-1H-indol-5-yl)pyrimidine-2,4-diamine

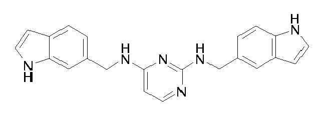

¹H NMR (500 MHz, DMSO-*d*₆) *δ* 10.97 (s, 1H), 10.94 (s, 1H), 7.63 (d, 1H), 7.44 (m, 2H), 7.31-7.23 (m, 5H), 7.05 (dd, 1H), 6.95 (dd, 1H), 6.79 (br s, 1H), 6.37 (m, 1H), 6.30 (br s, 1H), 5.74 (m, 1H), 4.55 (br s, 2H), 4.48 (d, 2H).
MS (ESI⁺) *m*/*z* 369.3 (M⁺+H).

### Example 23

### N²,N⁴-bis-(1H-indol-6-ylmethyl)pyrimidine-2,4-diamine

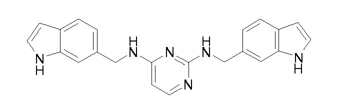

¹H NMR (500 MHz, DMSO-*d*₆) *δ* 10.96 (s, 1H), 10.92 (s, 1H), 7.63 (d, 1H), 7.43 (d, 1H), 7.39 (d, 1H), 7.31 (m, 3H), 7.27 (t, 1H), 7.25 (t, 1H), 6.95 (m, 2H), 6.87 (br s, 1H), 6.36 (m, 1H), 6.34 (m, 1H), 5.75 (m, 1H), 4.53 (br s, 2H), 4.51 (d, 2H).
MS (ESI⁺) *m*/*z* 369.3 (M⁺+H).

### Example 24

### N²-(1H-indol-5-ylmethyl)-N⁴-(1H-indol-4-yl)pyrimidine-2,4-diamine

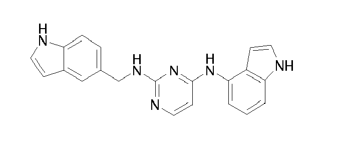

¹H-NMR (500 MHz, DMSO-*d*₆): *δ* 11.05 (br s, 1H), 10.94 (br s, 1H), 8.70 (s, 1H), 7.82 (d, 1H), 7.72 (br s, 1H), 7.46 (s, 1H), 7.29-7.25 (m, 3H), 7.12-7.02 (m, 3H), 6.95 (t, 1H), 6.67 (s, 1H), 6.33 (s, 1H), 6.15 (d, 1H), 4.54 (d, 2H).
MS (ESI⁺) *m*/*z* 355.3 (M⁺+H).

### Example 25

### N²-(1H-indol-5-ylmethyl)-N⁴-(2-methyl-1H-indol-5-yl)pyrimidine-2,4-diamine

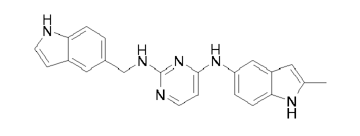

¹H-NMR (500 MHz, DMSO-*d*₆) *δ* 10.95 (s, 1H), 10.73 (s, 1H), 8.74 (s, 1H), 7.74 (d, 1H), 7.70 (s, 1H), 7.47 (s, 1H), 7.29 (d, 1H), 7.27-7.25 (m, 1H), 7.14 (d, 1H), 7.10 (d, 1H), 7.06 (d, 1H), 7.00 (br. s, 1H), 6.33 (s, 1H), 6.01 (s, 1H), 5.88 (d, 1H), 4.53 (d, 2H), 2.35 (s, 3H).
MS (ESI⁺) *m*/*z* 369.3 (M⁺+H).

### Example 26

### N⁴-[2-(1H-indol-3-yl)ethyl]-N²-(1H-indol-5-ylmethyl)pyrimidine-2,4-diamine

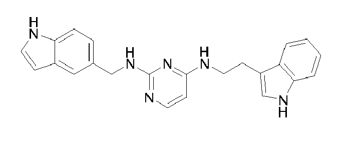

¹H NMR (500 MHz, DMSO-*d*₆) *δ* 10.95 (s, 1H), 10.80 (s, 1H), 7.62 (br s, 1H), 7.51 (d, 1H), 7.45 (s, 1H), 7.33-7.26 (m, 3H), 7.14 (s, 1H), 7.08-6.92 (m, 4H), 6.81 (br s, 1H), 6.31 (s, 1H), 5.69 (m, 1H), 4.51 (d, 2H), 3.52 (m, 2H), 2.90 (t, 2H).
MS (ESI⁺) *m*/*z* 383.3 (M⁺+H).

### Example 27

### 3-{2-[2-(1H-indol-5-ylmethylamino)-pyrimidin-4-ylamino]ethyl}-1H-indol-5-ol

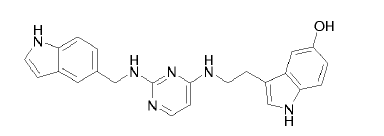

¹H-NMR (500 MHz, DMSO-*d*₆, 75°C) *δ* 10.75 (s, 1H), 10.28 (s, 1H), 8.32 (br. s, 1H), 7.62 (d, 1H), 7.48 (s, 1H), 7.28 (d, 1H), 7.24-7.23 (m, 1H), 7.12 (d, 1H), 7.09 (d, 1H), 7.02 (s, 1H), 6.87 (d, 1H), 6.62 (br. s, 1H), 6.60 (dd, 1H), 6.41 (br. s, 1H), 6.33 (s, 1H), 5.73 (d, 1H), 4.54 (d, 2H), 3.50 (q, 2H), 2.85 (t, 2H).
MS (ESI⁺) *m*/*z* 399.2 (M⁺+H).

### Example 28

### N²-(1H-indol-5-ylmethyl)-N⁴-[2-(5-methyl-1H-indol-3-yl)ethyl]pyrimidine-2,4-diamine diamine

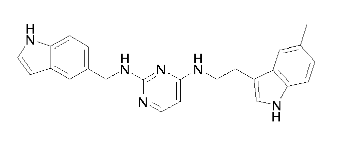

¹H-NMR (500 MHz, DMSO-*d*₆) *δ* 10.93 (s, 1H), 10.65 (s, 1H), 7.62 (br. s, 1H), 7.46 (s, 1H), 7.30 (s, 1H), 7.27-7.25 (m, 2H), 7.20 (d, 1H), 7.10-7.06 (m, 2H), 6.93 (br. s, 1H), 6.87 (d, 1H), 6.77 (br. s, 1H), 6.30 (s, 1H), 5.70 (d, 1H), 4.51 (d, 2H), 3.51 (br. s, 2H), 2.88 (t, 2H), 2.33 (s, 3H).
MS (ESI⁺) *m*/*z* 397.3 (M⁺+H).

### Example 29

### N²-(1H-indol-5-ylmethyl)-N⁴-[2-(5-methyl-1H-indol-3-yl)ethyl]pyrimidine-2,4-diamine diamine

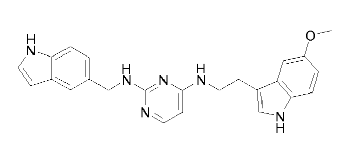

¹H-NMR (500 MHz, DMSO-*d*₆) *δ* 10.93 (s, 1H), 10.63 (s, 1H), 7.62 (br. s, 1H), 7.45 (s, 1H), 7.28-7.25 (m, 2H), 7.21 (d, 1H), 7.09 (s, 1H), 7.07 (d, 1H), 6.98 (d, 1H), 6.93 (br. s, 1H), 6.74 (br. s, 1H), 6.69 (dd, 1H), 6.30 (s, 1H), 5.70 (d, 1H), 4.50 (d, 2H), 3.69 (s, 3H), 3.51 (br. s, 2H), 2.88 (t, 2H).
MS (ESI⁺) *m*/*z* 413.3 (M⁺+H).

### Example 30

### N²-(1H-indazol-5-ylmethyl)-N⁴-(1H-indol-4-yl)pyrimidine-2,4-diamine

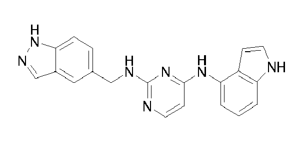

¹H-NMR (300 MHz, DMSO-*d*₆) *δ* 12.94 (br s, 1H), 11.07 (br s, 1H), 8.74 (s, 1 H), 7.97 (s, 1H), 7.82 (d, 1H), 7.68 (m, 1H), 7.61 (s, 1H), 7.45 (d, 1H), 7.35 (d, 1H), 7.25 (m, 2H), 7.06 (d, 1H), 6.96 (m, 1H), 6.16 (d, 1H),4.55 (d, 2H).
MS (ESI⁺) *m*/*z* 356.3 (M⁺+H).

### Example 31

### N²-(1H-indol-4-yl)-N⁴-(1H-indol-5-ylmethyl)-6-methylpyrimidine-2,4-diamine

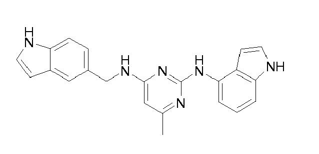

¹H-NMR (500 MHz, DMSO-*d*₆) *δ* 10.99 (s, 1H), 10.93 (s, 1H), 8.26 (s, 1H), 7.90 (br s, 1H), 7.48 (s, 1H), 7.45 (br s, 1H), 7.33 (d, 1H), 7.29 (s, 1H), 7.18 (s, 1H), 7.08 (d, 1H), 6.94 (m, 2H), 6.82 (s, 1H), 6.36 (s, 1H), 5.85 (s, 1H), 4.59 (br s, 2H), 2.12 (s, 3H).
MS (ESI⁺) *m*/*z* 369.3 (M⁺+H).

### Example 32

### N²,N⁴-bis-(1H-indol-5-ylmethyl)-6-methylpyrimidine-2,4-diamine

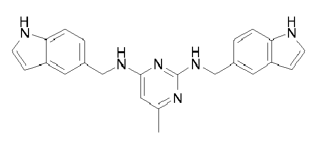

¹H-NMR (300 MHz, CD₃OD) *δ* 7.49 (d, 2H), 7.28 (d, 2H), 7.19 (d, 2H), 7.07 (dt, 2H), 6.35 (d, 2H), 5.71 (s, 1H), 4.62-4.60 (m, 4H), 2.11 (s, 3H).
MS (ESI⁺) *m*/*z* 383.3 (M⁺+H).

### Example 33

### 3-{2-[4-(1H-indol-5-ylmethylamino)-6-methyl-pyrimidin-2-ylamino]-ethyl}-1H-indol-5-ol

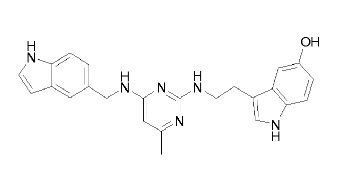

¹H-NMR (300 MHz, CDCl₃+CD₃OD) *δ* 7.51 (s, 1H), 7.32 (d, 1H), 7.18 (d, 1H), 7.14 (d, 1H), 7.08 (d, 1H), 6.97-6.89 (m, 2H), 6.69 (dd, 1H), 6.43 (d, 1H), 5.56 (s, 1H), 4.50 (br. s, 2H), 3.65-3.56 (m, 2H), 2.93-2.87 (m, 2H), 2.09 (s, 3H).
MS (ESI⁺) *m*/*z* 413.3 (M⁺+H).

### Example 34

### N⁴-(1H-indol-5-ylmethyl)-N²-(2-methyl-1H-indol-5-yl)trifluormethylpyrimidine-2,4-diamine

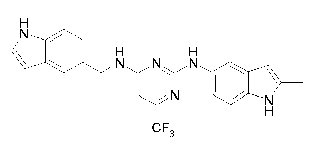

¹H NMR (300 MHz, DMSO-*d*₆) *δ* 11.01 (s, 1H), 10.67 (s, 1H), 9.14 (s, 1H), 8.11 (m, 1H), 7.76 (s, 1H), 7.48 (s, 1H), 7.33 (d, 1H), 7.29 (t, 1H), 7.20 (m, 1H), 7.09 (m, 2H), 6.34 (br. s, 1H), 6.26 (s, 1H), 5.95 (s, 1H), 4.64 (d, 2H), 2.32 (s, 3H).
MS (ESI⁺) *m*/*z* 437.3 (M⁺+H).

### Example 35

### N²,N⁴-bis-(1H-indol-5-ylmethyl)-6-trifluoromethylpyrimidine-2,4-diamine

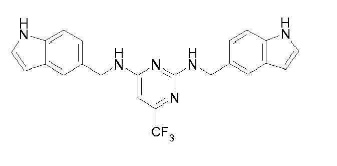

¹H NMR (300 MHz, CD₃OD) *δ* 7.53 (m, 1H), 7.50 (m, 1H), 7.30 (m, 2H), 7.20 (m, 2H), 7.09 (m, 2H), 6.37 (d, 2H), 6.12 (s, 1H), 4.66 (s, 4H).
MS (ESI⁺) *m*/*z* 437.3 (M⁺+H).

### Example 36

### N²-(2-(1H-indol-5-yl)ethyl)-N⁴-(1H-indol-5-ylmethyl)-6-trifluoromethylpyrimidine-2,4-diamine

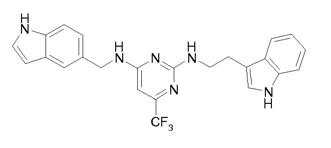

¹H NMR (300 MHz, CDCl₃) *δ* 8.20 (br. s, 1H), 7.95 (br. s, 1H), 7.65 (d, 1H), 7.59 (s, 1H), 7.35 (m, 2H), 7.26-7.09 (m, 4H), 7.00 (s, 1H), 6.53 (s, 1H), 6.02 (s, 1H), 5.15 (br. s, 2H), 4.63 (br. s, 2H), 3.75 (q, 2H), 3.04 (t, 2H).
MS (ESI⁺) *m*/*z* 451.3 (M⁺+H).

### Example 37

### N²,N⁴-bis(1H-indol-5-ylmethyl)-6-benzylpyrimidine-2,4-diamine

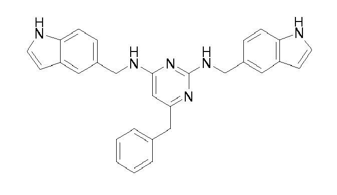

¹H NMR (500 MHz, DMSO-*d*6) *δ* 10.97 (s, 1H), 10.94 (s, 1H), 7.45 (s, 1H), 7.41 (s, 1H), 7.28-7.25 (m, 4H), 7.22 (m, 4H), 7.18 (m, 2H), 7.07 (dd, 1H), 7.01 (dd, 1H), 6.82 (br s, 1H), 6.30 (m, 2H), 5.55 (s, 1H), 4.49 (d, 4H), 3.58 (s, 2H).
MS (ESI⁺) *m*/*z* 459.3 (M⁺+H).

### Example 38

### N⁴-(1H-indol-5-ylmethyl)-N²-(1H-indol-4-yl)-6-methylpyrimidine-2,4-diamine

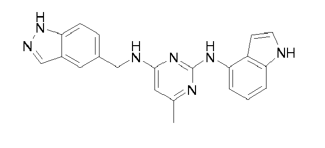

¹H-NMR (300 MHz, DMSO-*d*₆): *δ* 12.98 (s, 1H), 10.95 (s, 1H), 8.34 (s, 1H), 7.99 (s, 1H), 7.79 (m, 1H), 7.63 (s, 1H), 7.57 (br s, 1H), 7.49 (d, 1H), 7.34 (d, 1H), 7.17 (t, 1H), 6.91 (m, 2H), 6.81 (br s, 1H),5.86 (s, 1H), 4.61 (br s, 2H), 2.13 (s, 3H).
MS (ESI⁺) *m*/*z* 370.2 (M⁺+H).

### Example 39

### N⁴-(1H-indol-5-ylmethyl)-N²-(1H-indol-4-yl)pyrimidine-2,4,5-triamine

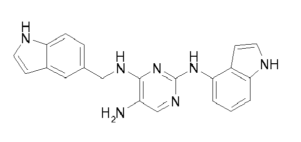

¹H-NMR (500 MHz, DMSO-*d*₆) *δ* 11.00 (s, 1H), 10.89 (s, 1H), 7.87 (br. s, 1H), 7.82 (s, 1H), 7.54 (s, 1H), 7.44 (s, 1H), 7.34 (d, 1H), 7.29 (s, 1H), 7.15 (s, 2H), 6.88 (s, 2H), 6.80 (s, 2H), 6.36 (s, 1H), 4.74 (s, 2H), 4.14 (s, 2H).
MS (ESI⁺) *m*/*z* 370.2 (M⁺+H).

### Example 40

### N⁴-(1H-indol-5-ylmethyl)-N²-(1H-indol-5-yl)pyrimidine-2,4,5-diamine

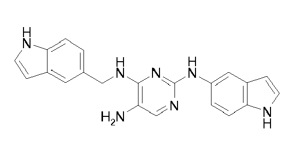

¹H-NMR(500 MHz, DMSO-*d*₆) *δ* 11.00 (s, 1H), 10.72 (s, 1H), 8.10 (s, 1H), 7.98 (s, 1H), 7.53 (s, 1H), 7.40 (s, 1H), 7.34 (d, 1H), 7.29 (t, 1H), 7.25 (dd, 2H), 7.18 (t, 2H), 7.16-7.13 (m, 2H), 6.72 (t, 1H), 6.35 (br. s, 1H), 6.22 (br. s, 1H), 4.72 (d, 2H), 3.99 (s, 2H).
MS (ESI⁺) *m*/*z* 370.2 (M⁺+H).

### Example 41

### N⁴-(1H-indol-5-ylmethyl)-N²-(1H-indol-6-yl)pyrimidine-2,4,5-triamine

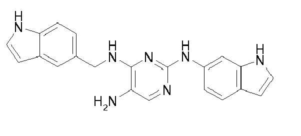

¹H-NMR (300 MHz, CD₃OD) *δ* 7.81-7.79 (m, 1H), 7.60-7.59 (m, 1H), 7.50 (s, 1H), 7.45 (d, 1H), 7.42 (d, 1H), 7.23-7.18 (m, 2H), 7.07 (d, 1H), 7.00 (dd, 1H), 6.40 (dd, 1H), 6.33 (dd, 1H), 4.81 (s, 2H).
MS (ESI⁺) *m*/*z* 370.3 (M⁺+H).

### Example 42

### N²,N⁴-bis(1H-indol-5-ylmethyl)pyrimidine-2,4,5-triamine

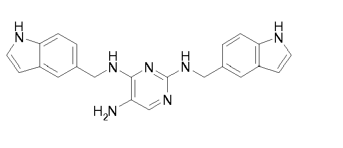

¹H-NMR (500 MHz, CD₃OD) *δ* 7.52 (s, 1H), 7.48 (s, 1H), 7.33 (s, 1H), 7.26 (dd, 2H), 7.17 (dd, 2H), 7.09 (dd, 1H), 7.07 (dd, 1H), 6.34 (dd, 2H), 4.71 (s, 2H), 4.55 (s, 2H).
MS (ESI⁺) *m*/*z* 384.3 (M⁺).

### Intermediate 43

### N-(1H-indol-5-ylmethyl)-2-chloro-pyrimidin-4-amine

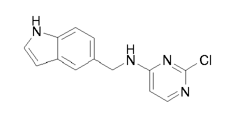

¹H-NMR (500 MHz, CD₃OD) *δ* 7.81 (s, 1H), 7.51 (s, 1H), 7.34 (d, 1H), 7.21 (d, 1H), 7.09 (d, 1H), 6.40 (d, 2H), 4.61 (s, 2H).
MS (ESI⁺) *m*/*z* 259.1 (M⁺+H).

### Intermediate 44

### N-(1H-indol-5-yl)methyl)-4-chloro-pyrimidin-2-amine

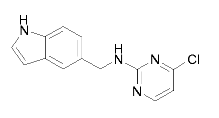

¹H-NMR (500 MHz, CD₃OD) *δ* 8.13 (d, 1H), 7.51 (s, 1H), 7.32 (d, 1H), 7.19 (d, 1H), 7.10 (dd, 1H), 6.59 (d, 1H), 6.38 (d, 1H), 4.61 (s, 2H).
MS (ESI⁺) *m*/*z* 259.1 (M⁺+H).

### Intermediate 45

### N-(2-methyl-1H-indol-5-ylmethyl)-4-chloro-pyrimidin-2-amine

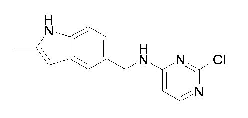

¹H-NMR (500 MHz, DMSO-*d*₆) δ 10.86 (s, 1H), 8.27 (br s, 1H), 7.90 (br s, 1H), 7.33 (s, 1H), 7.21 (d, 1H), 6.95 (d, 1H), 6.49 (d, 1H), 6.07 (s, 1H), 4.51 (br s, 2H), 2.36 (s, 3H).
MS (ESI⁺) *m*/*z* 273.1 (M⁺+H).

### Intermediate 46

### N-(1H-indazol-5-ylmethyl)-2-chloro-pyrimidin-4-amine

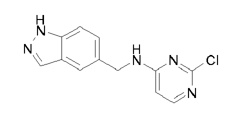

¹H-NMR (500 MHz, DMSO-*d*₆) δ 13.03 (s, 1H), 8.37 (br s, 1H), 8.04 (br s, 1H), 7.92 (m, 1H), 7.67 (s, 1H), 7.51 (d, 1H), 7.31 (d, 1H), 6.51 (d, 1H),4.58 (br s, 2H).

### Intermediate 47

### N-(1H-benzo[d]imidazol-5-ylmethyl)-2-chloro-pyrimidin-4-amine

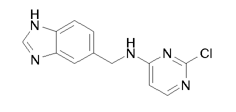

¹H NMR (500 MHz, DMSO-*d*₆, 75°C) *δ* 12.22 (br s, 1H), 8.21 (br s, 1H), 8.13 (s, 1H), 7.93 (d, 1H), 7.53 (m, 2H), 7.17 (d, 1H), 6.51 (d, 1H), 4.59 (d, 2H).
MS (ESI⁺) *m*/*z* 260.2 (M⁺+H).

### Intermediate 48

### N-(1H-indol-6-ylmethyl)-2-chloro-pyrimidin-4-amine

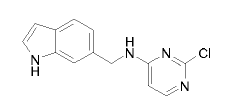

¹H NMR (500 MHz, DMSO-*d*₆, 75°C) δ 10.86 (br s, 1H), 8.18 (br s, 1H), 7.92 (d, 1H), 7.49 (d, 1H), 7.34 (s, 1H), 7.27 (t, 1H), 6.97 (d, 1H), 6.50 (d, 1H), 6.39 (br s, 1H), 4.56 (d, 2H).
MS (ESI⁺) *m*/*z* 259.1 (M⁺+H).

### Intermediate 49

### N-(1H-indol-6-ylmethyl)-4-chloro-pyrimidin-2-amine

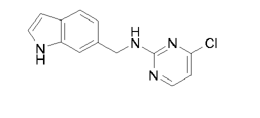

¹H NMR (500 MHz, DMSO-d₆) δ 10.99 (br s, 1H), 8.23-8.20 (m, 2H), 7.45 (d, 1H), 7.30 (s, 1H), 7.28 (t, 1H), 6.95 (d, 1H), 6.66 (d, 1H), 6.36 (br s, 1H), 4.57 (br s, 1H).
MS (ESI⁺) *m*/*z* 259.0 (M⁺+H).

### Intermediate 50

### N-(1H-indol-5-ylmethyl)-2-chloro-6-methyl-pyrimidin-4-amine

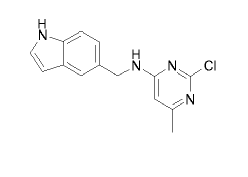

¹H-NMR (300 MHz, CDCl₃) δ 8.23 (br. s, 1H), 7.58 (s, 1H), 7.40 (d, 1H), 7.25 (s, 1H), 7.13 (d, 1H), 6.55-6.53 (m, 1H), 6.11 (s, 1H), 4.58 (br. s, 1H), 2.32 (s, 3H).
MS (ESI⁺) *m*/*z* 273.1 (M⁺+H).

### Intermediate 51

### N-(1H-indol-5-ylmethyl)-4-chloro-6-methyl-pyrimidin-2-amine

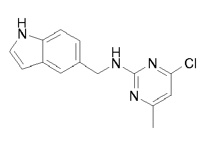

¹H-NMR (500 MHz, DMSO-d₆) δ 11.04 (s, 1H), 8.16 (br s, 1H), 7.46 (s, 1H),7.34 (d, 1H), 7.32 (m, 1H), 7.04 (d, 1H), 6.38 (br s, 1H), 6.32 (br s, 1H), 4.53 (br s, 2H), 2.17 (s, 3H).

### Intermediate 52

### N-(1H-indazol-5-ylmethyl)-2-chloro-6-methyl-pyrimidin-4-amine

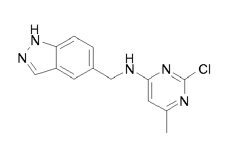

¹H-NMR (500 MHz, CDCl₃) δ 10.37 (m, 1H), 8.05 (d, 1H), 7.71 (d, 1H), 7.48 (m, 1H), 7.38 (m, 1H), 6.50 (s, 1H), 5.62 (br s, 1H), 4.73 (d, 2H), 2.32 (s, 3H).

### Intermediate 53

### N-(1H-indol-5-ylmethyl)-2-chloro-6-trifluoromethyl-pyrimidin-4-amine

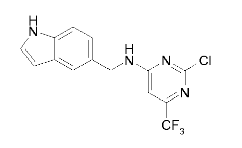

¹H NMR (300 MHz, CDCl₃, 75°C) δ 8.16 (br s, 1H), 7.60 (s, 1H), 7.41 (d, 1H), 7.24 (m, 1H), 7.15 (d, 1H), 6.58 (m, 2H), 5.62 (br s, 1H), 4.69 (d, 2H).
MS (ESI⁺) *m*/*z* 327.1 (M⁺+H).

### Intermediate 54

### N-(1H-indol-5-ylmethyl)-6-benzyl-2-chloro-pyrimidin-4-amine

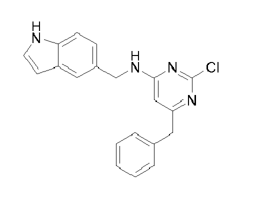

¹H NMR (500 MHz, DMSO-*d*₆, 75°C) δ 10.86 (br s, 1H), 8.04 (br s, 1H), 7.45 (s, 1H), 7.34 (d, 1H), 7.31-7.28 (m, 3H), 7.24-7.20 (m, 3H), 7.03 (d, 1H), 6.38 (m, 1H), 6.28 (s, 1H), 4.50 (d, 2H), 3.80 (s, 2H).
MS (ESI⁺) *m*/*z* 349.2 (M⁺+H).

### Intermediate 55

### N-(1H-indol-5-ylmethyl)-2-chloro-5-nitro-pyrimidin-4-amine

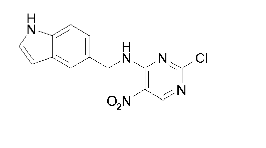

¹H-NMR (500 MHz, DMSO-*d*₆) δ 11.06 (s, 1H), 9.53 (t, 1H), 9.03 (s, 1H), 7.54 (s, 1H), 7.34 (d, 1H), 7.32 (t, 1H), 7.13 (dd, 1H), 6.38 (br. s, 1H), 4.80 (d, 2H).
MS (ESI⁺) *m*/*z* 304.1 (M⁺+H).

### Intermediate 56

### N-(1H-indol-4-yl)-2-chloro-pyrimidin-4-amine

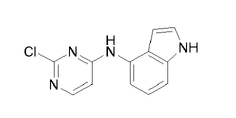

¹H-NMR (500 MHz, CDCl₃) δ 8.39 (br s, 1H), 8.07 (d, 1H), 7.36 (d, 1H), 7.26 (m, 1H), 7.23 (t, 1H), 7.18 (br s, 1H), 7.10 (d, 1H), 6.52 (d, 1H), 6.44 (br s, 1H).
MS (ESI⁺) *m*/*z* 245.1 (M⁺+H).

### Intermediate 57

### N-[2-(1H-indol-3-yl)ethyl]-2-chloro-pyrimidin-4-amine

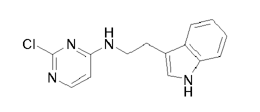

¹H NMR (500 MHz, DMSO-*d*₆, 75°C) δ 10.66 (br s, 1H), 7.90 (d, 1H), 7.79 (br s, 1H), 7.58 (d, 1H), 7.35 (d, 1H), 7.15 (s, 1H), 7.07 (t, 1H), 6.99 (t, 1H), 6.44 (d, 1H), 3.56 (m, 2H), 2.96 (t, 2H).
MS (ESI⁺) *m*/*z* 273.2 (M⁺+H).

### Intermediate 58

### N⁴-(1H-indol-5-ylmethyl)-N²-(1H-indol-4-yl)-5-nitropyrimidine-2,4-diamine

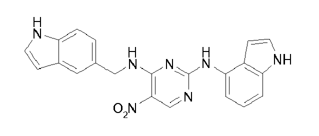

¹H-NMR (500 MHz, DMSO-d₆) δ 11.13 (s, 1H), 10.99 (s, 1H), 10.09 (s, 1H), 9.20 (s, 1H), 8.98 (s, 1H), 7.44 (br. s, 1H), 7.29-7.25 (m, 4H), 7.21 (d, 1H), 7.02 (t, 1H), 6.68 (br. s, 1H), 6.26 (br. s, 1H), 4.70 (s, 2H).
MS (ESI⁺) *m*/*z* 400.3 (M⁺+H).

### Intermediate 59

### N⁴-(1H-indol-5-ylmethyl)-N²-(1H-indol-5-yl)-5-nitropyrimidine-2,4-diamine

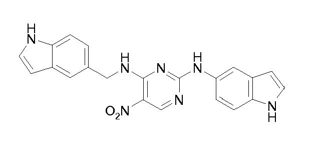

¹H-NMR (500 MHz, DMSO-*d*₆, 75°C) δ 10.85 (s, 2H), 9.97 (br. s, 1H), 9.05 (br. s, 1H), 9.20 (s, 1H), 7.91 (s, 1H), 7.51 (s, 1H), 7.33-7.30 (m, 3H), 7.28 (dt, 2H), 7.12 (d, 1H), 6.33 (d, 2H), 4.85 (d, 2H).
MS (ESI⁺) *m*/*z* 400.2 (M⁺+H).

### Intermediate 60

### N⁴-(1H-indol-5-ylmethyl)-N²-(1H-indol-6-yl)-5-nitropyrimidine-2,4-diamine

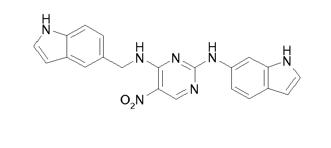

¹H-NMR (500 MHz, DMSO-*d*₆, 75°C) δ 10.86 (br. s, 2H), 10.07 (br. s, 1H), 8.97 (br. s, 1H), 8.95 (s, 1H), 7.94 (s, 1H), 7.54 (s, 1H), 7.44 (d, 1H), 7.35-7.31 (m, 2H), 7.28-7.25 (m, 2H), 7.15 (d, 1H), 6.38 (br. s, 1H), 6.31 (br. s, 1H), 4.88 (d, 2H).
MS (ESI⁺) *m*/*z* 400.3 (M⁺+H).

### Intermediate 61

### N²,N⁴-bis(1H-indol-5-ylmethyl)-5-nitropyrimidine-2,4-diamine

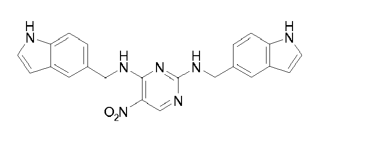

¹H-NMR (500 MHz, DMSO-*d*₆, 105°C) δ 10.76 (m, 2H), 8.86 (br. s, 2H), 8.35 (br. s, 1H), 7.52 (d, 2H), 7.32 (t, 2H), 7.26 (m, 2H), 7.10 (dd, 2H), 4.83 (d, 2H), 4.66 (d, 2H).
MS (ESI⁺) *m*/*z* 414.3 (M⁺+H).

### Biological Assays

The Fluorometric Microculture Cytotoxicity Assay, FMCA, is a three day non-clonogenic microplate-based cell viability assay used for measurement of the cytotoxic and/or cytostatic effect of compounds *in vitro* (Lindhagen, E., et al. Nat Protoc, 2008. 3(8): p. 1364-9). FMCA (Larsson, R. and P. Nygren, Anticancer Res, 1989. 9(4): p. 1111-9) represents a valuable method to measure cytotoxicity in a number of cell types, both cell lines and primary cells from patients (Larsson, R., et al., Int J Cancer, 1992. 50(2): p. 177-85; Fridborg, H., et al., Eur J Cancer, 1999. 35(3): p. 424-32; Dhar, S., et al., Br J Cancer, 1996. 74(6): p. 888-96).

FMCA is based on the principle that fluorescein diacetate (FDA) is converted to the fluorescent probe fluorescein by esterases in the plasma membranes of living cells. For experiments, 96 or 384-well microplates are prepared with compounds and stored at -70°C until use. Cells are then seeded into the drug-prepared plates and placed in an incubator for 72 h. On the last day of incubation, the plates are washed and a buffer containing FDA is added and incubated with the cells for 45 minutes. Finally the fluorescence per well is measured in a fluorometer and a Survival Index % (SI) for each compound-treated well is calculated with the equation: Compound-treated cells minus blank divided by control cells minus blank. A high SI-value indicates a large percentage of living cells and vice versa.

For experiments with compounds of the invention, 96-well plates were prepared as follows:

Compounds were dissolved in DMSO to 10 mM and stored at -20°C. 96-well plates were prepared with 297 µl of sterile PBS added to each well. The test compounds were thawed, protected from light, mixed, and 3 µl stock solution was added to the 96-well plate to give the concentration 100 µM. Then, an assay plate was prepared by transferring 20 µl of compound solution to a V-bottomed 96-well plate. Compounds at 100 µM were diluted with PBS to 10 µM, and an assay plate containing 20 µl was prepared. The plates were stored at - 70°C until use.

On the day of cell seeding, 180 µl of cell suspension was added to each well in the two assay plates. The final concentration of compounds tested was thus 10 µM and 1 µM.

In subsequent experiments, compounds were tested, along with comparative kinase inhibitors (erlotinib, gefitinib, imatinib, dasatinib, pazopanib, sorafenib, and sunitinib) as described above. Initially, the acute lymphoblastic leukemia cell line CCRF-CEM was used throughout. In the assay plates, medium was added to six empty wells (blank wells) and wells were filled with PBS and cell suspension and served as control wells. SI-values were then calculated for each compound-treated well as described above. All experiments were conducted twice and a new batch of plates was prepared for each experiment. The data obtained showed the activity of the example compounds compared to the comparative compounds.

The compounds of the invention are also tested on colon cancer cell line HCT 116, breast cancer cell line MCF7, teponisid resistant cell line CCRF-CEM & CEM/VM1, doxorubicin resistant lung cancer cell liner H69 & H69AR, doxorubicin resistant myeloma cell line RPMI8226 & 8226/DOX40, vincristine resistant lymphoma cell line U937 & U937-VCR, doxorubicin respective cisplatin resistant ovarie carcinoma cell line A2780 & A2780/Adr & A2780/Cis, and multiresistent kidney cancer cell line ACHN.

For dose-response experiments, 384-well plates were prepared as follows: Compounds of the invention as well comparative kinase inhibitors sorafenib and sunitinib (reference compounds) were diluted with PBS to a concentration ten-times higher than the desired starting concentration. Then, a Biomek 2000 liquid handling system was employed to serially dilute the compounds in a deep-well 384-well plate. From this plate, assay plates containing 5 µl compound per well were prepared with the Biomek 2000. Certain compounds precipitated when diluted with PBS, and these compounds were therefore prepared in a 96-well plate manually as described above using culture medium RPMI 1640 instead of PBS.

The compounds were also tested at five concentrations, with five times serial dilution on the following cell types: CCRF-CEM, hTERT-RPE1 (normal retinal epithelial cells), hRPTEpiC (normal renal cells) and peripheral blood mononuclear cells (PBMC). Each experiment was performed three times, except for PBMC and hRPTEpiC, which were performed twice. SI-values were calculated, graphs were plotted using GraphPadPrism 5.0 (GraphPad Software Inc. La Jolla, CA) and IC₅₀-values for each cell type and compound were determined from the curves. The data obtained showed the selectivity of the example compounds compared to the reference compounds in eliminating lymphoblastic leukemia cells (CCRF-CEM) versus normal cells (normal retinal epithelial cells, hTERT-RPE1; normal renal cells, hRPTEpiC and peripheral blood mononuclear cells (PBMC).

The example compounds of the invention were active in the CCRF-CEM cell measurements, showing IC₅₀ values less than 10 µM. Preferred compounds of the invention had IC₅₀ values less than 1 µM. Sorafenib and sunitinib had IC₅₀ values of 8.3 µM and 14.1 µM, respectively, in this assay. Most of the compounds of the invention showed lower IC₅₀ values than the reference compounds and the primary results also showed that the compounds of the invention exhibited an enhanced selectivity towards CCRF-CEM cells, compared to the tested hTERT-RPE1 (normal retinal epithelial cells), hRPTEpiC (normal renal cells) and peripheral blood mononuclear cells (PBMC).

## Claims

1. A compound of formula I or a pharmaceutically acceptable ester, amide, solvate or salt thereof, 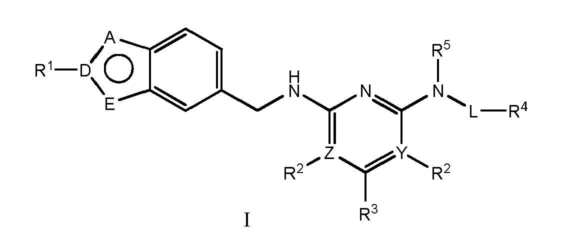 wherein
Z represents carbon or nitrogen;
Y represents carbon or nitrogen, wherein one of Z and Y represents nitrogen;
A, D and E is selected from carbon and nitrogen, wherein A represents nitrogen and D and E represents carbon; or A and D represent nitrogen and E represents carbon; or A and E represent nitrogen and D represents carbon; or E represents nitrogen and A and D represent carbon;
L represents a bond or (C₁-C₂)alkyl;
R¹ represents hydrogen or methyl, when D represents carbon;
R² represents hydrogen or amino, when Y or Z represents carbon;
R³ represents hydrogen, (C₁-C₃)alkyl, amino, trifluoromethyl or (C₀-C₁)alkylaryl;
R⁴ represents heteroaryl, optionally substituted with one or more substituents; and
R⁵ represents hydrogen or methyl.

2. A compound according to claim 1, wherein Z, D and E represent carbon; and Y and A represents nitrogen.

3. A compound according to claim 1, wherein Z and E represent carbon; and Y, D and A represent nitrogen.

4. A compound according to claim 1, wherein Z and D represent carbon; and Y, E and A represent nitrogen.

5. A compound according to claim 1, wherein Z, A and D represent carbon; and Y and E represent nitrogen.

6. A compound according to claim 1, wherein Y, D and E represent carbon; and Z and A represent nitrogen.

7. A compound according to claim 1, wherein Y and E represent carbon; and Z, D and A represent nitrogen.

8. A compound according to claim 1, wherein Y and D represent carbon; and Z, E and A represent nitrogen.

9. A compound according to claim 1, wherein Y, A and D represent carbon; and Z and E represent nitrogen.

10. A compound according to any one of claims 1 to 9, wherein R⁴ represents a heteroaryl that is optionally substituted with one or more substituents selected from the group consisting of halogen, hydroxy, amino, nitro, cyano, (C₁-C₄)alkyl, (C₁-C₄)alkyl(C₂-C₉)heterocyclyl, (C₁-C₄)alkyl(CO)OH, (C₁-C₄)alkyl(CO)O(C₁-C₄)alkyl, (C₁-C₄)alkyl(CO)NH₂, (C₁-C₄)alkyl(CO)NH(C₁-C₄)alkyl, (C₁-C₄)alkyl-OH, (C₁-C₄)alkyl-O(C₆-C₁₀)aryl, (C₁-C₄)alkyl-O(CO)(C₁-C₄)alkyl, (C₁-C₄)alkyl-O(CO)(C₆-C₁₀)aryl, (C₁-C₄)alkyl-NH₂, (C₁-C₄)alkyl-NH(C₁-C₄)alkyl, (C₁-C₄)alkyl-N[(C₁-C₄)alkyl][(C₁-C₄)-alkyl], (C₁-C₄)alkyl-NH(CO)(C₁-C₄)alkyl, (C₁-C₄)alkyl(CN), (C₁-C₄)alkyl(C₆-C₁₀)aryl, (CO)OH, (CO)O(C₁-C₄)alkyl, (CO)NH₂, (CO)NH(C₁-C₄)alkyl, (CO)(C₁-C₄)alkyl, (C₆-C₁₀)aryl, (C₆-C₁₀)aryl-halogen, (C₆-C₁₀)aryl-OH, (C₆-C₁₀)aryl-O(C₁-C₄)alkyl, (C₁-C₉)heteroaryl, (C₁-C₉)heteroaryl-halogen, (C₁-C₉)heteroaryl-OH, (C₁-C₉)heteroaryl-O(C₁-C₄)alkyl, (C₂-C₉)heterocyclyl, (C₂-C₉)heterocyclyl(C₁-C₄₎alkyl, (C₂-C₉)heterocyclyl(C₁-C₄₎alkyl-OH, O(EtO)₁₋₃H, O(EtO)₁₋₃(C₁-C₄)alkyl, O(C₆-C₁₀)aryl, O(CO)(C₁-C₄)alkyl, O(CO)(C₆-C₁₀)aryl, OSO₂OH, NH(C₁-C₄)alkyl, N[(C₁-C₄)alkyl][(C₁-C₄)alkyl], NH(CO)(C₁-C₄)alkyl, NH(CO)(C₆-C₁₀)aryl, and CF₃.

11. A compound according to claim 10, wherein said one or more substituents selected from the group consisting of hydroxy, methyl, and methoxy.

12. A compound according to any one of claims 1 to 9, wherein L-R⁴ is selected from: 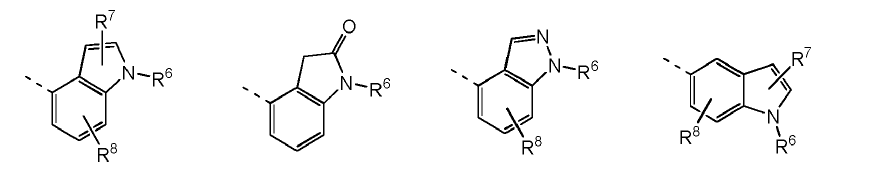 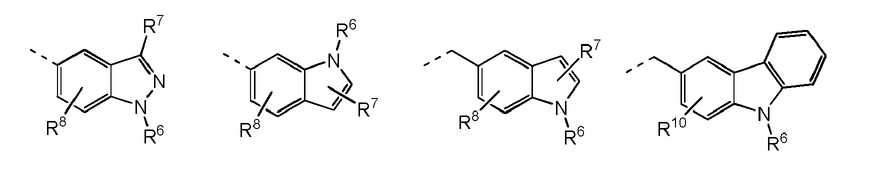 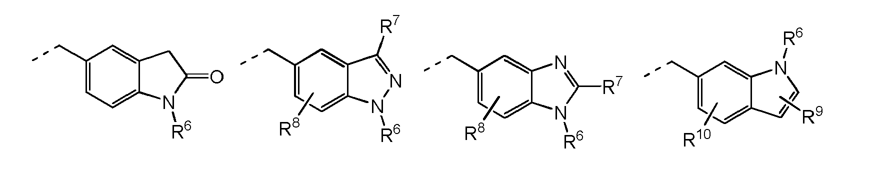 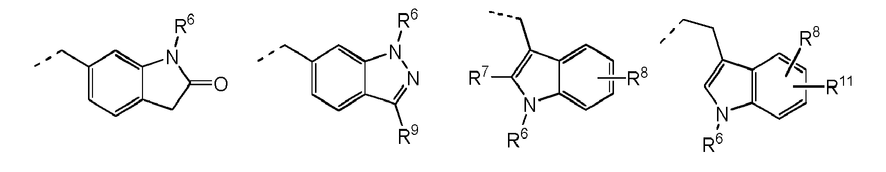 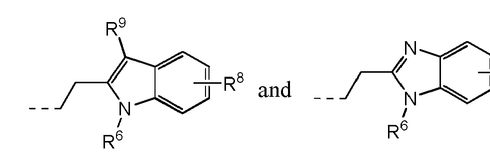
wherein R⁶ is selected from hydrogen and (C₁-C₄)alkyl;
R⁷ is selected from hydrogen, halogen, nitro, cyano, (C₁-C₄)alkyl, (C₁-C₄)alkyl(CO)OH, (C₁-C₄)alkyl(CO)O(C₁-C₄)alkyl, (C₁-C₄)alkyl(CO)NH₂, (C₁-C₄)alkyl(CO)NH(C₁-C₄)alkyl, (C₁-C₄)alkyl-OH, (C₁-C₄)alkyl-O(C₆-C₁₀)aryl, (C₁-C₄)alkyl-O(CO)(C₁-C₄)alkyl, (C₁-C₄)alkyl-O(CO)(C₆-C₁₀)aryl, (C₁-C₄)alkyl-NH₂, (C₁-C₄)alkyl-NH(C₁-C₄)alkyl, (C₁-C₄)alkyl-N[(C₁-C₄)alkyl][(C₁-C₄)alkyl], (C₁-C₄)alkyl-NH(CO)(C₁-C₄)alkyl, (C₁-C₄)alkyl(CN), (C₁-C₄)alkyl(C₆-C₁₀)aryl, (C₁-C₄)alkyl(C₂-C₉)heterocyclyl, (CO)OH, (CO)O(C₁-C₄)alkyl, (CO)NH₂, (CO)NH(C₁-C₄)alkyl, (CO)(C₁-C₄)alkyl, (C₆-C₁₀)aryl, (C₆-C₁₀)aryl-halogen, (C₆-C₁₀)aryl-OH, (C₆-C₁₀)aryl-O(C₁-C₄)alkyl, (C₁-C₉)heteroaryl, (C₁-C₉)heteroaryl-halogen, (C₁-C₉)heteroaryl-OH, (C₁-C₉)heteroaryl-O(C₁-C₄)alkyl, (C₂-C₉)heterocyclyl, (C₂-C₉)heterocyclyl(C₁-C₄)alkyl, and (C₂-C₉)heterocyclyl(C₁-C₄)alkyl-OH;
R⁸ is selected from hydrogen, halogen, nitro, cyano, hydroxy, amino, (C₁-C₄)alkyl, (C₁-C₄)alkyl(C₂-C₉)heterocyclyl, (C₁-C₄)alkyl-NH₂, (C₁-C₄)alkyl-NH(C₁-C₄)alkyl, (C₁-C₄)alkyl-N[(C₁-C₄)alkyl][(C₁-C₄)-alkyl], (C₁-C₄)alkyl-NH(CO)(C₁-C₄)alkyl, (C₁-C₄)alkyl-NH(CO)(C₆-C₁₀)aryl, (CO)(C₁-C₄)alkyl, (CO)OH, (CO)O(C₁-C₄)alkyl, (CO)NH₂, (CO)NH(C₁-C₄)alkyl, O(C₁-C₄)alkyl, O(C₁-C₄)alkyl(C₆-C₁₀)aryl, O(C₁-C₄)alkyl(C₁-C₉)heteroaryl, O(C₁-C₄)alkyl(C₂-C₉)heterocyclyl, O(C₁-C₄₎alkyl(C₂-C₉)heterocyclyl(C₁-C₄₎alkyl, O(C₁-C₄)alkyl(C₂-C₉)heterocyclyl(C₁-C₄)alkyl-OH, O(EtO)₁₋₃H, O(EtO)₁₋₃(C₁-C₄)alkyl, O(C₆-C₁₀)aryl, O(CO)(C₁-C₄)alkyl, O(CO)(C₆-C₁₀)aryl, OSO₂OH, NH(C₁-C₄)alkyl, N[(C₁-C₄)alkyl][(C₁-C₄)alkyl], NH(CO)(C₁-C₄)alkyl, NH(CO)(C₆-C₁₀)aryl, CF₃, (C₆-C₁₀)aryl, (C₆-C₁₀)aryl-halogen, (C₆-C₁₀)aryl-OH, (C₆-C₁₀)aryl-O(C₁-C₄)alkyl, (C₁-C₉)heteroaryl, (C₁-C₉)heteroaryl-halogen, (C₁-C₉)heteroaryl-OH, (C₁-C₉)heteroaryl-O(C₁-C₄)alkyl, (C₂-C₉)heterocyclyl, (C₂-C₉)heterocyclyl(C₁-C₄)alkyl, and (C₂-C₉)heterocyclyl(C₁-C₄)alkyl-OH;
R⁹ is selected from hydrogen, halogen, (C₁-C₄)alkyl, (C₁-C₄)alkyl-OH, (C₁-C₄)alkyl-O(C₁-C₄)alkyl, (CO)OH, (CO)O(C₁-C₄)alkyl, (CO)NH₂, (CO)NH(C₁-C₄)alkyl, and (C₆-C₁₀)aryl;
R¹⁰ is selected from hydrogen, halogen, hydroxy, (C₁-C₄)alkyl, (C₁-C₄)alkyl-OH, (C₁-C₄)alkyl-O(C₁-C₄)alkyl, and O(C₁-C₄)alkyl; and
R¹¹ is selected from hydrogen, hydroxy, (C₁-C₄)alkyl, and O(C₁-C₄)alkyl.

13. A compound according to any one of claims 1 to 9, wherein L-R⁴ is selected from: 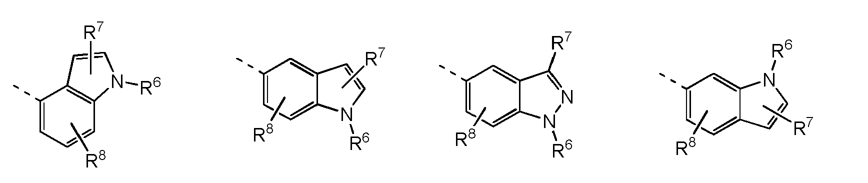 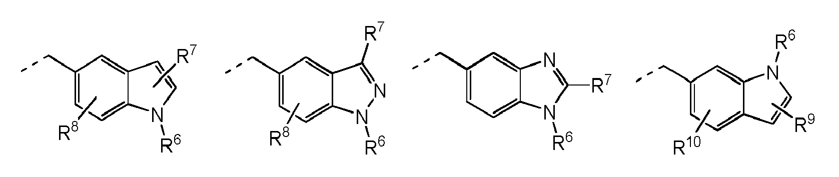 and 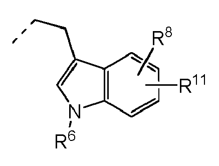 wherein
R⁶ represents hydrogen;
R⁷ is selected from hydrogen and (C₁-C₄)alkyl, preferably methyl;
R⁸, R⁹ and R¹⁰ represents hydrogen; and
R¹¹ is selected from hydrogen, hydroxy, (C₁-C₄)alkyl, preferably methyl, and O(C₁-C₄)alkyl, preferably methoxy.

14. A compound according to any one of claims 1 to 13, wherein R⁵ represents hydrogen.

15. A compound according to any one of claims 1 to 13, wherein R⁵ represents methyl.

16. A compound according to any one of claims 1 to 15, wherein R² represents amino.

17. A compound according to any one of claims 1 to 15, wherein R² represents hydrogen.

18. A compound according to any one of claims 1 to 15, wherein R² represents hydrogen; and R³ represents hydrogen, methyl, trifluoromethyl or benzyl.

19. A compound according to claim 1, wherein
Z, D and E represent carbon;
Y and A represent nitrogen;
L represents a bond or (C₁-C₂)alkyl;
R¹ represents hydrogen or methyl;
R² represents hydrogen;
R³ represents hydrogen or methyl;
R⁴ represents a heteroaryl selected from indolyl, indazolyl, benzimidazolyl, indolinonyl, and carbazolyl, said heteroaryl optionally substituted with one or two substituents selected from the group consisting of halogen, hydroxy, amino, nitro, cyano, (C₁-C₄)alkyl, (C₁-C₄)alkyl(C₂-C₉)heterocyclyl, (C₁-C₄)alkyl(CO)OH, (C₁-C₄)alkyl(CO)O(C₁-C₄)alkyl, (C₁-C₄)alkyl(CO)NH₂, (C₁-C₄)alkyl(CO)NH(C₁-C₄)alkyl, (C₁-C₄)alkyl-OH, (C₁-C₄)alkyl-O(C₆-C₁₀)aryl, (C₁-C₄)alkyl-O(CO)(C₁-C₄)alkyl, (C₁-C₄)alkyl-O(CO)(C₆-C₁₀)aryl, (C₁-C₄)alkyl-NH₂, (C₁-C₄)alkyl-NH(C₁-C₄)alkyl, (C₁-C₄)alkyl-N[(C₁-C₄)alkyl][(C₁-C₄)-alkyl], (C₁-C₄)alkyl-NH(CO)(C₁-C₄)alkyl, (C₁-C₄)alkyl(CN), (C₁-C₄)alkyl(C₆-C₁₀)aryl, (CO)OH, (CO)O(C₁-C₄)alkyl, (CO)NH₂, (CO)NH(C₁-C₄)alkyl, (CO)(C₁-C₄)alkyl, (C₆-C₁₀)aryl, (C₆-C₁₀)aryl-halogen, (C₆-C₁₀)aryl-OH, (C₆-C₁₀)aryl-O(C₁-C₄)alkyl, (C₁-C₉)heteroaryl, (C₁-C₉)heteroaryl-halogen, (C₁-C₉)heteroaryl-OH, (C₁-C₉)heteroaryl-O(C₁-C₄)alkyl, (C₂-C₉)heterocyclyl, (C₂-C₉)heterocyclyl(C₁-C₄)alkyl, (C₂-C₉)heterocyclyl(C₁-C₄)alkyl-OH, O(EtO)₁₋₃H, O(EtO)₁₋₃(C₁-C₄)alkyl, O(C₆-C₁₀)aryl, O(CO)(C₁-C₄)alkyl, O(CO)(C₆-C₁₀)aryl, OSO₂OH, NH(C₁-C₄)alkyl, N[(C₁-C₄)alkyl][(C₁-C₄)alkyl], NH(CO)(C₁-C₄)alkyl, NH(CO)(C₆-C₁₀)aryl, and CF₃; and
R⁵ represents hydrogen or methyl.

20. A compound according to claim 1, wherein
Z and E represent carbon;
Y, D, and A represent nitrogen;
L represents a bond or (C₁-C₂)alkyl;
R² represents hydrogen;
R³ represents hydrogen or methyl;
R⁴ represents a heteroaryl selected from indolyl, indazolyl, benzimidazolyl, indolinonyl, and carbazolyl, said heteroaryl optionally substituted with one or two substituents selected from the group consisting of halogen, hydroxy, amino, nitro, cyano, (C₁-C₄)alkyl, (C₁-C₄)alkyl(C₂-C₉)heterocyclyl, (C₁-C₄)alkyl(CO)OH, (C₁-C₄)alkyl(CO)O(C₁-C₄)alkyl, (C₁-C₄)alkyl(CO)NH₂, (C₁-C₄)alkyl(CO)NH(C₁-C₄)alkyl, (C₁-C₄)alkyl-OH, (C₁-C₄)alkyl-O(C₆-C₁₀)aryl, (C₁-C₄)alkyl-O(CO)(C₁-C₄)alkyl, (C₁-C₄)alkyl-O(CO)(C₆-C₁₀)aryl, (C₁-C₄)alkyl-NH₂, (C₁-C₄)alkyl-NH(C₁-C₄)alkyl, (C₁-C₄)alkyl-N[(C₁-C₄)alkyl][(C₁-C₄)-alkyl], (C₁-C₄)alkyl-NH(CO)(C₁-C₄)alkyl, (C₁-C₄)alkyl(CN), (C₁-C₄)alkyl(C₆-C₁₀)aryl, (CO)OH, (CO)O(C₁-C₄)alkyl, (CO)NH₂, (CO)NH(C₁-C₄)alkyl, (CO)(C₁-C₄)alkyl, (C₆-C₁₀)aryl, (C₆-C₁₀)aryl-halogen, (C₆-C₁₀)aryl-OH, (C₆-C₁₀)aryl-O(C₁-C₄)alkyl, (C₁-C₉)heteroaryl, (C₁-C₉)heteroaryl-halogen, (C₁-C₉)heteroaryl-OH, (C₁-C₉)heteroaryl-O(C₁-C₄)alkyl, (C₂-C₉)heterocyclyl, (C₂-C₉)heterocyclyl(C₁-C₄)alkyl, (C₂-C₉)heterocyclyl(C₁-C₄)alkyl-OH, O(EtO)₁₋₃H, O(EtO)₁₋₃(C₁-C₄)alkyl, O(C₆-C₁₀)aryl, O(CO)(C₁-C₄)alkyl, O(CO)(C₆-C₁₀)aryl, OSO₂OH, NH(C₁-C₄)alkyl, N[(C₁-C₄)alkyl][(C₁-C₄)alkyl], NH(CO)(C₁-C₄)alkyl, NH(CO)(C₆-C₁₀)aryl, and CF₃; and
R⁵ represents hydrogen or methyl.

21. A compound according to claim 1, wherein
Z and D represent carbon;
Y, E and A represent nitrogen;
L represents a bond or (C₁-C₂)alkyl;
R¹ represents hydrogen;
R² represents hydrogen;
R³ represents hydrogen or methyl;
R⁴ represents a heteroaryl selected from indolyl, indazolyl, benzimidazolyl, indolinonyl, and carbazolyl, said heteroaryl optionally substituted with one or two substituents selected from the group consisting of halogen, hydroxy, amino, nitro, cyano, (C₁-C₄)alkyl, (C₁-C₄)alkyl(C₂-C₉)heterocyclyl, (C₁-C₄)alkyl(CO)OH, (C₁-C₄)alkyl(CO)O(C₁-C₄)alkyl, (C₁-C₄)alkyl(CO)NH₂, (C₁-C₄)alkyl(CO)NH(C₁-C₄)alkyl, (C₁-C₄)alkyl-OH, (C₁-C₄)alkyl-O(C₆-C₁₀)aryl, (C₁-C₄)alkyl-O(CO)(C₁-C₄)alkyl, (C₁-C₄)alkyl-O(CO)(C₆-C₁₀)aryl, (C₁-C₄)alkyl-NH₂, (C₁-C₄)alkyl-NH(C₁-C₄)alkyl, (C₁-C₄)alkyl-N[(C₁-C₄)alkyl][(C₁-C₄)-alkyl], (C₁-C₄)alkyl-NH(CO)(C₁-C₄)alkyl, (C₁-C₄)alkyl(CN), (C₁-C₄)alkyl(C₆-C₁₀)aryl, (CO)OH, (CO)O(C₁-C₄)alkyl, (CO)NH₂, (CO)NH(C₁-C₄)alkyl, (CO)(C₁-C₄)alkyl, (C₆-C₁₀)aryl, (C₆-C₁₀)aryl-halogen, (C₆-C₁₀)aryl-OH, (C₆-C₁₀)aryl-O(C₁-C₄)alkyl, (C₁-C₉)heteroaryl, (C₁-C₉)heteroaryl-halogen, (C₁-C₉)heteroaryl-OH, (C₁-C₉)heteroaryl-O(C₁-C₄)alkyl, (C₂-C₉)heterocyclyl, (C₂-C₉)heterocyclyl(C₁-C₄)alkyl, (C₂-C₉)heterocyclyl(C₁-C₄)alkyl-OH, O(EtO)₁₋₃H, O(EtO)₁₋₃(C₁-C₄)alkyl, O(C₆-C₁₀)aryl, O(CO)(C₁-C₄)alkyl, O(CO)(C₆-C₁₀)aryl, OSO₂OH, NH(C₁-C₄)alkyl, N[(C₁-C₄)alkyl][(C₁-C₄)alkyl], NH(CO)(C₁-C₄)alkyl, NH(CO)(C₆-C₁₀)aryl, and CF₃; and
R⁵ represents hydrogen or methyl.

22. A compound according to claim 1, wherein
Y, D and E represent carbon;
Z and A represent nitrogen;
L represents a bond or (C₁-C₂)alkyl;
R¹ represents hydrogen or methyl;
R² represents hydrogen;
R³ represents hydrogen or methyl;
R⁴ represents a heteroaryl selected from indolyl, indazolyl, benzimidazolyl, indolinonyl, and carbazolyl, said heteroaryl optionally substituted with one or two substituents selected from the group consisting of halogen, hydroxy, amino, nitro, cyano, (C₁-C₄)alkyl, (C₁-C₄)alkyl(C₂-C₉)heterocyclyl, (C₁-C₄)alkyl(CO)OH, (C₁-C₄)alkyl(CO)O(C₁-C₄)alkyl, (C₁-C₄)alkyl(CO)NH₂, (C₁-C₄)alkyl(CO)NH(C₁-C₄)alkyl, (C₁-C₄)alkyl-OH, (C₁-C₄)alkyl-O(C₆-C₁₀)aryl, (C₁-C₄)alkyl-O(CO)(C₁-C₄)alkyl, (C₁-C₄)alkyl-O(CO)(C₆-C₁₀)aryl, (C₁-C₄)alkyl-NH₂, (C₁-C₄)alkyl-NH(C₁-C₄)alkyl, (C₁-C₄)alkyl-N[(C₁-C₄)alkyl][(C₁-C₄)-alkyl], (C₁-C₄)alkyl-NH(CO)(C₁-C₄)alkyl, (C₁-C₄)alkyl(CN), (C₁-C₄)alkyl(C₆-C₁₀)aryl, (CO)OH, (CO)O(C₁-C₄)alkyl, (CO)NH₂, (CO)NH(C₁-C₄)alkyl, (CO)(C₁-C₄)alkyl, (C₆-C₁₀)aryl, (C₆-C₁₀)aryl-halogen, (C₆-C₁₀)aryl-OH, (C₆-C₁₀)aryl-O(C₁-C₄)alkyl, (C₁-C₉)heteroaryl, (C₁-C₉)heteroaryl-halogen, (C₁-C₉)heteroaryl-OH, (C₁-C₉)heteroaryl-O(C₁-C₄)alkyl, (C₂-C₉)heterocyclyl, (C₂-C₉)heterocyclyl(C₁-C₄)alkyl, (C₂-C₉)heterocyclyl(C₁-C₄)alkyl-OH, O(EtO)₁₋₃H, O(EtO)₁₋₃(C₁-C₄)alkyl, O(C₆-C₁₀)aryl, O(CO)(C₁-C₄)alkyl, O(CO)(C₆-C₁₀)aryl, OSO₂OH, NH(C₁-C₄)alkyl, N[(C₁-C₄)alkyl][(C₁-C₄)alkyl], NH(CO)(C₁-C₄)alkyl, NH(CO)(C₆-C₁₀)aryl, and CF₃; and
R⁵ represents hydrogen or methyl.

23. A compound according to claim 1, wherein
Y and E represent carbon;
Z, D, and A represent nitrogen;
L represents a bond or (C₁-C₂)alkyl;
R² represents hydrogen;
R³ represents hydrogen or methyl;
R⁴ represents a heteroaryl selected from indolyl, indazolyl, benzimidazolyl, indolinonyl, and carbazolyl, said heteroaryl optionally substituted with one or two substituents selected from the group consisting of halogen, hydroxy, amino, nitro, cyano, (C₁-C₄)alkyl, (C₁-C₄)alkyl(C₂-C₉)heterocyclyl, (C₁-C₄)alkyl(CO)OH, (C₁-C₄)alkyl(CO)O(C₁-C₄)alkyl, (C₁-C₄)alkyl(CO)NH₂, (C₁-C₄)alkyl(CO)NH(C₁-C₄)alkyl, (C₁-C₄)alkyl-OH, (C₁-C₄)alkyl-O(C₆-C₁₀)aryl, (C₁-C₄)alkyl-O(CO)(C₁-C₄)alkyl, (C₁-C₄)alkyl-O(CO)(C₆-C₁₀)aryl, (C₁-C₄)alkyl-NH₂, (C₁-C₄)alkyl-NH(C₁-C₄)alkyl, (C₁-C₄)alkyl-N[(C₁-C₄)alkyl][(C₁-C₄)-alkyl], (C₁-C₄)alkyl-NH(CO)(C₁-C₄)alkyl, (C₁-C₄)alkyl(CN), (C₁-C₄)alkyl(C₆-C₁₀)aryl, (CO)OH, (CO)O(C₁-C₄)alkyl, (CO)NH₂, (CO)NH(C₁-C₄)alkyl, (CO)(C₁-C₄)alkyl, (C₆-C₁₀)aryl, (C₆-C₁₀)aryl-halogen, (C₆-C₁₀)aryl-OH, (C₆-C₁₀)aryl-O(C₁-C₄)alkyl, (C₁-C₉)heteroaryl, (C₁-C₉)heteroaryl-halogen, (C₁-C₉)heteroaryl-OH, (C₁-C₉)heteroaryl-O(C₁-C₄)alkyl, (C₂-C₉)heterocyclyl, (C₂-C₉)heterocyclyl(C₁-C₄)alkyl, (C₂-C₉)heterocyclyl(C₁-C₄)alkyl-OH, O(EtO)₁₋₃H, O(EtO)₁₋₃(C₁-C₄)alkyl, O(C₆-C₁₀)aryl, O(CO)(C₁-C₄)alkyl, O(CO)(C₆-C₁₀)aryl, OSO₂OH, NH(C₁-C₄)alkyl, N[(C₁-C₄)alkyl][(C₁-C₄)alkyl], NH(CO)(C₁-C₄)alkyl, NH(CO)(C₆-C₁₀)aryl, and CF₃; and
R⁵ represents hydrogen or methyl.

24. A compound according to claim 1, wherein
Y and D represent carbon;
Z, E and A represent nitrogen;
L represents a bond or (C₁-C₂)alkyl;
R¹ represents hydrogen;
R² represents hydrogen;
R³ represents hydrogen or methyl;
R⁴ represents a heteroaryl selected from indolyl, indazolyl, benzimidazolyl, indolinonyl, and carbazolyl, said heteroaryl optionally substituted with one or two substituents selected from the group consisting of halogen, hydroxy, amino, nitro, cyano, (C₁-C₄)alkyl, (C₁-C₄)alkyl(C₂-C₉)heterocyclyl, (C₁-C₄)alkyl(CO)OH, (C₁-C₄)alkyl(CO)O(C₁-C₄)alkyl, (C₁-C₄)alkyl(CO)NH₂, (C₁-C₄)alkyl(CO)NH(C₁-C₄)alkyl, (C₁-C₄)alkyl-OH, (C₁-C₄)alkyl-O(C₆-C₁₀)aryl, (C₁-C₄)alkyl-O(CO)(C₁-C₄)alkyl, (C₁-C₄)alkyl-O(CO)(C₆-C₁₀)aryl, (C₁-C₄)alkyl-NH₂, (C₁-C₄)alkyl-NH(C₁-C₄)alkyl, (C₁-C₄)alkyl-N[(C₁-C₄)alkyl][(C₁-C₄)-alkyl], (C₁-C₄)alkyl-NH(CO)(C₁-C₄)alkyl, (C₁-C₄)alkyl(CN), (C₁-C₄)alkyl(C₆-C₁₀)aryl, (CO)OH, (CO)O(C₁-C₄)alkyl, (CO)NH₂, (CO)NH(C₁-C₄)alkyl, (CO)(C₁-C₄)alkyl, (C₆-C₁₀)aryl, (C₆-C₁₀)aryl-halogen, (C₆-C₁₀)aryl-OH, (C₆-C₁₀)aryl-O(C₁-C₄)alkyl, (C₁-C₉)heteroaryl, (C₁-C₉)heteroaryl-halogen, (C₁-C₉)heteroaryl-OH, (C₁-C₉)heteroaryl-O(C₁-C₄)alkyl, (C₂-C₉)heterocyclyl, (C₂-C₉)heterocyclyl(C₁-C₄)alkyl, (C₂-C₉)heterocyclyl(C₁-C₄)alkyl-OH, O(EtO)₁₋₃H, O(EtO)₁₋₃(C₁-C₄)alkyl, O(C₆-C₁₀)aryl, O(CO)(C₁-C₄)alkyl, O(CO)(C₆-C₁₀)aryl, OSO₂OH, NH(C₁-C₄)alkyl, N[(C₁-C₄)alkyl][(C₁-C₄)alkyl], NH(CO)(C₁-C₄)alkyl, NH(CO)(C₆-C₁₀)aryl, and CF₃; and
R⁵ represents hydrogen or methyl.

25. A compound according to claim 1, wherein
Z, D and E represent carbon;
Y and A represent nitrogen;
L represents a bond or (C₁-C₂)alkyl;
R¹ represents hydrogen or methyl;
R² represents hydrogen;
R³ represents hydrogen or methyl;
R⁴ represents a heteroaryl selected from indolyl, indazolyl, and benzimidazolyl, said heteroaryl optionally substituted with one or two substituents selected from the group consisting of hydroxy, methyl, and methoxy; and
R⁵ represents hydrogen.

26. A compound according to claim 1, wherein
Z and E represent carbon;
Y, D, and A represent nitrogen;
L represents a bond or (C₁-C₂)alkyl;
R² represents hydrogen;
R³ represents hydrogen or methyl;
R⁴ represents a heteroaryl selected from indolyl, indazolyl, and benzimidazolyl, said heteroaryl optionally substituted with one or two substituents selected from the group consisting of hydroxy, methyl, and methoxy; and
R⁵ represents hydrogen.

27. A compound according to claim 1, wherein
Z and D represent carbon;
Y, E and A represent nitrogen;
L represents a bond or (C₁-C₂)alkyl;
R¹ represents hydrogen;
R² represents hydrogen;
R³ represents hydrogen or methyl;
R⁴ represents a heteroaryl selected from indolyl, indazolyl, and benzimidazolyl, said heteroaryl optionally substituted with one or two substituents selected from the group consisting of hydroxy, methyl, and methoxy; and
R⁵ represents hydrogen.

28. A compound according to claim 1, wherein
Y, D and E represent carbon;
Z and A represent nitrogen;
L represents a bond or (C₁-C₂)alkyl;
R¹ represents hydrogen or methyl;
R² represents hydrogen;
R³ represents hydrogen or methyl;
R⁴ represents a heteroaryl selected from indolyl, indazolyl, and benzimidazolyl, said heteroaryl optionally substituted with one or two substituents selected from the group consisting of hydroxy, methyl, and methoxy; and
R⁵ represents hydrogen.

29. A compound according to claim 1, wherein
Y and E represent carbon;
Z, D, and A represent nitrogen;
L represents a bond or (C₁-C₂)alkyl;
R² represents hydrogen;
R³ represents hydrogen or methyl;
R⁴ represents a heteroaryl selected from indolyl, indazolyl, and benzimidazolyl, said heteroaryl optionally substituted with one or two substituents selected from the group consisting of hydroxy, methyl, and methoxy; and
R⁵ represents hydrogen.

30. A compound according to claim 1, wherein
Y and D represent carbon;
Z, E and A represent nitrogen;
L represents a bond or (C₁-C₂)alkyl;
R¹ represents hydrogen;
R² represents hydrogen;
R³ represents hydrogen or methyl;
R⁴ represents a heteroaryl selected from indolyl, indazolyl, and benzimidazolyl, said heteroaryl optionally substituted with one or two substituents selected from the group consisting of hydroxy, methyl, and methoxy; and
R⁵ represents hydrogen.

31. A compound according to any one of claims 1 to 30, said compound being selected from:
*N*⁴*-*(1*H-*indol-5-ylmethyl)-*N*²-(1*H*-indol-4-yl)pyrimidine-2,4-diamine;
*N*⁴*-*(1*H-*indol-5-ylmethyl)-*N*²-(1*H*-indol-5-yl)pyrimidine-2,4-diamine;
*N*⁴*-*(1*H* indol-5-ylmethyl)-*N*²-(2-methyl-1H-indol-5-yl)pyrimidine-2,4-diamine;
*N*⁴-(1*H* indol-5-ylmethyl)-*N*²-(1*H*-indazol-5-yl)pyrimidine-2,4-diamine;
*N*²,*N*⁴-bis(1*H*-indol-5-ylmethyl)pyrimidine-2,4-diamine;
*N*⁴-(1*H-*indol-5-ylmethyl)-*N*²-(2-methyl-1*H*-indol-5-ylmethyl)pyrimidine-2,4-diamine;
*N*²- (1*H*-indazol-5-ylmethyl)-*N*⁴-(1*H*-indol-5-ylmethyl)pyrimidine-2,4-diamine;
*N*²-(1*H-benzo*[*d*]imidazol-5-ylmethyl)-*N*⁴-(1*H*-indol-5-ylmethyl)pyrimidine-2,4-diamine;
*N*⁴-(1*H*-indol-5-ylmethyl)-*N*²-(1*H*-indol-6-ylmethyl)pyrimidine-2,4-diamine;
*N*²-[2-(1*H-*indol-3-yl)ethyl]-*N*⁴-(1*H*-indol-5-ylmethyl)pyrimidine-2,4-diamine;
3-{2-[4-(1*H*-indol-5-ylmethylamino)-pyrimidin-2-ylamino]ethyl}-1*H*-indol-5-ol;
*N*⁴-(1*H-*indol-5-ylmethyl)-*N*²-[2-(5-methyl-1*H*-indol-3-yl)ethyl]pyrimidine-2,4-diamine;
*N*⁴-(1*H*-indol-5-ylmethyl)-*N*²-[2-(5-methoxy-1*H*-indol-3-yl)ethyl]pyrimidine-2,4-diamine;
*N*²-(1*H*-indol-4-yl)-*N*⁴-(2-methyl-1*H*-indol-5ylmethyl)pyrimidine-2,4-diamine;
*N*²-(1*H*-indol-5-ylmethyl)-*N*⁴-(2-methyl-1*H*-indol-5-ylmethyl)pyrimidine-2,4-diamine;
*N*²,*N*⁴-Bis-(2-methyl-1*H*-indol-5-ylmethyl)pyrimidine-2,4-diamine;
*N*²-(1*H*-indazol-5-ylmethyl)-*N*⁴-(2-methyl-1*H*-indol-5ylmethyl)pyrimidine-2,4-diamine;
*N*²-(2-(1*H*-indol-3-yl)-ethyl)-*N*⁴-(2-methyl-1*H*-indol-5ylmethyl)pyrimidine-2,4-diamine;
*N*²-(1*H-*indol-4-yl)-*N*⁴-(1*H*-indazol-5ylmethyl)pyrimidine-2,4-diamine;
*N*⁴-(1*H-*benzo[*d*]imidazol-5-ylmethyl)-*N*²-(1*H*-indol-4-yl)pyrimidine-2,4-diamine;
*N*⁴-(1*H-*indol-6-ylmethyl)-*N*²-(1*H*-indol-4-yl)pyrimidine-2,4-diamine;
*N*²*-*(1*H-*indol-5-ylmethyl)-*N*⁴-(1*H*-indol-6-ylmethyl)pyrimidine-2,4-diamine;
*N*²*,N*⁴-bis-(1*H-*indol-6-ylmethyl)pyrimidine-2,4-diamine;
*N*²-(1*H*-indol-5-ylmethyl)-*N*⁴-(1*H*-indol-4-yl)pyrimidine-2,4-diamine;
*N*²-(1*H-*indol-5-ylmethyl)-*N*⁴-(2-methyl-1*H*-indol-5-yl)pyrimidine-2,4-diamine;
*N*⁴-[2-(1*H-*indol-3-yl)ethyl]-*N*²-(1*H*-indol-5-ylmethyl)pyrimidine-2,4-diamine;
3-{2-[2-(1*H*-indol-5-ylmethylamino)-pyrimidin-4-ylamino]ethyl}-1*H*-indol-5-ol;
*N*²-(1*H-*indol-5-ylmethyl)-*N*⁴-[2-(5-methyl-1*H*-indol-3-yl)ethyl]pyrimidine-2,4-diamine;
*N*²-(1*H*-indol-5-ylmethyl)-*N*⁴-[2-(5-methoxy-1*H*-indol-3-yl)ethyl]pyrimidine-2,4-diamine;
*N*²-(1*H-*indazol-5-ylmethyl)-*N*⁴-(1*H*-indol-4-yl)pyrimidine-2,4-diamine;
*N*²-(1*H-*indol-4-yl)-*N*⁴-(1*H*-indol-5ylmethyl)-6-methylpyrimidine-2,4-diamine *N*²*,N*⁴-bis(1*H-*indol-5-ylmethyl)-6-methylpyrimidine-2,4-diamine;
3-{2-[4-(1*H*-indol-5-ylmethylamino)-6-methyl-pyrimidin-2-ylamino]-ethyl}-1*H*-indol-5-ol;
*N*⁴-(1*H-*indol-5-ylmethyl)-*N*²-(2-methyl-1*H*-indol-5-yl)-6-trifluoromethylpyrimidine-2,4-diamine;
*N*²*,N*⁴-bis-(1*H-*indol-5-ylmethyl)-6-trifluoromethylpyrimidine-2,4-diamine;
*N*²-(2-(1*H-*indol-3-yl)ethyl)-*N*⁴-(1*H*-indol-5-ylmethyl)-6-trifluoromethylpyrimidine-2,4-diamine;
*N*²*,N*⁴-bis(1*H*-indol-5-ylmethyl)-6-benzylpyrimidine-2,4-diamine;
*N*⁴-(1*H-*indazol-5-ylmethyl)-*N*²-(1*H*-indol-4-yl)-6-methylpyrimidine-2,4-diamine;
*N*⁴-(1*H-*indol-5-ylmethyl)-*N*²-(1*H*-indol-4-yl)pyrimidine-2,4,5-triamine;
*N*⁴-(1*H-*indol-5-ylmethyl)-*N*²-(1*H*-indol-5-yl)pyrimidine-2,4,5-triamine;
*N*⁴-(1*H-*indol-5-ylmethyl)-*N*²-(1*H*-indol-6-yl)pyrimidine-2,4,5-triamine; and
*N*²*,N*⁴-bis(1*H-*indol-5-ylmethyl)pyrimidine-2,4,5-triamine.

32. A compound according to any one of claims 1 to 31, for use in therapy.

33. A compound according to any one of claims 1 to 31, for use in treatment of cancer.

34. Use of a compound according to any one of claims 1 to 31, in the manufacture of a medicament and pharmaceutical compositions for treatment of cancer.

35. A pharmaceutical composition comprising a compound according to any one of claims 1 to 31 together with pharmaceutically acceptable diluents and carriers.

36. A method for treatment of cancer, which comprises administering to a subject in need thereof, a therapeutically effective amount of a compound according to claim 1.

37. A method for treatment of cancer, which comprises administering to a subject in need thereof, a therapeutically effective amount of a compound according to claim 1, in combination with another compound according to claim1, in combination with radiation therapy, or in combination with another anticancer agent selected from alkylating agents, antimetabolites, anticancer camptothecin derivatives, plan-derived anticancer agents, antibiotics, enzymes, platinum coordination complexes, tyrosine kinase inhibitors, hormones, hormone antagonists, monoclonal antibodies, interferons, and biological response modifiers.
